(19) Europäisches Patentamt / European Patent Office / Office européen des brevets

(11) **EP 3 816 185 A1**

(12) **EUROPEAN PATENT APPLICATION**

(43) Date of publication:
**05.05.2021 Bulletin 2021/18**

(51) Int Cl.:
**C07K 16/28** (2006.01)          **C07K 16/32** (2006.01)

(21) Application number: **19206959.9**

(22) Date of filing: **04.11.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Numab Therapeutics AG
8820 Wädenswil (CH)**

(72) Inventor: **The designation of the inventor has not yet been filed**

(74) Representative: **Virnekäs, Bernhard
Wallinger Ricker Schlotter Tostmann
Patent- und Rechtsanwälte Partnerschaft mbB
Zweibrückenstrasse 5-7
80331 München (DE)**

(54) **MULTISPECIFIC ANTIBODY DIRECTED AGAINST PD-L1 AND A TUMOR-ASSOCIATED ANTIGEN**

(57)     The present invention relates to a multispecific antibody comprising at least one domain specifically binding to a tumor-associated immune checkpoint antigen with low affinity, and at least one domain specifically binding to a tumor-associated antigen (TAA), and pharmaceutical compositions and methods of use thereof.

The present invention further relates to a nucleic acid encoding said multispecific antibody, a vector comprising said nucleic acid, a host cell comprising said nucleic acid or said vector, and a method of producing said multispecific antibody

**Figure 3:**

Tribody          DVD-Tribody          MATCH4

⊐  Inderdomain disulfide bond (ID DiS)
ʗ ʅ  Peptide linker
N  N-terminus
C  C-terminus
CL/CH₁  Constant light (CL) and heavy 1 (CH1) domain
VLx/VHx  Variable light (VL) and heavy (VH) domain #

**Description**

**FIELD OF THE INVENTION**

The present invention relates to a multispecific antibody comprising at least one domain specifically binding to a tumor-associated immune checkpoint antigen with low affinity, at least one domain specifically binding to a tumor-associated antigen (TAA), and optionally at least one domain specifically binding to an immune cell antigen. Additionally, the present invention relates to specific domains for use in said multispecific antibody, and pharmaceutical compositions and methods of use thereof. The present invention further relates to a nucleic acid encoding said multispecific antibody or specific domains thereof, a vector comprising said nucleic acid, a host cell comprising said nucleic acid or said vector, and a method of producing said multispecific antibody or specific domains thereof.

**BACKGROUND OF THE INVENTION**

Cancer continues to pose a major unmet medical need, despite the considerable progress made in its treatment. Some of the most substantial progress made in cancer treatment in recent years has come with the advent of immunotherapies of various molecular classes, including, but not limited to: monoclonal antibodies (mAbs), bispecific antibodies (bsAbs), recombinant proteins, and chimeric antigen receptor-T cell (CAR-T cell) therapies. Such therapies induce anti-tumor immunity by: a) actively directing immune-effector cells to tumor-resident cells and/or b) stimulating immune-effector cells and/or c) relieving tumor-mediated immune-suppression. These immunotherapies commonly exploit the overexpression - as compared to extratumoral loci - of specific antigens by tumor-resident cells (e.g., malignant cells, cells of the tumor vasculature, stromal cells, immune cells, etc.) to target their pharmacological activity to tumors. Among these antigens, tumor-associated antigens (TAAs) comprise cell-surface proteins selectively overexpressed by malignant cells. By binding to TAAs with high affinity, immunotherapies can, to a degree, restrict their immunomodulatory activity to immunological synapses between tumor cells and immune effector cells.

A common class of TAA-binding immunotherapies are mAbs that elicit anti-tumor immunity by opsonizing tumor-cells and triggering antibody-dependent cell-mediated cytotoxicity (ADCC) by Fcγ receptor (FcγR)-expressing cells, primarily natural killer (NK) cells. Other TAA-binding immunotherapies leverage cytotoxic T lymphocytes (CTLs) to induce targeted depletion of malignant cells, such as CAR-T cells as well as bsAbs that simultaneously engage the T cell antigen, CD3 (TAA/CD3 bsAbs). While the therapeutic utility of TAA-(re)directed CTLs has been clinically validated, such utility can be limited in instances when tumor-mediated immune-suppression impairs the activation/stimulation of CTLs. Even in tumors where tumor-infiltrating lymphocytes (TILs) are abundant (i.e., "inflamed" or "hot" tumors), tumor immune-evasion can be induced by a variety of means, such as through the expression of immune-checkpoint ligands/receptors (e.g., PD-1, PD-L1, CTLA-4) as well as the recruitment of regulatory T cells (Tregs) and myeloid-derived suppressor cells (MDSCs).

Immune checkpoints are regulators of the immune system and are involved in processes such as self-tolerance or immune suppression in cancer.

PD-L1 (CD274, B7-H1) is a 40 kDa type I transmembrane protein. PD-L1 is a surface glycoprotein ligand for PD-1, a key immune checkpoint receptor expressed by activated T and B cells and mediates immunosuppression. PD-L1 is implicated in the suppression of immune system responses during chronic infections, pregnancy, tissue allografts, autoimmune diseases, and cancer. PD-L1 is found on both antigen-presenting cells and human cancer cells, such as squamous cell carcinoma of the head and neck, melanoma, and brain tumor, thyroid, thymus, esophagus, lung, breast, gastrointestinal tract, colorectum, liver, pancreas, kidney, adrenal cortex, bladder, urothelium, ovary, and skin (Katsuya Y, et al., Lung Cancer.88(2):154-159 (2015); Nakanishi J, et al., Cancer Immunol Immunother. 56(8):1173-1182 (2007); Nomi T, et al., Clin Cancer Res. 13(7):2151-2157 (2007); Fay AP, et al., J Immunother Cancer. 3:3 (2015); Strome SE, et al., Cancer Res. 63(19):6501-6505 (2003); Jacobs JF, et al. Neuro Oncol.11(4):394-402 (2009); Wilmotte R, et al. Neuroreport. 16(10):1081-1085 (2005)). PD-L1 is rarely expressed on normal tissues but inducibly expressed on tumor site (Dong H, et al., Nat Med. 8(8):793-800 (2002); Wang et al., Onco Targets Ther. 9: 5023-5039 (2016)). PD-L1 downregulates T cell activation and cytokine secretion by binding to PD-1 (Freeman et al., 2000; Latchman et al, 2001). PD-1, activated by PD-L1, potentially provides an immune-tolerant environment for tumor development and growth. PD-L1 also negatively regulates T-cell function through interaction with another receptor, B7.1 (B7-1, CD80).

Inhibition of the PD-L1/PD-1 interaction allows for potent anti-tumor activity. A number of antibodies that disrupt the PD-1 signaling have entered clinical development. These antibodies belong to the following two main categories: those that target PD-1 (nivolumab, Bristol-Myers Squibb; pembrolizumab, Merck, Whitehouse Station, NJ; pidilizumab, CureTech, Yavne, Israel) and those that target PD-L1 (MPDL3280A, Genentech, South San Francisco, CA; MEDI4736, MedImmune/AstraZeneca; BMS-936559, Bristol-Myers Squibb; MSB0010718C, EMD Serono, Rockland, MA) (for review see Postow MA et al., J Clin Oncol. Jun 10;33(17):1974-82 (2015)). Targeting PD-L1 versus targeting PD-1 may result in different biologic effects. PD-1 antibodies prevent interaction of PD-1 with both its ligands, PD-L1 and PD-L2. PD-L1 antibodies do not prevent PD-1 from interacting with PDL2, although the effect of this interaction remains unknown. PD-L1 antibodies however prevent interaction of PD-L1 with not only PD-1, but also B7-1 (Butte MJ, et al., Immunity 27:111-122, (2007)), which is believed to exert negative signals on T cells. Blocking PD-L1 has demonstrated promising early data, and currently, four clinical anti-PD-L1 mAbs are in the testing: atezolizumab and MEDI4736 (both are Fc null variants of human IgG1), MSB001078C (IgG1), and BMS-936559 (IgG4) (Chester C., et al., Cancer Immunol Immunother Oct;65(10):1243-8 (2016)).

New and emerging treatments frequently combine TAA-targeting immunotherapies with one or more additional immunotherapies that target immune-checkpoint pathways in an effort to further relieve, or overcome, tumor-mediated immune-suppression. Monoclonal antibodies that block immune-suppressive antigens, such as CTLA-4 (e.g., ipiliumumab), PD-1 (e.g., nivolumab, pembrolizumab) and PD-L1 (e.g., avelumab, atezolizumab), have elicited impressive response rates in patients exhibiting a variety of tumor histologies. Initial results of combined treatment with immune-checkpoint modulators and TAA-binding immunotherapies have been encouraging. As an example, the HER2-targeting mAb, trastuzumab (Herceptin®, Genentech), is currently being clinically evaluated (phase II) in combination with nivolumab (Opdivo®, Bristol-Myers Squibb) as well as in combination with both nivolumab and ipilimumab (Yervoy®, Bristol-Myers Squibb), National Clinical Trial (NCT) 03409848. Similarly, the CD19/CD3 bsAb, blinatumomab (Blincyto®, Amgen), is currently being clinically evaluated (phase I/II) in combination with pembrolizumab (Keytruda®, Merck) as well as in a phase I trial as part of a triple immunotherapy combination with both nivolumab and ipilimumab, NCT03512405 and NCT02879695, respectively.

Additionally, combinations of targeting immune checkpoints and T-cell co-stimulatory receptors have been evaluated. The combination of anti-PD-L1 and anti-CD137 antibodies increased overall survival and enhanced T-cell effector function in the ID-8 ovarian adenocarcinoma model (Duraiswamy J, et al., Cancer Res 73:6900-6912 (2013)). The combination of urelumab (anti-CD137) with nivolumab (anti-PD-1) in both solid tumors and B-cell non-Hodgkin's lymphoma is being tested in a phase I/II trial (NCT02253992), while PF-05082566 (anti-CD137) is being tested in a phase Ib trial with pembrolizumab (anti-PD-1) in patients with solid tumors (NCT02179918) (Chester C., et al., Cancer Immunol Immunother Oct;65(10):1243-8 (2016)).

Recently, the effect of multivalent and multispecific fusion polypeptides that bind PD-L1 and CD137 has been evaluated in vitro on T-cell activation and proliferation. Using an autologous in vitro co-culture system implementing immature DC and donor matched T-cells, it has been demonstrated that INBRX-105, a multispecific and multivalent polypeptide having two PD-L1 binding domains, two CD137 binding domains and an Fc region, is superior in stimulating interferon-gamma production, when compared to the monospecific PD-L1 sd-Ab-Fc fusion protein, the CD137 sdAb-Fc fusion protein, the combination of the two, the anti-PD-L1 antibody atezolizumab, the anti-CD137 antibody utomilumab (PF-05082566), or the anti-PD-L1 antibody prembrolizumab, and combinations thereof, at inducing INF$\gamma$ or mediating CD8+ T-cell proliferation and activation (WO 2017/123650). Additionally, WO 2016/149201 discloses certain antibodies directed against PD-L1 and suggests creating bispecific antibody constructs further comprising a T-cell engaging antibody, with CD137 being contained in a non-exclusive list of more than 20 potential T-cell targets.

While immunotherapy combinations have demonstrated their potential to enhance anti-tumor responses through additive or synergistic activity, they are beset with two consistent limitations: 1) clinical development challenges due to the complexity of adjusting the doses of multiple component therapies across various patient cohorts, and 2) reliance on two or more separate manufacturing processes for component therapies, with the attendant implication of high cost of goods sold (COGS) and treatment-pricing. These limitations are even more severe when the number of immunotherapies included in combination regimens increases. Additionally, even for treatment regimens that include only a single immunotherapy, dose-limiting toxicities (DLTs) often preclude administration at maximally effective doses (MEDs) or lead to discontinuation of treatment, resulting in limited efficacy. Unfortunately, similar to their anti-tumor activity, the drug-related toxicities elicited by each component immunotherapy in combination regimens also tend to be additive or synergistic.

Thus, despite the promising opportunities offered by inhibiting the interaction between PD-1 and PD-L1, the applications described above have often resulted in toxicities caused by binding of anti-PD-L1 antibodies to PD-L1 expressed on non-target cells (for a review, see Wang et al., Cancer J. 24 (2018) 36-40).

The exact pathways by which such DLTs arise can vary, but the risk of immunotherapy-related toxicities can typically be minimized, or eliminated, by enhancing the tumor-localization of pharmacological activity. Extratumoral activity of immunotherapies results in the secretion of pro-inflammatory cytokines in healthy tissues, which can result in undesirable safety profiles. Leveraging T cell-engaging bsAbs that require binding to a TAA to elicit immunomodulatory activity is a promising strategy to restrict such cytokine release to cytolytic/immunological synapses between tumor-resident cells and T cells. However, conventional TAA/CD3 bsAbs are also commonly associated with toxicities, such as cytokine release syndrome (CRS), putatively due to excessive activity of anti-CD3 domains. Additionally, while TAA/CD3 bsAbs potently deplete TAA-overexpressing cells, they do so by recruiting and stimulating CTLs whether or not such cells express a T cell receptor (TCR) that recognizes a tumor-antigen(s) (i.e., tumor-reactive T cell). Therefore, rather than stimulating, or reactivating, the host's native anti-tumor immunity, TAA/CD3 bsAbs somewhat indiscriminately stimulate CTLs, potentially posing safety risks and leading to insufficient anti-cancer immune-memory formation.

In addition to CD3, T cell co-stimulatory receptors (e.g., 4-1BB, OX40, ICOS, GITR) are currently being clinically evaluated as targets for therapeutic stimulation of T cells in cancer. One putative advantage of anti-tumor T cell stimulation via such targets is that they are transiently expressed upon TCR signaling. As such, their expression tends to be selectively heightened in inflamed TMEs, particularly on tumor-reactive T cells, whose TCRs are receiving consistent stimulation by dint of abundant interactions with major histocompatibility complexes (MHCs) expressed by malignant cells and antigen-presenting cells (APCs). Therefore, targeting costimulatory receptors with, e.g., mAbs and bsAbs, should more selectively stimulate, and expand, pre-existing anti-tumor T cells than CD3-targeting approaches, potentially rendering such biologics safer and their effects more durable.

Among costimulatory receptors, 4-1BB (CD137, TNF-receptor superfamily 9, TNFRSF9) has emerged as especially promising due to its expression profile and its role as a multipotent mediator of anti-tumor immunity (Bartkowiak and Curran 2015; Yonezawa et al. 2015). 4-1BB is an inducible T cell costimulatory receptor. Its expression is activation-dependent and encompasses a broad subset of immune cells, including activated CD8+ T cells, CD4+ T cells, NK and NKT cells, Tregs, dendritic cells (DC) including follicular DC, stimulated mast cells, differentiating myeloid cells, monocytes, neutrophils, eosinophils (Wang et al, Immunol Rev. 229(1): 192-215 (2009)), and activated B cells (Zhang et al, J Immunol. 184(2):787-795 (2010)). In addition, 4-1BB expression has also been demonstrated on tumor vasculature (Broil K et al., Am J Clin Pathol. 115(4):543-549 (2001); Seaman et al, Cancer Cell 11(6):539-554 (2007)) and atherosclerotic endothelium (Olofsson et al, Circulation 117(10): 1292 1301 (2008)).

4-1BB costimulates T cells to carry out effector functions such as eradication of established tumors, broadening primary CD8+ T cell responses, and enhancing the memory pool of antigen-specific CD8+ T cells. In vivo efficacy studies in mice have revealed that 4-1BB-agonistic mAbs, administered as both a monotherapy and as a component of combination regimens, leads to anti-tumor protective T cell memory responses and tumor regression in multiple tumor models. Additionally, two 4-1BB-agonistic mAbs are currently in the clinic: urelumab (Bristol-Myers Squibb), a fully humanized IgG4 mAb, and utomilumab (PF-05082566, Pfizer), a fully human IgG2 mAb (Chester C., et al., Cancer Immunol Immunother Oct;65(10):1243-8 (2016)). Although utilization of 4-1BB-agonistic mAbs is a very promising treatment strategy, clinical data collected thus far suggest that an mAb-based approach to 4-1BB stimulation results in a trade-off between efficacy and safety. Namely, highly active 4-1BB-agonistic mAbs elicit DLTs that attenuate treatment efficacy, whereas weakly active 4-1BB-agonistic mAbs are well tolerated but do not seem to be highly efficacious, including at their predicted MED.

Highly active 4-1BB-agonistic mAbs lead to alterations in the immune system and organ function, increasing risks of toxicities. High doses of such mAbs in naïve and tumor-bearing mice have been reported to induce T cell-infiltration to the liver and elevations of aspartate aminotransferase and alanine aminotransferase, consistent with liver inflammation (Niu L, et al. J Immunol 178(7):4194-4213 (2007); Dubrot J, et al., Int J Cancer 128(1):105-118 (2011)). Initial clinical studies into the human therapeutic use of 4-1BB-agonistic mAbs have also demonstrated elevations of liver enzymes and increased incidence of hepatitis (Sznol M., et al., J Clin Oncol 26(115S):3007 (2008); Ascierto PA, et al., Semin Oncol 37(5):508-516 (2010); Chester C., et al., Cancer Immunol Immunother Oct;65(10):1243-8 (2016)). Potentially fatal hepatitis was observed in a Bristol-Myers Squibb (BMS) phase II anti-CD137 study for previously treated stage III/IV melanoma, National Clinical Trial (NCT) 00612664. This study and several others (NCT00803374, NCT00309023, NCT00461110, NCT00351325) were terminated due to adverse events (Chester C., et al., Cancer Immunol Immunother Oct;65(10):1243-8 (2016)). Such adverse events are most probably due to systemic overstimulation of T cells.

Similar to TAA/CD3 bsAbs, TAA/4-1BB bsAbs are designed to selectively agonize 4-1BB in the context of immunological synapses between tumor-resident cells and immune-effector cells, thereby preventing the toxicities associated with extratumoral T cell stimulation. As an example, the 5T4/4-1BB bsAb (APV-527) being co-developed by Aptevo Therapeutics and Alligator Biosciences (WO2017182672 A1) is designed to elicit targeted costimulation of T cells by anchoring to 5T4, a TAA expressed by a variety of solid tumors. APV-527 pre-clinical data suggest that conditionally stimulating 4-1BB in the presence of 5T4 effectively tumor-localizes T cell costimulation, leads to considerable enhancement of T cell activation in the TME and inhibits tumor growth in 5T4+ tumor models. This same tumor-localizing strategy can conceivably leverage a variety of clinically validated TAAs, for which therapeutic targeting has been demonstrated to be both efficacious and safe.

HER2 has been established as a TAA that can be targeted effectively and safely to address HER2+ cancers. The most notable HER2-targeted therapies approved for use in patients with HER2+ tumors are the mAbs, trastuzumab (Herceptin®, Genentech) and pertuzumab (Perjeta®, Genentech). While trastuzumab and pertuzumab are similar insofar as they act, in part, by opsonizing HER2+ cells and triggering ADCC, the two antibodies are dissimilar in the means by which they prevent pro-proliferative HER2-signaling. In the case of trastuzumab, binding to its epitope prevents HER2 homodimerization and thereby inhibits HER2-signaling. However, in a subset of patients, compensatory HER3 overexpression, and the formation of HER2/HER3 heterodimers, leads to heightened signaling, rendering such patients refractory to treatment with trastuzumab. By contrast, pertuzumab binds to an epitope that prevents HER2/HER3 heterodimerization, likewise inhibiting pro-proliferative signaling. Due to this mechanism of action (MoA) complementarity, pertuzumab and trastuzumab are synergistic, and their combination has been approved for the treatment of HER2+ breast cancer.

While combined inhibition of HER2-signaling and ADCC-mediated depletion of HER2+ cells has been effective for many patients, many other patients exhibit a HER2+ tumor phenotype that very weakly responds to treatment with conventional antibodies. In some cases, this is because the particular HER2+ tumor does not rely on HER2-signaling for proliferation, rendering the primary MoA of trastuzumab/pertuzumab ineffective. This has engendered the hypothesis that a targeted cytotoxic approach that is more potent than ADCC could be of considerable benefit. Validation of this concept has somewhat emerged with the market approval of the ADC trastuzumab-emtansine (Kadcyla®, Genentech). In that same vein, multiple companies are currently developing HER2/CD3 bsAbs to stimulate redirected T cells to induce potent, targeted cytotoxicity. Additionally, in several patients exhibiting primary or secondary non-responsiveness to HER2-targeting mAbs, the HER2+ tumor phenotype includes heightened expression of ligands/receptors (e.g., PD-L1) that actively suppress anti-tumor immune-responses. Predictably, this has led to the combination of HER2-targeting mAbs with immune-checkpoint-modulating mAbs, which have achieved some success in clinical settings. TAAs that are almost exclusively expressed on cancer cells, such as oncofetal tumor antigens, are referred to as clean TAAs. TAA that are also expressed on normal, non-cancer cells - typically at lower level compared to cancer cells - are non-clean TAAs. Due to the very high potency of TAA/CD3 bsAbs approaches, non-clean TAAs are a challenge as they lead to the depletion of non-tumor cells also expressing the TAA. A well known example of a non-clean TAA is HER2, which is not only expressed on tumor cells but - at a lower level - also in various other tissues. Therefore, novel therapies that improve the selectivity of TAA/CD3 bsAbs approaches for tumor tissues are needed.

There is precedent, as well, for the use of HER2 as a target to tumor-localize 4-1BB stimulation by a bispecific molecule. Pieris Pharmaceuticals has initiated clinical trials to evaluate a HER2/4-1 BB bispecific fusion protein (PRS-343) (NCT03330561). PRS-343 comprises an IgG4 variant of trastuzumab fused to a bivalent 4-1 BB-binding anticalin. Preclinical and clinical evidence supporting: 1) the potential benefits of PD-(L)1 blockade and 4-1 BB stimulation, 2) the benefits of combining HER2-targeting immunotherapies with PD-(L)1-blocking immunotherapies, and 3) the synergy of trastuzumab and pertuzumab, suggests a potential benefit to combining such a HER2/4-1 BB bispecific molecule with as many as two additional immunotherapies in a single treatment. In fact, PRS-343 is also currently being clinically evaluated in combination with the PD-L1-blocking mAb, atezolizumab (Tecentriq®, Genentech) (NCT03650348).

As mentioned previously, an inevitable drawback of combination therapies, particularly as the number of component therapies increases, is that their clinical development can be burdensomely complex and therefore expensive. The requirement to develop multiple manufacturing processes adds further up the development cost and multiplies COGS. The inclusion of more than two specificities in a single molecule (e.g., tri- or tetra-specific antibodies) could theoretically address many of the foregoing limitations with respect to safety, efficacy and cost. Tri-/tetra-specific molecules that are TAA-targeted are theoretically capable of eliciting highly tumor-localized, and synergistic, anti-tumor modulation of multiple immune-checkpoint pathways, which could provide safer and more effective therapies for a variety of cancers. Additionally, such molecules would further limit the need for coadministration of additional immunotherapies to boost patient responses, supporting ease-of-development and minimizing treatment costs. However, implementation of tri-/tetra-specific antibodies for therapeutic use has been complicated due to issues with their molecular architecture, the properties of their component antigen-binding domains and/or poor biophysical properties. Therefore, there remains a clear need for novel tri-/tetra-specific antibodies that elicit tumor-localized, synergistic immunomodulation and that have biophysical properties rendering them suitable for pharmaceutical development.

In addition, despite the fact that numerous antibodies already exist that are specific for tumor-associated immune checkpoint antigens, TAAs and/or T cell co-stimulatory receptor, the complex and specific requirements of such tri- or tetraspecific antibodies require the development of novel antibody domains with tailor-made properties.

Thus, in spite of numerous treatment options for patients suffering from cancer, there remains a need for effective and safe therapeutic agents and a need for their preferential use in a more targeted manner. Immune-modulating biologics offer promising approaches in treatment of cancers due to their modes of actions, however global immunostimulation and lack of any restriction of this immunomodulation to pathologically relevant cells and sites causes numerous side effects and significant toxicities, which potentially may lead to increased morbidity and mortality of patients. It is therefore an object of the present invention to provide a medicament to improve treatment of a proliferative disease, particularly a cancer.

## SUMMARY OF THE INVENTION

It is an object of the present invention to provide a medicament to improve treatment of a proliferative disease, particularly a cancer. The present invention addresses the need for precision therapeutics for immuno-oncology that target only the disease-related cells.

In one aspect, the present invention relates to a multispecific antibody comprising at least a first domain specifically binding to a tumor-associated immune checkpoint antigen with low affinity, and at least a second domain specifically binding to a tumor-associated antigen (TAA).

The present invention further relates to a multispecific antibody comprising at least a first domain specifically binding to a tumor-associated immune checkpoint antigen with low affinity, at least a second domain specifically binding to a tumor-associated antigen (TAA), and at least a third domain specifically binding to an immune cell antigen, in particular wherein said immune cell antigen is present on T cell or NK cell.

More specifically, the present invention relates multispecific antibody wherein said first domain specifically binding to PD-L1 comprises a VH sequence of SEQ ID NO: 11 and a VL sequence of SEQ ID NO: 16.

The present invention further relates to a combination comprising (i) the multispecific antibody of the present invention and (ii) a second compound selected from (iia) an antibody directed at a TAA, in particular an antibody directed at HER2, in particular wherein the antibody is Trastuzumab, (iib) a modulator of an immune checkpoint antigen, in particular wherein said immune checkpoint antigen is not a tumor-associated immune checkpoint antigen, and/or in particular wherein said immune checkpoint antigen is present on T cell or NK cell, and (iic) a modulator of angiogenesis.

In another aspect, the present invention relates to a pharmaceutical composition comprising the multispecific antibody of the invention and a pharmaceutically acceptable carrier.

In a further aspect, the present invention provides the multispecific antibody of the invention or the pharmaceutical composition of the invention for use as a medicament.

In a further aspect, the present invention provides the multispecific antibody of the invention or the pharmaceutical composition of the invention for use in treatment of cancer in a subject in need thereof.

In one aspect, the present invention provides use of the multispecific antibody of the invention or the pharmaceutical composition of the invention for treating cancer in a subject in need thereof.

In one aspect, the present invention provides use of the multispecific antibody of the invention or the pharmaceutical composition of the invention in the manufacture of a medicament for treatment of a cancer, in a subject in need thereof.

In yet another aspect, the present invention provides a method of treating a cancer in a subject in need thereof comprising administering to the subject a therapeutically effective amount of the multispecific antibody of the invention or the pharmaceutical composition of the invention.

In a further aspect, the present invention provides a nucleic acid comprising a nucleotide sequence encoding the multispecific antibody of the invention. In a further aspect, the present invention provides a vector comprising said nucleic acid. In a further aspect, the present invention provides a host cell comprising said nucleic or said vector.

In yet another aspect, the present invention provides a method of producing the multispecific antibody of the invention or a binding domain thereof or a fragment thereof, the method comprising the step of culturing a host cell comprising a nucleic acid or a vector encoding the multispecific antibody of the invention or a binding domain thereof or a fragment thereof.

The aspects, advantageous features and preferred embodiments of the present invention summarized in the following items, respectively alone or in combination, further contribute to solving the object of the invention:

1. A multispecific antibody comprising

(a) a first domain specifically binding to a tumor-associated immune checkpoint antigen wherein said first domain binds to said tumor-associated immune checkpoint antigen with a dissociation constant (KD) of more than 50 nM, particularly with a dissociation constant (KD) of betweem 50 nM and 1 μM, particularly more than 100 nM, particularly with a dissociation constant (KD) of betweem 100 nM and 900 nM, particularly more than 200 nM, particularly with a dissociation constant (KD) of betweem 200 nM and 800 nM, particularly more than 300 nM, particularly with a dissociation constant (KD) of betweem 300 nM and 700 nM, particularly more than 400 nM, particularly with a dissociation constant (KD) of betweem 400 nM and 600 nM, particularly more than 450 nM, particularly with a dissociation constant (KD) of betweem 450 nM and 550 nM, particularly more than 475 nM, particularly with a dissociation constant (KD) of betweem 475 nM and 525 nM, particularly with a dissociation constant (KD) of about 500 nM, in each case when measured by SPR in an scFv format (monovalent affinity), and

(b) a second domain specifically binding to a tumor-associated antigen (TAA).

2. The multispecific antibody of item 1, wherein said second domain binds to said TAA with a dissociation constant (KD) of less than 50 nM, particularly less than 20 nM, particularly less than 10 nM, particularly less than 5 nM, particularly less than 2 nM , particularly less than 1 nM, particularly less than 0.5 nM, when measured by SPR in an scFv format (monovalent affinity).

3. The multispecific antibody of item 1 or item 2, wherein said tumor-associated immune checkpoint antigen and said TAA are both present on the same tumor cell.

4. The multispecific antibody of any one of the preceding items, wherein said tumor-associated immune checkpoint antigen is selected from the group consisting of PD-L1, PD-L2, CD80, CD86, CD276 (B7-H3), and VTCN1 (B7-H4).

5. The multispecific antibody of item 4, wherein said tumor-associated immune checkpoint antigen is PD-L1.

6. The multispecific antibody of any one of the preceding items, wherein said first domain is an inhibitor of said tumor-associated immune checkpoint antigen.

7. The multispecific antibody of any one of the preceding items, wherein said TAA is not PD-L1.

8. The multispecific antibody of any one of the preceding items, wherein said TAA is selected from the group consisting of EGFRvIII, 5T4, CD19, CD20, CD22, CD38, BCMA, IL4RA, mesothelin, GD2, Tn antigen, sTn antigen, Tn-O-Glycopeptides, sTn-O-Glycopeptides, PSMA, CD97, TAG72, CD44v6, CEA, EPCAM, KIT, IL-13Ra2, leguman, GD3, CD171, IL-11Ra, IL-13RA2, ROR1, PSCA, MAD-CT-1, MAD-CT-2, VEGFR2, CLEC12A, LewisY, CD24, PDGFR-beta, SSEA-4, folate receptor alpha, ERBBs (e.g., ERBB2), Her2/neu (HER2), MUC1, MUC16, EGFR, NCAM, Ephrin B2, CAIX, LMP2, sLe, HMWMAA, o-acetyl-GD2, folate receptor beta, TEM1/CD248, CD33, CD123, CD133, CD135, TEM7R, FAP, Legumain, HPV E6 or E7, ML-IAP, CLDN6, TSHR, GPRC5D, ALK, Polysialic acid, Fos-related antigen, neutrophil elastase, TRP-2, CYP1B1, sperm protein 17, beta human chorionic gonadotropin, AFP, thyroglobulin, PLAC1, globoH, RAGE1, MN-CA IX, human telomerase reverse transcriptase, intestinal carboxyl esterase, mut hsp 70-2, NA-17, NY-BR-1, UPK2, HAVCR1, ADRB3, PANX3, NY-ESO-1, GPR20, Ly6k, OR51E2, TARP, GFRalpha4; GPC3, CDH3, B7H3; FGFR1, SSTR2, CECAM6, GA733, and gp120.

9. The multispecific antibody of item 8, wherein said TAA is selected from HER2 and mesothelin, in particular HER2.

10. The multispecific antibody of any one of the preceding items further comprising a third domain specifically binding to an immune cell antigen, in particular wherein said immune cell antigen is present on T cell or NK cell.

11. The multispecific antibody of item 10, wherein said third domain is specifically binding to an immune cell antigen, which is a stimulatory or co-stimulatory molecule of said immune cell.

12. The multispecific antibody of item 11, wherein said third domain is an agonist and said immune cell antigen is a stimulatory immune cell antigen.

13. The multispecific antibody of item 12, wherein said stimulatory immune cell antigen is selected from the group consisting of CD3 and CD16.

14. The multispecific antibody of item 13, wherein said stimulatory immune cell antigen is CD3, in particular CD3ε.

15. The multispecific antibody of item 11, wherein said third domain is an agonist and said immune cell antigen is a co-stimulatory immune cell antigen.

16. The multispecific antibody of item 15, wherein said co-stimulatory immune cell antigen is selected from the group consisting of CD137, CD28, ICOS, HVEM, CD27, OX40, DR3, GITR, CD30, SLAM, CD2, 2B4, TIM1, TIM2, and CD226.

17. The multispecific antibody of item 16, wherein said stimulatory immune cell antigen is CD137.

18. The multispecific antibody of item 17, wherein said third domain specifically binds to CD137 at an epitope comprised in the distal part of the extracellular domain of CD137, particularly within the cysteine-rich domains CRD1 and/or CRD2, more particularly within amino acid residues 24-86 of SEQ ID NO: 130, provided that amino acid residue Asn42 of CD137 is not a critical residue for binding.

19. The multispecific antibody of item 11, wherein said third domain is an inhibitor and said immune checkpoint antigen is an inhibitory immune cell antigen.

20. The multispecific antibody of item 19, wherein said inhibitory immune cell antigen is selected from the group consisting of cytotoxic T-lymphocyte-associated protein 4 (CTLA4), PD-1, lymphocyte-activation gene 3, and T-cell immunoglobulin mucin-3, BTLA, TIM3, TIGIT, CD160, LAG3, LAIR1, B7-1, and B7-H1.

21. The multispecific antibody of any one of the preceding items further comprising a domain specifically binding to human serum albumin (HSA).

22. The multispecific antibody of any one of the preceding items, wherein said domains are capable of binding to their respective antigens simultaneously.

23. The multispecific antibody of any one of the preceding items, wherein said domains are independently selected from the group consisting of a Fab, an Fv, an scFv, dsFv, an scAb, STAB, a single domain antibody (sdAb or dAb), a single domain heavy chain antibody, a single domain light chain antibody, a VHH, and a single domain antibody based on the VNAR structure from shark,.

24. The multispecific antibody of any one of the preceding items, wherein said multispecific antibody is in a format selected from the group consisting of a single-chain diabody (scDb), a tandem scDb (Tandab), a linear dimeric scDb (LD-scDb), a circular dimeric scDb (CD-scDb), a bispecific T-cell engager (BiTE; tandem di-scFv), a tandem tri-scFv, a tribody (Fab-(scFv)2) or bibody (Fab-(scFv)1), Fab, Fab-Fv2, Morrison (IgG CH3-scFv fusion (Morrison L) or IgG CL-scFv fusion (Morrison H)), triabody, scDb-scFv, bispecific Fab2, di-miniantibody, tetrabody, scFv-Fc-scFv fusion, scFv-HSA-scFv fusion, di-diabody, DVD-Ig, COVD, IgG-scFab, scFab-dsscFv, Fv2-Fc, IgG-scFv fusions, such as bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain), Ts2Ab (dsscFv linked to C-terminus of heavy chain), Bispecific antibodies based on heterodimeric Fc domains, such as Knob-into-Hole antibodies (KiHs); scDb, tandem-di-scFv, tandem tri-scFv, Fab-(scFv)2, Fab-(scFv)1, Fab, Fab-Fv2, COVD fused to the N- and/or the C-terminus of either chain of a heterodimeric Fc domain or any other heterodimerization domain, a MATCH and DuoBodies.

25. The multispecific antibody of any one of the preceding items, wherein said antibody does not comprise an immunoglobulin Fc region polypeptide, and, optionally, does not comprise CH1 and/or CL regions.

26. The multispecific antibody of any one of the preceding items, wherein said antibody comprises CH1 and/or CL regions, and optionally comprises an immunoglobulin Fc region polypeptide.

27. The multispecific antibody of any one of the preceding items, wherein said antibody is monovalent for each specificity.

28. The multispecific antibody of any one of the preceding items, wherein said antibody is a scDb-scFv, tribody, DVD-tribody, MATCH, in particular wherein said multispecific antibody is in a MATCH or tribody format, more particularly wherein said multispecific antibody is in a MATCH format, more particularly wherein said multispecific antibody is a MATCH3 or a MATCH4.

29. The multispecific antibody of any one of items 5 to 28, wherein:

   a. said first domain binds to human PD-L1 with a dissociation constant (KD) of 100 nM to 1000 nM, e.g., of 100 nM to 900 nM, of 150 nM to 850 nM, of 200 nM to 800 nM, of 250 nM to 750 nM, of 300 nM to 700 nM, preferably of 350 nM to 650 nM, more preferably of 400 nM to 600 nM, in particular as measured by SPR;

   b. said first domain, when in scFv format, does not bind to cells expressing PD-L1, in particular as determined by flow cytometry, in particular wherein said scFv is at a concentration of less than 100 μg/ml;

   c. said first domain, when in scFv format,

      (i) does not neutralize PD-L1 binding to PD-1, in particular as determined by NFAT reporter gene assay, or
      (ii) neutralizes PD-L1 binding to PD-1 with a potency relative to that of avelumab (relative potency), as determined by NFAT reporter gene assay, of less than 0.001, preferably less than 0.0005, and wherein said relative potency is the ratio of the $IC_{50}$ value in ng/ml of avelumab as measured in the NFAT reporter gene assay to the $IC_{50}$ value in ng/ml of said scFv as measured in the NFAT reporter gene assay;

30. The multispecific antibody of any one of items 5 to 29, wherein said multispecific antibody

   (i) in the presence of TAA-/PD-L1 + cells, has the ability to block interaction between PD-L1 and PD-1 with a potency relative to that of avelumab (relative potency), as determined by flow cytometry assay, of less than 0.001, preferably less than 0.0005, and wherein said relative potency is the ratio of the IC50 value in ng/ml of avelumab as measured in the flow cytometry assay to the IC50 value in ng/ml of said multispecific antibody as measured in flow cytometry assay; and

(ii) in the presence of TAA+/PD-L1 + cells, has the ability to block interaction between PD-L1 and PD-1 with a potency relative to that of avelumab (relative potency), as determined by flow cytometry assay, of more than 0.01, preferably more than 0.05, more preferably more than 0.1, and wherein said relative potency is the ratio of the IC50 value in ng/ml of avelumab as measured in the flow cytometry assay to the IC50 value in ng/ml of said multispecific antibody as measured in flow cytometry assay.

31. The multispecific antibody of any one of items 5 to 30, wherein:

a. said first domain, when in scFv format, has a melting temperature (Tm), determined by differential scanning fluorimetry, of at least 65°C, preferably at least 70°C, in particular wherein said scFv is formulated in 50 mM phosphate-citrate buffer at pH 6.4, 150 mM NaCl;

b. said first domain, when in scFv format, has a loss in monomer content, after four consecutive freeze-thaw cycles, of less than 3 %, preferably less than 1 %, when said scFv is at a starting concentration of 10 mg/ml, in particular wherein said scFv is formulated in 50 mM phosphate citrate buffer with 150 mM NaCl at pH 6.4; and

c. said first domain, when in scFv format, has a loss in monomer content, after storage for at least two weeks, particularly for at least four weeks, at 4°C, of less than 10 %, e.g., less than 9 %, less than 8 %, less than 7 %, less than 6 %, preferably less than 5 %, when said scFv is at a starting concentration of 10 mg/ml, and in particular wherein said scFv is formulated in 50 mM phosphate citrate buffer with 150 mM NaCl at pH 6.4.

32. The multispecific antibody of any one of the preceding items wherein each domain comprises a heavy chain variable region (VH) and a light chain variable region (VL), wherein:

a. said VH comprises, in sequence, the three complementary determining regions HCDR1, HCDR2 and HCDR3, and
b. said VL comprises, in sequence, the three complementary determining regions LCDR1, LCDR2 and LCDR3.

33. The multispecific antibody of any one of the preceding items, wherein said first domain specifically binding to said tumor-associated immune checkpoint antigen, and/or said second domains specifically binding to said TAA, and, optionally, said third domain specifically binding to an immune cell antigen, and, optionally, a further domain specifically binding to human serum albumin (HSA) comprise(s) a light chain variable region (VL) and wherein said VL comprises Vκ frameworks FR1, FR2 and FR3, particularly Vκ1 or Vκ3 FR1 to FR3, preferably Vκ1 FR1 to FR3, and a framework FR4, which is selected from a Vκ FR4, particularly Vκ1 FR4, Vκ3 FR4, and Vλ FR4, particularly Vλ FR4 comprising the amino acid sequence having at least 80, particularly at least 90 percent identity to an amino acid sequence selected from any of SEQ ID NO: 123 to SEQ ID NO: 129, preferably Vλ FR4 as set forth in any of SEQ ID NO: 123 to SEQ ID NO: 129, preferably Vλ FR4 as set forth in SEQ ID NO: 123 or 124, more preferably Vλ FR4 as set forth in SEQ ID NO: 124.

34. The multispecific antibody of any one of the preceding items, wherein said first domain comprises HCDR1, HCDR2, and HCDR3 sequences of SEQ ID NOs: 1, 2, and 3, respectively, and LCDR1, LCDR2, and LCDR3 sequences of SEQ ID NOs: 5, 6, and 7, wherein one or more of said CDR sequences optionally comprises one or two mutations, particularly mutations to an alanine residue, more particularly wherein: (i) said LCDR3 comprises Q108A (according to AHo numbering), (ii) said LCDR3 comprises G109A (according to AHo numbering), or (iii) said LCDR3 comprises Q108A and G109A (according to AHo numbering), and/or (iv) said HCDR3 comprises Y112A (according to AHo numbering).

35. The multispecific antibody of item 34, wherein said first domain comprises HCDR1, HCDR2, and HCDR3 sequences of SEQ ID NOs: 1, 2, and 3, respectively, and LCDR1, LCDR2, and LCDR3 sequences of SEQ ID NOs: 5, 6, and 9.

36. The multispecific antibody of item 34, wherein said first domain comprises HCDR1, HCDR2, and HCDR3 sequences of SEQ ID NOs: 1, 2, and 3, respectively, and LCDR1, LCDR2, and LCDR3 sequences of SEQ ID NOs: 5, 6, and 10.

37. The multispecific antibody of item 35 or item 36, wherein said first domain comprises a heavy chain variable region (VH) and wherein said VH is VH3 or VH4, preferably VH4.

38. The multispecific antibody of item 35, wherein said antibody comprises a VH comprising an amino acid sequence that is at least 90 percent, in particular at least 95 percent identical to the amino acid sequence SEQ ID NO: 11; and a VL comprising an amino acid sequence that is at least 90 percent, in particular at least 95 percent identical to the amino acid sequence SEQ ID NO: 15.

39. The multispecific antibody of item 38 comprising a VH sequence of SEQ ID NO: 11 and a VL sequence of SEQ ID NO: 15.

40. The multispecific antibody of item 36, wherein said antibody comprises a VH comprising an amino acid sequence that is at least 90 percent, in particular at least 95 percent identical to the amino acid sequence SEQ ID NO: 11; and a VL comprising an amino acid sequence that is at least 90 percent, in particular at least 95 percent identical to the amino acid sequence SEQ ID NO: 16.

41. The multispecific antibody of item 40, comprising a VH sequence of SEQ ID NO: 11 and a VL sequence of SEQ ID NO: 16.

42. The multispecific antibody of any one of the preceding items, wherein said second domain comprises (i) HCDR1, HCDR2, and HCDR3 sequences of SEQ ID NOs: 17, 18 and 19, respectively, and LCDR1, LCDR2, and LCDR3 sequences of SEQ ID NOs: 20, 21 and 22, or, in particular, (ii) HCDR1, HCDR2, and HCDR3 sequences of SEQ ID NOs: 27, 28 and 29, respectively, and LCDR1, LCDR2, and LCDR3 sequences of SEQ ID NOs: 30, 31 and 32, wherein one or more of said CDR sequences optionally comprises one or two mutations, particularly mutations to an alanine residue.

43. The multispecific antibody of item 42, wherein said second domain comprises a VH comprising an amino acid sequence that is (i) at least 90 percent, particularly at least 95 percent identical to the amino acid sequence SEQ ID NO: 23; and a VL comprising an amino acid sequence that is at least 90 percent, particularly at least 95 percent identical to the amino acid sequence SEQ ID NO: 25, in particular wherein said VH comprises Cys at the position 51, and said VL comprises Cys at the position 141 (AHo numbering), or, in particular, (ii) at least 90 percent, particularly at least 95 percent identical to the amino acid sequence SEQ ID NO: 33; and a VL comprising an amino acid sequence that is at least 90 percent, particularly at least 95 percent identical to the amino acid sequence SEQ ID NO: 35, in particular wherein said VH comprises Cys at the position 51, and said VL comprises Cys at the position 141 (AHo numbering).

44. The multispecific antibody of item 43 comprising (i) a VH sequence of SEQ ID NO: 24 and a VL sequence of SEQ ID NO: 26, or (ii) a VH sequence of SEQ ID NO: 34 and a VL sequence of SEQ ID NO: 36, particularly a VH sequence of SEQ ID NO: 34 and a VL sequence of SEQ ID NO: 36.

45. The multispecific antibody of any one of items 10 to 44, wherein said third domain (i) is directed at CD3, in particular wherein said third domain comprises HCDR1, HCDR2, and HCDR3 sequences of SEQ ID NOs: 37, 38 and 39, respectively, and LCDR1, LCDR2, and LCDR3 sequences of SEQ ID NOs: 40, 41 and 42, or (ii) is directed at CD137, in particular wherein said third domain comprises HCDR1, HCDR2, and HCDR3 sequences of SEQ ID NOs: 61, 62 and 63, respectively, and LCDR1, LCDR2, and LCDR3 sequences of SEQ ID NOs: 64, 65 and 66, wherein one or more of said CDR sequences optionally comprises one or two mutations, particularly mutations to an alanine residue,.

46. The multispecific antibody of item 45, wherein said third domain (i) is directed at CD3 and comprises a VH comprising an amino acid sequence that is at least 90 percent, in particular at least 95 percent identical to the amino acid sequence SEQ ID NO: 43; and a VL comprising an amino acid sequence that is at least 90 percent, in particular at least 95 percent identical to the amino acid sequence SEQ ID NO: 44, or (ii) is directed at CD137 and comprises a VH comprising an amino acid sequence that is at least 90 percent, in particular at least 95 percent identical to the amino acid sequence SEQ ID NO: 67; and a VL comprising an amino acid sequence that is at least 90 percent, in particular at least 95 percent identical to the amino acid sequence SEQ ID NO: 68.

47. The multispecific antibody of item 46 (i) is directed at CD3 and comprises a VH sequence of SEQ ID NO: 43 and a VL sequence of SEQ ID NO: 44, or (ii) is directed at CD137 and comprises a VH sequence of SEQ ID NO:

67 and a VL sequence of SEQ ID NO: 68.

48. The multispecific antibody of any one of items 21 to 28, wherein said domain specifically binding to HSA comprises (i) HCDR1, HCDR2, and HCDR3 sequences of SEQ ID NOs: 45, 46 and 47, respectively, and LCDR1, LCDR2, and LCDR3 sequences of SEQ ID NOs: 48, 49 and 50, or (ii) HCDR1, HCDR2, and HCDR3 sequences of SEQ ID NOs: 53, 54 and 55, respectively, and LCDR1, LCDR2, and LCDR3 sequences of SEQ ID NOs: 56, 57 and 58, wherein one or more of said CDR sequences optionally comprises one or two mutations, particularly mutations to an alanine residue.

49. The multispecific antibody of item 48, wherein said domain specifically binding to HSA comprises (i) a VH comprising an amino acid sequence that is at least 90 percent, in particular at least 95 percent identical to the amino acid sequence SEQ ID NO: 51; and a VL comprising an amino acid sequence that is at least 90 percent, in particular at least 95 percent identical to the amino acid sequence SEQ ID NO: 52, or (i) a VH comprising an amino acid sequence that is at least 90 percent, in particular at least 95 percent identical to the amino acid sequence SEQ ID NO: 59; and a VL comprising an amino acid sequence that is at least 90 percent, in particular at least 95 percent identical to the amino acid sequence SEQ ID NO: 60.

50. The multispecific antibody of item 49 comprising (i) a VH sequence of SEQ ID NO: 51 and a VL sequence of SEQ ID NO: 52, or (ii) a VH sequence of SEQ ID NO: 59 and a VL sequence of SEQ ID NO: 60.

51. The multispecific antibody of any of the preceding items, wherein the antibody comprises a combination of two chains, each having an amino acid sequence having at least 80 % identity, particularly at least 90 % identity, more particularly at least 95 % identity, including 100 % identity (i) to the sequences of a combination of chains selected from SEQ ID NOs: 69 and 70; SEQ ID NOs: 71 and 72, SEQ ID NOs: 73 and 74, SEQ ID NOs: 75 and 76, SEQ ID NOs: 77 and 78, SEQ ID NOs: 79 and 80, SEQ ID NOs: 81 and 82, SEQ ID NOs: 83 and 84, SEQ ID NOs: 85 and 86, SEQ ID NOs: 87 and 88, SEQ ID NOs: 89 and 90, SEQ ID NOs: 91 and 92, SEQ ID NOs: 93 and 94, SEQ ID NOs: 95 and 96, SEQ ID NOs: 97 and 98, SEQ ID NOs: 99 and 100, SEQ ID NOs: 101 and 102, SEQ ID NOs: 103 and 104, and SEQ ID NOs: 113 and 114, or to the combination of sequences comprised in one of the sequences selected from SEQ ID NOs: 105 to 114, in particular the combination of chains of SEQ ID NOs: 113 and 114, and wherein the antibody comprises

(i) a first domain specifically binding PD-L1 comprising HCDR1, HCDR2, and HCDR3 sequences of SEQ ID NOs: 1, 2, and 3, respectively, or of SEQ ID NOs: 1, 2, and 4, respectively, and LCDR1, LCDR2, and LCDR3 sequences of SEQ ID NOs: 5, 6, and 7, respectively, SEQ ID NOs: 5, 6, and 8, respectively, SEQ ID NOs: 5, 6, and 9, respectively, or, in particular SEQ ID NOs: 5, 6, and 10, respectively, and
(ii) a second domain specifically binding HER2 comprising (i) HCDR1, HCDR2, and HCDR3 sequences of SEQ ID NOs: 17, 18 and 19, respectively, and LCDR1, LCDR2, and LCDR3 sequences of SEQ ID NOs: 20, 21 and 22, respectively, or, in particular, (ii) HCDR1, HCDR2, and HCDR3 sequences of SEQ ID NOs: 27, 28 and 29, respectively, and LCDR1, LCDR2, and LCDR3 sequences of SEQ ID NOs: 30, 31 and 32, respectively
(iii) optionally, a third domain specifically binding (i) CD3 comprising HCDR1, HCDR2, and HCDR3 sequences of SEQ ID NOs: 37, 38 and 39, respectively, and LCDR1, LCDR2, and LCDR3 sequences of SEQ ID NOs: 40, 41 and 42, respectively, or (ii) CD137 comprising HCDR1, HCDR2, and HCDR3 sequences of SEQ ID NOs: 61, 62 and 63, respectively, and LCDR1, LCDR2, and LCDR3 sequences of SEQ ID NOs: 64, 65 and 66, respectively,
(iv) optionally, a further domain specifically binding HSA comprising (i) HCDR1, HCDR2, and HCDR3 sequences of SEQ ID NOs: 45, 46 and 47, respectively, and LCDR1, LCDR2, and LCDR3 sequences of SEQ ID NOs: 48, 49 and 50, respectively, or (ii) HCDR1, HCDR2, and HCDR3 sequences of SEQ ID NOs: 53, 54 and 55, respectively, and LCDR1, LCDR2, and LCDR3 sequences of SEQ ID NOs: 56, 57 and 58, respectively.

52. A combination comprising (i) the multispecific antibody of any one of items 1 to 51 and (ii) a second compound selected from (iia) an antibody directed at a TAA, in particular an antibody directed at HER2, in particular wherein the antibody is Trastuzumab, (iib) a modulator of an immune checkpoint antigen, in particular wherein said immune checkpoint antigen is not a tumor-associated immune checkpoint antigen, and/or in particular wherein said immune checkpoint antigen is present on T cell or NK cell, and (iic) a modulator of angiogenesis.

53. The combination of item 52, wherein said modulator is an antibody.

54. The combination of item 52 or 53, wherein said modulator is an agonist and said immune checkpoint antigen is an immune cell antigen.

55. The combination of item 54, wherein said immune cell antigen is selected from the group consisting of CD28, ICOS, HVEM, CD27, OX40, DR3, GITR, CD30, SLAM, CD2, 2B4, TIM1, TIM2, CD226, CTLA4, PD-1, lymphocyte-activation gene 3, and T-cell immunoglobulin mucin-3, BTLA, TIM3, TIGIT, CD160, LAG3, LAIR1, B7-1, and B7-H1.

56. The combination of item 55, wherein said stimulatory immune cell antigen is CD3 or CD137, in particular CD3.

57. The combination of item 52 or 53, wherein said modulator is an inhibitor and said immune cell antigen is an inhibitory immune checkpoint antigen.

58. The combination of item 57, wherein said inhibitory immune cell antigen is selected from the group consisting of cytotoxic T-lymphocyte-associated protein 4 (CTLA4), PD-1, lymphocyte-activation gene 3, and T-cell immunoglobulin mucin-3, preferably wherein said inhibitory immune checkpoint antigen is cytotoxic T-lymphocyte-associated protein 4 (CTLA4), more preferably wherein said modulator is ipilimumab.

59. A combination comprising (i) the multispecific antibody of any one of items 1 to 51 and (ii) an antibody directed against a TAA.

60. The combination of item 59, wherein said TAA is selected from HER2 and mesothelin, particularly HER2, particularly wherein said antibody is Trastuzumab.

61. A pharmaceutical composition comprising the multispecific antibody of any one of items 1 to 51, or the combination of any one of items 52 to 60, and a pharmaceutically acceptable carrier.

62. A PD-L1 -binding domain as defined in any one of items 29, 30, and 34 to 41.

63. A HER2-binding domain as defined in any one of items 42 to 44.

64. A CD3-binding domain as defined in any one of items 45(i) to 47(i).

65. A CD137-binding domain as defined in any one of items 45(ii) to 47(ii).

66. An HSA-binding domain as defined in any one of items 48 to 50.

67. The multispecific antibody of any one of items 1 to 51, the combination of any one of items 52 to 60, or a binding domain of any one of items 62 to 66 for use as a medicament.

68. The multispecific antibody of any one of items 1 to 51, the combination of any one of items 52 to 60, the pharmaceutical composition of item 61, or a binding domain of any one of items 62 to 66 for use in treatment of a cancer in a subject in need thereof.

69. Use of the multispecific antibody of any one of items 1 to 51, the combination of any one of items 52 to 60, the pharmaceutical composition of item 61, or a binding domain of any one of items 62 to 66 for treating a cancer in a subject in need thereof.

70. Use of the multispecific antibody of any one of items 1 to 51, the combination of any one of items 52 to 60, the pharmaceutical composition of item 61, or a binding domain of any one of items 62 to 66 in the manufacture of a medicament for treatment of a cancer, in a subject in need thereof.

71. A method of treating a cancer in a subject in need thereof comprising administering to the subject a therapeutically effective amount of the multispecific antibody of any one of items 1 to 51, 67 and 68, the combination of any one of items 52 to 60, the pharmaceutical composition of item 61, or a binding domain of any one of items 62 to 66, or the use of items 69 or 70, wherein said cancer is a cancer positive for said TAA and said tumor-associated immune checkpoint antigen, in particular wherein said cancer is TAA$^+$/PDL$^+$, more particularly wherein said cancer is HER2$^+$/PD-L1$^+$.

72. The multispecific antibody of item 68, the use of items 69 or 70, or the method of item 71, wherein said cancer is a cancer positive HER2 and PD-L1, and wherein said cancer is refractory to a standard of care therapy, in particular to trastuzumab.

73. A nucleic acid encoding the multispecific antibody of any one of items 1 to 51 or a binding domain of any one of items 62 to 66.

74. A vector comprising the nucleic acid of item 73.

75. A host cell comprising the nucleic acid of item 73 or the vector of item 74.

76. A method of producing the multispecific antibody of any one of items 1 to 51, a binding domain of any one of items 62 to 66, the method comprising the step of culturing a host cell comprising a nucleic acid or a vector encoding the multispecific antibody of any one of items 1 to 51, a binding domain of any one of items 62 to 66.

77. A kit comprising the multispecific antibody of any one of items 1 to 51, the combination of any one of items 52 to 60, the pharmaceutical composition of item 61, or a binding domain of any one of items 62 to 66.

78. The multispecific antibody of any of items 1 to 50, wherein the antibody comprises a combination of two chains a combination of chains selected from SEQ ID NOs: 69 and 70; SEQ ID NOs: 71 and 72, SEQ ID NOs: 73 and 74, SEQ ID NOs: 75 and 76, SEQ ID NOs: 77 and 78, SEQ ID NOs: 79 and 80, SEQ ID NOs: 81 and 82, SEQ ID NOs: 83 and 84, SEQ ID NOs: 85 and 86, SEQ ID NOs: 87 and 88, SEQ ID NOs: 89 and 90, SEQ ID NOs: 91 and 92, SEQ ID NOs: 93 and 94, SEQ ID NOs: 95 and 96, SEQ ID NOs: 97 and 98, SEQ ID NOs: 99 and 100, SEQ ID NOs: 101 and 102, SEQ ID NOs: 103 and 104, and SEQ ID NOs: 113 and 114, or to the combination of sequences comprised in one of the sequences selected from SEQ ID NOs: 105 to 114, in particular the combination of chains of SEQ ID NOs: 113 and 114.

## BRIEF DESCRIPTION OF THE DRAWINGS

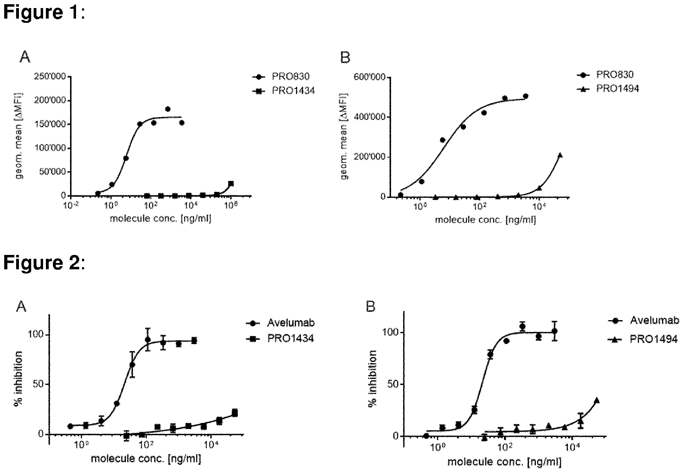

FIG. 1 Binding to PD-L1 expressing cells assessed by flow cytometry. Binding of (A) PRO1434 and (B) PRO1494 to PD-L1 expressing cells. PRO830 was used as a reference. PRO1434 showed a signal only at 100 $\mu$g/ml whereas binding was observed with 3.5 $\mu$g/ml for PRO1494.

FIG. 2 Blockade of PD-1/PD-L1 interaction in NFAT reporter gene assay. PD-L1 neutralization by (A) PRO1434 and (B) PRO1494. Avelumab was used as a reference. For both molecules tested only a partial neutralization of PD-L1 interaction with PD-1 was observed with the highest concentration (162 $\mu$g/ml) tested.

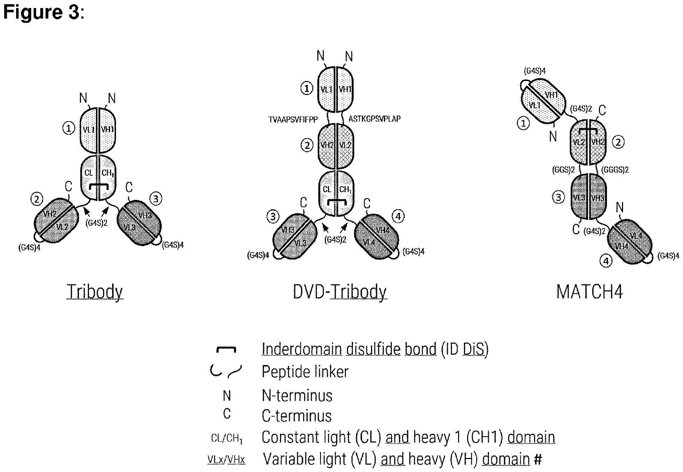

FIG. 3 Architecture of the multispecific molecules. Schematic representation and description of the three different multispecific formats Tribody, DVD-Tribody and MATCH-4. Table 14 describes the domains comprised in the different molecules produced and their positioning within the molecules. The targets for the different domains are: Trastuzumab: Her2; clone 14-11-D07: IL23R; clone 23-13-A01: human/mouse serum albumin; clone 28-21-D09: CD3e; clone 33-02-G02 and mutants thereof: PD-L1. Gly-Ser linker sequences connecting individual domains are indicated in the Figure.

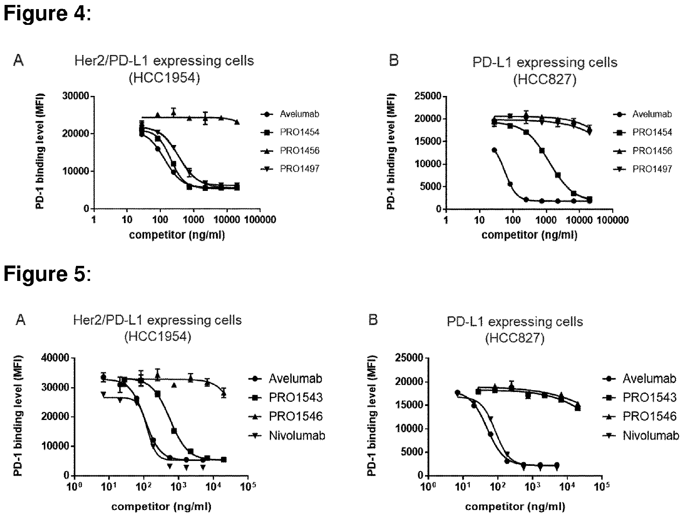

FIG. 4 Blockade of PD-1/PD-L1 interaction on Her2 expressing cells. Inhibition of PD-1 binding to (A) cells expressing PD-L1 and Her2 (HCC1954) or (B) cells expressing PD-L1 without significant expression of Her2 (HCC827) in presence of increasing concentrations of Avelumab, PRO1454, PRO1456 and PRO1497. PRO1454 inhibits PD-1 binding to PD-L1 on PD-L1/high Her2 expressing cells (HCC1954) with an $IC_{50}$ of 205 ng/ml as well as on PD-L1 expressing cells (HCC827) with an $IC_{50}$ of 1204 ng/ml. PRO1497 containing the anti-PD-L1 domain with a 50-fold lower affinity inhibited PD-1 binding to Her2/PD-L1 expressing cells with comparable potency as PRO1454 but showed only very weak inhibition of PD-1 binding to cells expressing only PD-L1 at the concentrations tested. PRO1456 which does not contain an anti-PD-L1 domain did not affect PD-1 binding on both cell lines. Data were fitted using sigmoidal 4PL fit (GraphPad Prism).

FIG. 5 Blockade of PD-1/PD-L1 interaction on Her2 expressing cells in presence of human serum albumin. A) Inhibition of PD-1 binding to cells expressing PD-L1 and Her2 (HCC1954) in presence of increasing concentrations of Avelumab, Nivolumab, PRO1543 (Her2xCD3xhSAxPD-L1 low affinity) and PRO1546 (HER2xCD3xhSAxIL23R). B) Inhibition of PD-1 binding to cells expressing PD-L1 without significant expression of Her2 (HCC827). PRO1543 inhibits PD-1 binding to PD-L1 exclusively on PD-L1 /high Her2 expressing cells with an $IC_{50}$ value of 600 ng/ml. PRO1546 which does not contain an anti-PD-L1 domain did not affect PD-1 binding on both cell lines. Data were fitted using sigmoidal 4PL fit (GraphPad Prism).

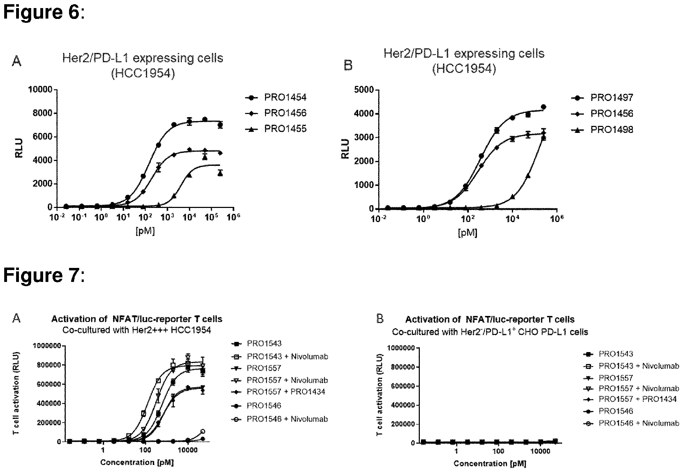

**FIG. 6** CD3 activation and concomitant PD-L1 blockade by PRO1454 or PRO1497 assessed in the NFAT-Luciferase reporter gene assay in presence of human serum albumin. A) In the presence of PD-L1/ Her2 expressing cells (HCC1954), PRO1454 and PRO1456 activated CD3 signaling in Jurkat cells with similar $EC_{50}$ but the maximal activation was higher for PRO1454, a molecule carrying the low affinity anti-PD-L1 domain, compared to PRO1456 containing an anti-IL23R dummy domain instead of the anti-PD-L1 domain. This suggests that PRO1454 simultaneously blocks PD-L1 and activates CD3 within the immunological synapse in presence of cells co-expressing Her2 and PD-L1. Weaker activation was observed with PRO1455, a molecule carrying no anti-Her2 domain but the low affinity anti-PD-L1 domain (33-03-G02 G109A). B) The Tribody molecule PRO1497 containing the anti-PD-L1 domain carrying two alanine mutations (Q108A and G109A) with at least 50 fold lower affinity than the domain incorporated in molecules tested in A and the corresponding controls were tested. For those molecules simultaneous blockade of PD-L1 and activation of CD3 was observed, since PRO1497, containing the low affinity anti-PD-L1 domain, induced a higher maximal activation than PRO1456 containing the anti-IL23R domain instead. In comparison to PRO1455, PRO1498 triggered a much weaker activation due to the much lower affinity of the incorporated anti-PD-L1 domain. Luminescence was read 5h after addition of Jurkat reporter cells and data were fitted using sigmoidal 4PL fit (GraphPad Prism).

**FIG. 7** CD3 activation and concomitant PD-L1 blockade by PRO1543 assessed in the NFAT-Luciferase reporter gene assay in presence of human serum albumin. A) In the presence of PD-L1/ Her2 expressing cells (HCC1954), PRO1543 and PRO1557 activated CD3 signaling in Jurkat cells with similar $EC_{50}$ but the maximal activation was higher for PRO1543, a molecule carrying the low affinity anti-PD-L1 domain, compared to PRO1557 containing an anti-IL23R dummy domain instead of the anti-PD-L1 domain. This suggests that PRO1543 simultaneously blocks PD-L1 and activates CD3 within the immunological synapse in presence of cells co-expressing Her2 and PD-L1. This observation was further supported by the addition of 1 $\mu$g/ml Nivolumab to all molecules resulting in similar maximal activation in presence of PRO1557 and PRO1543 as for PRO1543 alone confirming complete PD-L1/PD-1 blockade by PRO1543. No activation was observed with PRO1546, molecule carrying no anti-Her2 domain but the low affinity anti-PD-L1 domain. B) No activation was observed for the molecules carrying an anti-PD-L1 domain (PRO1543 and PRO1546) independently of the presence of the anti-Her2 domain in presence of PD-L1 expressing CHO cells. No activation was seen with PRO1557 since this molecule does not contain an anti-PD-L1 domain. Luminescence was read 5h after addition of Jurkat reporter cells and data were fitted using sigmoidal 4PL fit (Graph-Pad Prism).

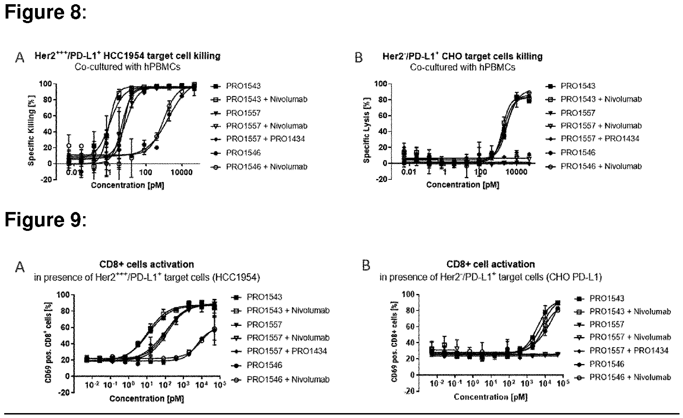

**FIG. 8** Cytotoxic activity of PRO1543, PRO1546 and PRO1557 after 40 h incubation in presence of human serum albumin. (A) On cells expressing PD-L1 and high Her2 levels the best potency (1.01 pM) was observed with PRO1543 (MATCH-4: PD-L1$_{low\ affinity}$xHer2xCD3xhSA) carrying the low affinity PD-L1 and the anti-Her2 domains. 7-fold lower potency (7.3 pM) was observed for PRO1557 (MATCH-4: IL23RxHer2xCD3xhSA) molecule containing no anti-PD-L1 domain. PRO1546 (MATCH-4: PD-L1low affinityxIL23RxCD3xhSA), which does not contain an anti-Her2 domain, was about 1'800 fold less potent than PRO1543. No effect was observed with the addition of 1 $\mu$g/ml of the PD-1 blocker Nivolumab indicating that the increased potency of PRO1543 is strictly due to avidity binding of the anti-PD-L1 domain. (B) In presence of PD-L1$^+$/Her2$^-$ expressing target cells, cytotoxic potency was drastically reduced. Target cells were analyzed by flow cytometry 40 h after the beginning of their incubation with the respective molecules and data were fitted using sigmoidal 4PL fit (GraphPad Prism). Similar data were obtained after 16 h.

**FIG. 9** Effect of PRO1543 on CD8+ T cell activation. After 40 h incubation of the following target cells (A) PD-L1/high Her2 expressing cells (HCC1954), (B) PD-L1$^+$/Her2$^-$ expressing cells (CHO-PD-L1) with freshly isolated human PBMCs, cells were stained with fluorescently labelled anti-CD8 and anti-CD69 antibodies to quantify activtated CD8+ T cells. In correlation with the cytotoxic effects observed, PRO1543 showed the best potency on HCC1954 cells with 12.1 pM. The second Her2 containing molecule (PRO1557) had a 10-fold reduced potency. The Her2 negative molecule which contains the anti-PD-L1 domain (PRO1546) had the lowest potency (below 5.5 nM). On Her2 negative/PD-L1 positive cells T cell activation by PRO1543 was reduced by about 200fold compared to PD-L1 /high Her2 expressing cells (HCC1954), while activation by PRO1546 remained comparably low with both cell lines.

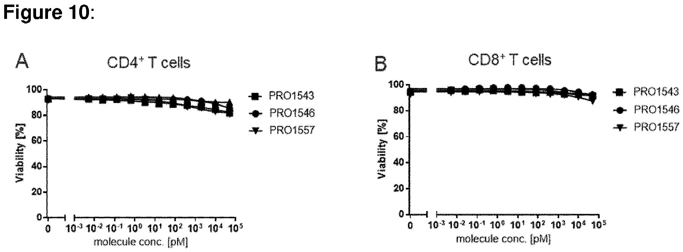

**FIG. 10** Viability of CD4+ and CD8+ T cells. CD4+ T cells (A) and CD8+ T cell (B) viability was only reduced by 5 to 10 % at the highest concentrations of molecules tested. CD4+ T cells and CD8+ T cells were stained by fluorescently labelled antibodies 40h after the start of the cytotoxicity assessment of PBMCs in presence of PD-L1 /high Her2 expressing cancer cells (HCC1954) and analyzed by flow cytometry. Similar data were obtained after 16 h.

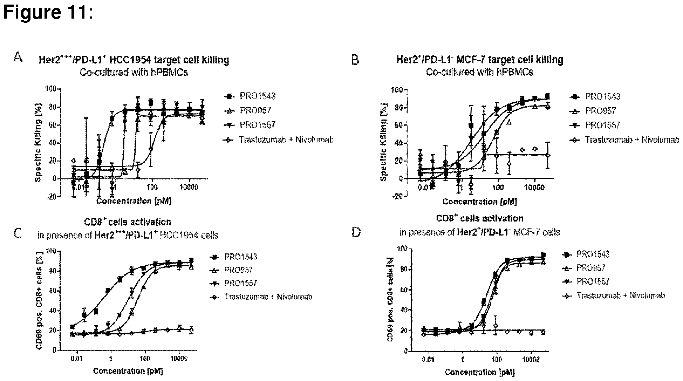

**FIG. 11** T cell mediated target cell killing and CD8+ cell activation in presence of A) Her2$^{+++}$/PD-L1$^+$ HCC1954 and B) Her2$^+$/PD-L1$^-$ MCF-7. In this assay, freshly isolated human PBMCs were co-cultured for 16 h with indicated target cells in presence of the different molecules tested. PRO1543 showed a 50 to 100-fold better potency than the Her2/CD3 scDb (PRO957) on Her2$^{+++}$/PD-L1$^+$ as compared to PD-L1 negative cells were only a slightly different potency is observed. As the $EC_{50}$ of PD-L1 blockade in these cells is considerably higher than the $EC_{50}$ of target cell lysis, it is highly likely that the improved activity of the molecule containing an anti-PD-L1 domains results from increased avidity. As a consequence, binding to Her2 and PD-L1 double positive cells is stronger than binding to PD-L1 negative cells expressing Her2. This avidity binding increases the therapeutic window as it selectively improves potency on tumor cells (Her2/PD-L1 double positive), but not on PD-L1 negative Her2 expressing normal cells.

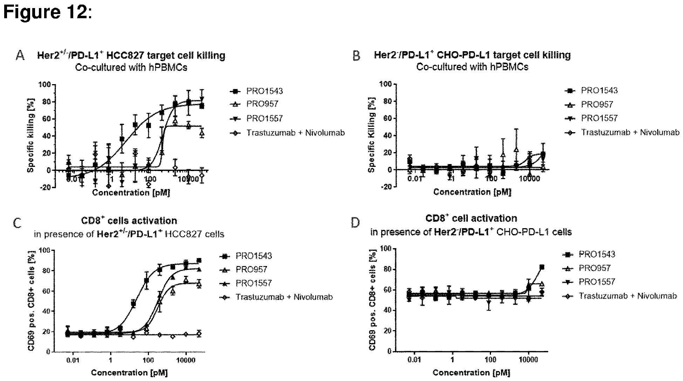

**FIG. 12** T cell mediated target cell killing and CD8+ cell activation in presence of A) Her2$^{+/-}$/PD-L1$^+$ HCC827 and B) Her2$^-$/PD-L1$^+$ CHO PD-L1 cells. Freshly isolated human PBMCs were co-cultured for 16 h with indicated target cells in presence of the different molecules. PRO1543 showed a 20-fold better potency than the Her2/CD3 scDb on Her2$^{+/-}$/PD-L1$^+$. In presence of Her2 negative PD-L1 positive cells only a minor target cell killing and CD8+ cell activation was observed at high concentrations of the MATCH4 harboring the low affinity PD-L1 domain providing a very large therapeutic window.

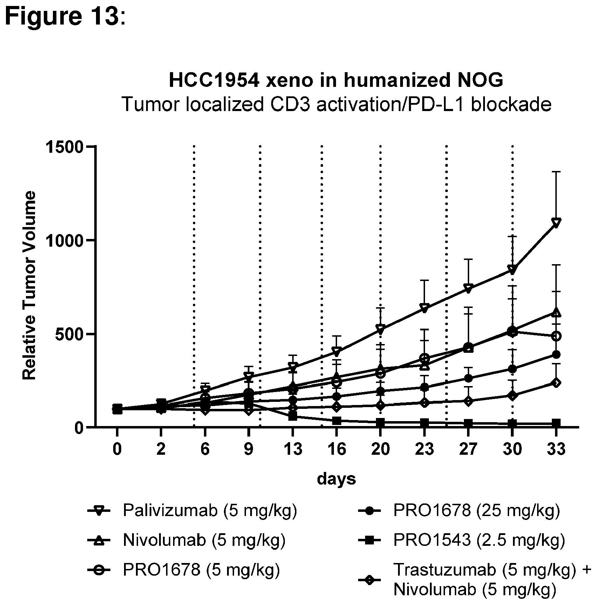

**FIG. 13** Human PBMC-substituted NOG mice were engrafted with HCC1954 ductal breast carcinoma cells (n=8 each). Mice were treated on day 0, 5, 10, 15, 20, 25 and 30 (dotted vertical lines). Tumor growth and body weight were recorded twice weekly. PRO1678 scMATCH3 had similar antitumoral effect as Nivolumab showing efficient tumor targeted PD-L1 blockade. PRO1543 MATCH4 therapy elicited greater antitumoral efficacy than Nivolumab/Trastuzumab combination.

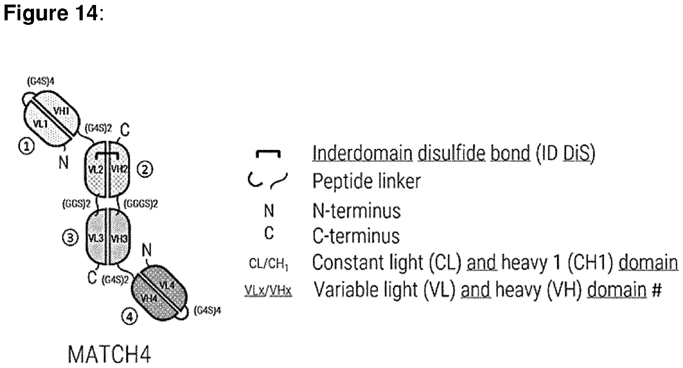

**FIG. 14** Design of the multispecific molecules. Schematic representation and description of the three different multispecific formats tribody, DVD-tribody and MATCH-4. Table 25 describes the domain composition of the different molecules produced and their positioning within the molecules. The targets for the different domains are: Trastuzumab: Her2; clone 14-11-D07: IL23R; clone 23-13-A01: human/mouse SA; clone 28-21-D09: CD3e; clone 33-02-G02 and mutants thereof: PD-L1. Gly-Ser linker sequences connecting individual domains are indicated in the Figure.

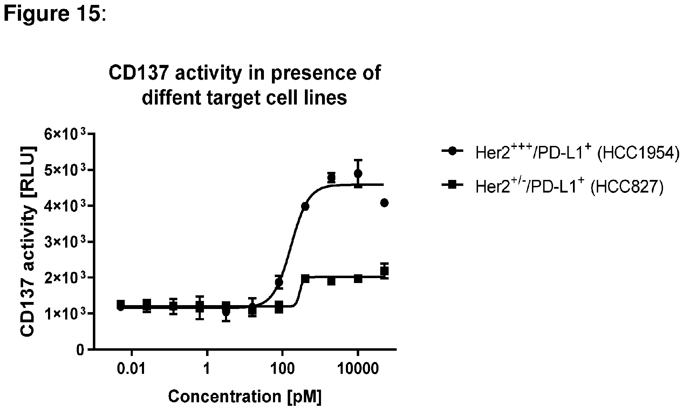

**FIG. 15** Effect of PRO1993 on CD137 signal activity in NF-kB Jurkat reporter cells. After 24h incubation of Jurkat cells with PRO1993 in presence of HCC1954 (high levels of expression for Her2 and PD-L1) and HCC827 (low levels of expression for Her2 but high levels for PD-L1), the activity of CD137 signaling was assessed by detection of luminescence. PRO1993 activated CD137 signaling in the presence of high Her2 expressing HCC1954 cells, whereas in presence of low levels of Her2 expressing HCC827 only a slight activation of CD137 signaling was observed. Data were fitted using sigmoidal 4PL fit (GraphPad Prism).

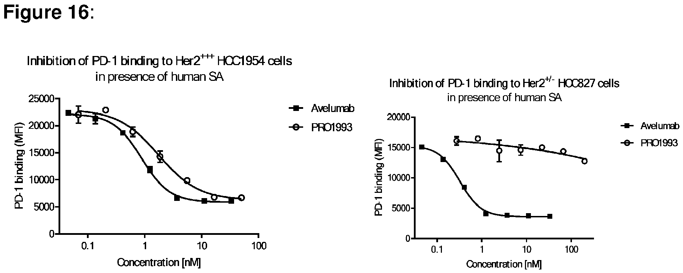

**FIG. 16** Blockade of PD-1/PD-L1 interaction on Her2 expressing cells in presence of human serum albumin. A) PD-1 binding level to cells expressing PD-L1 and high Her2 levels (HCC1954) or (B) cells expressing PD-L1 and a lower level of Her2 (HCC827) in presence of increasing concentrations of Avelumab and PRO1993 (Her2xCD137xhSAxPD-L1$_{low\ affinity}$). PRO1993 inhibits PD-1 binding to PD-L1 on PD-L1/high Her2 expressing cells with an IC50 value of 166.7 ng/ml whereas no inhibition of PD-1 binding was found on HCC827 cells. For comparison, Avelumab inhibit this interaction with an IC50 of 127.7 ng/ml (HCC1954) and 46.03 ng/ml (HCC827). Data were fitted using sigmoidal 4PL fit (GraphPad Prism).

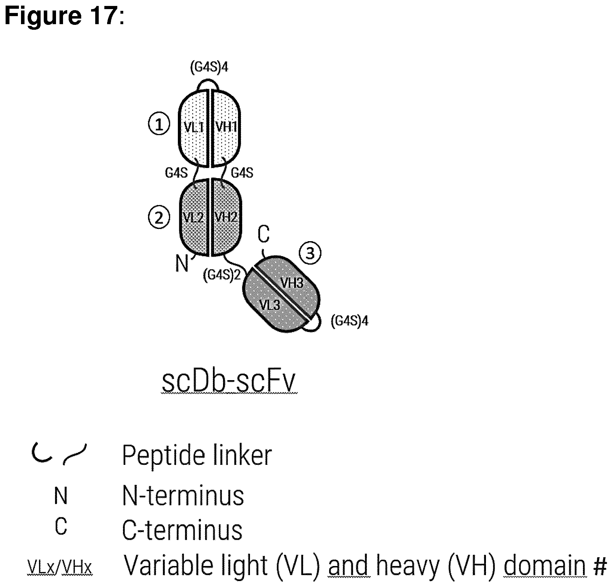

**FIG. 17** Design of the scDb-scFv molecules. Schematic representation and description of the scDb-scFv molecules.

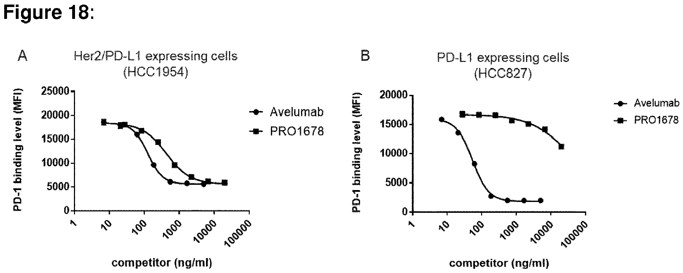

**FIG. 18** Blockade of PD-1/PD-L1 interaction on Her2 expressing cells in presence of human serum albumin. A) PD-1 binding level to cells expressing PD-L1 and high Her2 levels (HCC1954) or (B) cells expressing PD-L1 and a lower level of Her2 (HCC827) in presence of increasing concentrations of Avelumab and PRO1678 (Her2xhSAxPD-L1$_{low\ affinity}$). PRO1678 inhibits PD-1 binding to PD-L1 with a 100-fold better potency on PD-L1/high Her2 expressing cells than on PD-L1 expressing cells (HCC827) with an IC$_{50}$ value of 428.2 ng/ml. For comparison, Avelumab inhibit this interaction with an IC$_{50}$ of 127.7 ng/ml (HCC1954) and 46.03 ng/ml (HCC827). Data were fitted using sigmoidal 4PL fit (GraphPad Prism).

## DETAILED DESCRIPTION OF THE INVENTION

Even though utilization of therapeutic antibodies inhibiting interaction of a tumor-associated immune checkpoint antigen, such as PD-L1, which its cognate ligand, such as PD-1, is a very promising treatment strategy, it is coupled to such difficulties as high toxicities and adverse events. There is thus a need in the medical field for novel approaches of inhibiting the interaction of a tumor-associated immune checkpoint antigen which its cognate ligand, which have lower rate of dose-limiting toxicities and adverse events than the currently available approaches.

The present invention provides a multispecific antibody comprising: at least a first domain specifically binding to a tumor-associated immune checkpoint antigen with low affinity, and at least a second domain specifically binding to a tumor-associated antigen (TAA)). The multispecific antibody of the present disclosure are capable of binding to a target cell displaying said TAA by virtue of said first domain specifically binding to said TAA, and of simultaneous binding of said low affinity binding domain, due to avidity effects, to said tumor-associated immune checkpoint antigen present on the same target cell, so that interaction of said tumor-associated immune checkpoint antigen is inhibited. Due to the low affinity of said first domain, specific binding to non-target cells displaying only said tumor-associated immune checkpoint antigen, but not said TAA, is not occurring to any relevant extent. Thus, the multispecific antibody of the present invention due to its ability to mediate, e.g. agonize, potent signaling of said tumor-associated immune checkpoint antigen on said target cells without interacting with non-target cells, so that the treatment with the multispecific antibody of the present invention does not lead to depletion of cells not expressing the TAA..

In addition, it has been surprisingly found that, the multispecific antibody of the present disclosure comprising (a) at least said first domain, (b) at least said second domain, and (c) at least a third domain specifically binding to an immune cell antigen demonstrated further beneficial properties as shown in the Examples and accompanying figures. Furthermore, the optional addition of a half-life-extending anti-HSA domain not only enables convenient dosing, but also should promote delivery of the molecule to tumor microenvironments.

The multispecific antibodies of the present invention thus provide distinct therapeutic advantages over conventional compositions and therapies.

Unless defined otherwise, all technical and scientific terms used herein have the same meaning as commonly understood by those of ordinary skill in the art to which this invention pertains.

The terms "comprising" and "including" are used herein in their open-ended and non-limiting sense unless otherwise noted. With respect to such latter embodiments, the term "comprising" thus includes the narrower term "consisting of".

The terms "a" and "an" and "the" and similar references in the context of describing the invention (especially in the context of the following claims) are to be construed to cover both the singular and the plural, unless otherwise indicated herein or clearly contradicted by context. For example, the term "a cell" includes a plurality of cells, including mixtures thereof. Where the plural form is used for compounds, salts, and the like, this is taken to mean also a single compound, salt, or the like.

In one aspect, the present invention relates to a multispecific antibody comprising at least a first domain specifically binding to a tumor-associated immune checkpoint antigen with low affinity, and at least a second domain specifically binding to a tumor-associated antigen (TAA).

The term "antibody" and the like, as used herein, includes whole antibodies or single chains thereof; and any antigen-binding fragment (i.e., "antigen-binding portion") or single chains thereof; and molecules comprising antibody CDRs, VH regions or VL regions (including without limitation multispecific antibodies). A naturally occurring "whole antibody" is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The terms "binding domain", "antigen-binding fragment thereof", "antigen binding portion" of an antibody, and the like, as used herein, refer to one or more fragments of an intact antibody that retain the ability to specifically bind to a given antigen (e.g., CD137, PD-L1, HSA). Antigen binding functions of an antibody can be performed by fragments of an intact antibody. In some embodiments, a binding domain of a multispecific antibody of the present invention is selected from the group consisting of a Fab fragment, a monovalent fragment consisting of the VL, VH, CL and CH1 domains; a F (ab)2 fragment, a bivalent fragment comprising two Fab fragments linked by a disulfide bridge at the hinge region; an Fd fragment consisting of the VH and CH1 domains; an Fv fragment consisting of the VL and VH domains of a single arm of an antibody; a single domain antibody (dAb) fragment (Ward et al., 1989 Nature 341:544-546), which consists of a VH domain; an isolated complementarity determining region (CDR), dsFv, a scAb, STAB, a single domain antibody (sdAb or dAb), a single domain heavy chain antibody, and a single domain light chain antibody, a VHH, a VNAR, single domain antibodies based on the VNAR structure from shark, and binding domains based on alternative scaffolds including but limited to ankyrin-based domains, fynomers, avimers, anticalins, fibronectins, and binding sites being built into constant regions of antibodies (e.g. f-star technology (F-star's Modular Antibody Technology™)). Suitably, a binding domain of the present invention is a single-chain Fv fragment (scFv) or single antibody variable domains. In a preferred embodiment, a binding domain of the present invention is a single-chain Fv fragment (scFv).

The term "Complementarity Determining Regions" ("CDRs") are amino acid sequences with boundaries determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273, 927-948 ("Chothia" numbering scheme), ImMunoGenTics (IMGT) numbering (Lefranc, M.-P., The Immunologist, 7, 132-136 (1999); Lefranc, M.-P. et al., Dev. Comp. Immunol., 27, 55-77 (2003) ("IMGT" numbering scheme) and numbering scheme described in Honegger & Plückthun, J. Mol. Biol. 309 (2001) 657-670 ("AHo" numbering). For example, for classic formats, under Kabat, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under Chothia the CDR amino acids in the VH are numbered 26-32 (HCDR1), 52-56 (HCDR2), and 95-102 (HCDR3); and the amino acid residues in VL are numbered 24-34 (LCDR1), 50-56 (LCDR2), and 89-97 (LCDR3). By combining the CDR definitions of both Kabat and Chothia, the CDRs consist of amino acid residues 26-35 (HCDR1), 50-65 (HCDR2), and 95-102 (HCDR3) in human VH and amino acid residues 24-34 (LCDR1), 50-56 (LCDR2), and 89-97 (LCDR3) in human VL. Under IMGT the CDR amino acid residues in the VH are numbered approximately 26-35 (HCDR1), 51-57 (HCDR2) and 93-102 (HCDR3), and the CDR amino acid residues in the VL are numbered approximately 27-32 (LCDR1), 50-52 (LCDR2), and 89-97 (LCDR3) (numbering according to "Kabat"). Under IMGT, the CDRs of an antibody can be determined using the program IMGT/DomainGap Align.

In the context of the present invention, the numbering system suggested by Honegger & Plückthun ("AHo") is used (Honegger & Plückthun, J. Mol. Biol. 309 (2001) 657-670), unless specifically mentioned otherwise. Furthermore, the following residues are defined as CDRs according to AHo numbering scheme: LCDR1 (also referred to as CDR-L1): L24-L42; LCDR2 (also referred to as CDR-L2): L58-L72; LCDR3 (also referred to as CDR-L3): L107-L138; HCDR1 (also referred to as CDR-H1): H27-H42; HCDR2 (also referred to as CDR-H2): H57-H76; HCDR3 (also referred to as CDR-H3): H108-H138. For the sake of clarity, the numbering system according to Honegger & Plückthun takes the length diversity into account that is found in naturally occurring antibodies, both in the different VH and VL subfamilies and, in particular, in the CDRs, and provides for gaps in the sequences. Thus, in a given antibody variable domain usually not all positions 1 to 149 will be occupied by an amino acid residue.

The term "binding specificity" as used herein refers to the ability of an individual antibody to react with one antigenic determinant and not with a different antigenic determinant. As use herein, the term "specifically binds to" or is "specific for" refers to measurable and reproducible interactions such as binding between a target and an antibody, which is determinative of the presence of the target in the presence of a heterogeneous population of molecules including biological molecules. For example, an antibody that specifically binds to a target (which can be an epitope) is an antibody that binds this target with greater affinity, avidity, more readily, and/or with greater duration than it binds to other targets. In its most general form (and when no defined reference is mentioned), "specific binding" is referring to the ability of the antibody to discriminate between the target of interest and an unrelated molecule, as determined, for example, in accordance with a specificity assay methods known in the art. Such methods comprise, but are not limited to Western blots, ELISA, RIA, ECL, IRMA, SPR (Surface plasmon resonance) tests and peptide scans. For example, a standard ELISA assay can be carried out. The scoring may be carried out by standard colour development (e.g. secondary antibody with horseradish peroxide and tetramethyl benzidine with hydrogen peroxide). The reaction in certain wells is scored by the optical density, for example, at 450 nm. Typical background (= negative reaction) may be about 0.1 OD; typical positive reaction may be about 1 OD. This means the ratio between a positive and a negative score can be 10-fold or higher. In a further example, an SPR assay can be carried out, wherein at least 10-fold, preferably at least 100-fold difference between a background and signal indicates on specific binding. Typically, determination of binding specificity is performed by using not a single reference molecule, but a set of about three to five unrelated molecules, such as milk powder, transferrin or the like.

Suitably, the antibody of the invention is an isolated antibody. The term "isolated antibody", as used herein, refers to an antibody that is substantially free of other antibodies having different antigenic specificities (e.g., an isolated antibody that specifically binds PD-L1 and HER2 is substantially free of antibodies that specifically bind antigens other than PD-L1 and HER2, e.g., an isolated antibody that specifically binds PD-L1, HER2 and human serum albumin is substantially free of antibodies that specifically bind antigens other than PD-L1, HER2 and human serum albumin). Moreover, an isolated antibody may be substantially free of other cellular material and/or chemicals.

Suitably, the antibody of the invention is a monoclonal antibody. The term "monoclonal antibody" or "monoclonal antibody composition" as used herein refers to antibodies that are substantially identical to amino acid sequence or are derived from the same genetic source. A monoclonal antibody composition displays a binding specificity and affinity for a particular epitope, or binding specificities and affinities for specific epitopes.

Antibodies of the invention include, but are not limited to, the chimeric, human and humanized.

The term "chimeric antibody" (or antigen-binding fragment thereof) is an antibody molecule (or antigen-binding fragment thereof) in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity. For example, a mouse antibody can be modified by replacing its constant region with the constant region from a human immunoglobulin. Due to the replacement with a human constant region, the chimeric antibody can retain its specificity in recognizing the antigen while having reduced antigenicity in human as compared to the original mouse antibody.

The term "human antibody" (or antigen-binding fragment thereof), as used herein, is intended to include antibodies (and antigen-binding fragments thereof) having variable regions in which both the framework and CDR regions are derived from sequences of human origin. Furthermore, if the antibody contains a constant region, the constant region also is derived from such human sequences, e.g., human germline sequences, or mutated versions of human germline sequences. The human antibodies and antigen-binding fragments thereof of the invention may include amino acid residues not encoded by human sequences (e.g., mutations introduced by random or site-specific mutagenesis in vitro or by somatic mutation in vivo). This definition of a human antibody specifically excludes a humanized antibody comprising non-human antigen-binding residues. Human antibodies can be produced using various techniques known in the art, including phage-display libraries (Hoogenboom and Winter, J. Mol. Biol, 227:381 (1991); Marks et al, J. Mol. Biol, 222:581 (1991)). Also available for the preparation of human monoclonal antibodies are methods described in Cole et al, Monoclonal Antibodies and Cancer Therapy, Alan R. Liss, p. 77 (1985); Boemer et al, J. Immunol, 147(I):86-95 (1991). See also van Dijk and van de Winkel, Curr. Opin. Pharmacol, 5: 368-74 (2001). Human antibodies can be prepared by administering the antigen to a transgenic animal that has been modified to produce such antibodies in response to antigenic challenge, but whose endogenous loci have been disabled, e.g., immunized xenomice (see, e.g., U.S. Pat. Nos. 6,075,181 and 6,150,584 regarding XENOMOUSE™ technology). See also, for example, Li et al, Proc. Natl. Acad. Sci. USA, 103:3557-3562 (2006) regarding human antibodies generated via a human B-cell hybridoma technology.

A "humanized" antibody (or antigen-binding fragment thereof), as used herein, is an antibody (or antigen-binding fragment thereof) that retains the reactivity of a non-human antibody while being less immunogenic in humans. This can be achieved, for instance, by retaining the non-human CDR regions and replacing the remaining parts of the antibody with their human counterparts (i.e., the constant region as well as the framework portions of the variable region). Additional framework region modifications may be made within the human framework sequences as well as within the CDR sequences derived from the germline of another mammalian species. The humanized antibodies of the invention may include amino acid residues not encoded by human sequences (e.g., mutations introduced by random or site-specific mutagenesis in vitro or by somatic mutation in vivo, or a conservative substitution to promote stability or manufacturing). See, e.g., Morrison et al., Proc. Natl. Acad. Sci. USA, 81:6851-6855, 1984; Morrison and Oi, Adv. Immunol., 44:65-92, 1988; Verhoeyen et al., Science, 239: 1534-1536, 1988; Padlan, Molec. Immun., 28:489-498, 1991; and Padlan, Molec. Immun., 31: 169-217, 1994. Other examples of human engineering technology include, but is not limited to Xoma technology disclosed in U.S. Pat. No. 5,766,886.

The term "recombinant humanized antibody" as used herein, includes all human antibodies that are prepared, expressed, created or isolated by recombinant means, such as antibodies isolated from a host cell transformed to express the humanized antibody, e.g., from a transfectoma, and antibodies prepared, expressed, created or isolated by any other means that involve splicing of all or a portion of a human immunoglobulin gene, sequences to other DNA sequences.

Suitably, the antibody of the invention or antigen-binding fragment thereof is humanized. Suitably, the antibody of the invention or antigen-binding fragment thereof is humanized and comprises rabbit-derived CDRs.

The term "multispecific antibody" as used herein, refers to an antibody that binds to two or more different epitopes on at least two or more different targets (e.g., PD-L1 and HER2). The term "multispecific antibody" includes bispecific, trispecific, tetraspecific, pentaspecific and hexaspecific. The term "bispecific antibody" as used herein, refers to an antibody that binds to two different epitopes on at least two different targets (e.g., PD-L1 and HER2). The term "trispecific antibody" as used herein, refers to an antibody that binds to three different epitopes on at least three different targets (e.g., PD-L1, HER2 and HSA).

The term "epitope" means a protein determinant capable of specific binding to an antibody. Epitopes usually consist of chemically active surface groupings of molecules such as amino acids or sugar side chains and usually have specific three-dimensional structural characteristics, as well as specific charge characteristics. "Conformational" and "linear" epitopes are distinguished in that the binding to the former but not the latter is lost in the presence of denaturing solvents.

The term "conformational epitope" as used herein refers to amino acid residues of an antigen that come together on the surface when the polypeptide chain folds to form the native protein.

The term "linear epitope" refers to an epitope with all of the points of interaction between the protein and the interacting molecule (such as an antibody) occurring linearly along the primary amino acid sequence of the protein (continuous).

The term "distal epitope" refers to an epitope which is comprised in the region of the extracellular part of a cell-bound antigen that is distant from the cell surface.

The term "recognize" as used herein refers to an antibody antigen-binding fragment thereof that finds and interacts (e.g., binds) with its conformational epitope.

As used herein, the term "affinity" refers to the strength of interaction between antibody and antigen at single antigenic sites. Within each antigenic site, the variable region of the antibody "arm" interacts through weak non-covalent forces with antigen at numerous sites; the more interactions, the stronger the affinity.

"Binding affinity" generally refers to the strength of the sum total of non-covalent interactions between a single binding site of a molecule (e.g., of an antibody) and its binding partner (e.g., an antigen). Unless indicated otherwise, as used herein, "binding affinity", "bind to", "binds to" or "binding to" refers to intrinsic binding affinity that reflects a 1:1 interaction between members of a binding pair (e.g., an antibody fragment and antigen). The affinity of a molecule X for its partner Y can generally be represented by the dissociation constant (KD). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention. Specific illustrative and exemplary embodiments for measuring binding affinity, i.e. binding strength are described in the following.

The term "Kassoc", "Ka" or "Kon", as used herein, is intended to refer to the association rate of a particular antibody-antigen interaction, whereas the term "Kdis", "Kd" or "Koff", as used herein, is intended to refer to the dissociation rate of a particular antibody- antigen interaction. In one embodiment, the term "KD", as used herein, is intended to refer to the dissociation constant, which is obtained from the ratio of Kd to Ka (i.e. Kd/Ka) and is expressed as a molar concentration (M). The "KD" or "KD value" or "KD" or "KD value" according to this invention is in one embodiment measured by using surface-plasmon resonance assays. Affinity to PD-L1 was determined by surface plasmon resonance (SPR) measurements as described in section [0185]. Binding affinities of multi-specific constructs towards recombinant human CD3$\varepsilon$ ECD, recombinant human IL-23R and recombinant human Her2 ECD were measured by SPR as described in section [0198]. Affinity of molecules to human serum albumin (HSA) and mouse serum albumin (MSA) was determined by SPR measurements as described in section [0199].

Suitably, the multispecific antibody of the present invention is monovalent, bivalent or multivalent for PD-L1 specificity. In one embodiment, the multispecific antibody of the present invention is bivalent for PD-L1 specificity. In a preferred embodiment, the multispecific antibody of the present invention is monovalent for PD-L1 specificity.

Suitable PD-L1 -BDs for use in the multispecific antibody of the invention are binding domains provided in the present disclosure. The PD-L1-BDs of the invention include, but are not limited to, the humanized monoclonal antibodies whose sequences are listed in Table 1.

Suitably, the multispecific antibody of the present invention is monovalent, bivalent or multivalent for HER2 specificity. In one embodiment, the multispecific antibody of the present invention is bivalent for HER2 specificity. In a preferred embodiment, the multispecific antibody of the present invention is monovalent for HER2 specificity.

Suitable HER2-BDs for use in the multispecific antibody of the invention are binding domains provided in the present disclosure. The HER2-BDs of the invention include, but are not limited to, the humanized monoclonal antibodies whose sequences are listed in Table 2.

The term "multivalent antibody" refers to a single binding molecule with more than one valency, where "valency" is described as the number of antigen-binding moieties that binds to epitopes on identical target molecules. As such, the single binding molecule can bind to more than one binding site on a target molecule. Examples of multivalent antibodies include, but are not limited to bivalent antibodies, trivalent antibodies, tetravalent antibodies, pentavalent antibodies, and the like.

The term "monovalent antibody", as used herein, refers to an antibody that binds to a single epitope on a target molecule, such as PD-L1. Also, the term "binding domain" or "monovalent binding domain", as used herein, refers to a binding domain that binds to a single epitope on a target molecule such as PD-L1.

The term "bivalent antibody" as used herein, refers to an antibody that binds to two epitopes on at least two identical target molecules, such as PD-L1 target molecules.

The inventors of the present invention have now surprisingly found that addition of the tri-specific molecule PRO1678 (anti-HSAxPDL1xHER2) resulted in a significantly reduced tumor growth in a HCC1954 xenograft NOG mouse model when compared to an equipotent dose (same activity as determined in vitro) of nivolumab. A five time lower dose of PRO1678 led to the same reduction of tumor growth in this model (see Figure 13). The inventors have furthermore surprisingly found that a tetra-specific molecule comprising a fourth, CD3-BD such as PRO1543 (anti-CDAxHSAxPDL1xHER2) resulted in a complete regression of the tumor in the HCC1954 xenograft NOG mouse model. This finding is insofar surprising as it cannot a priori be expected that all four binding domains remain functional without sterically or otherwise inhbiting each other in a complex multi-target, multi-cell in vivo situation. As the EC$_{50}$ of PD-L1 blockade in these cells is considerably higher than the EC$_{50}$ of target cell lysis, it is highly likely that the improved activity of the molecule containing an anti-PD-L1 domains results from increased avidity. As a consequence, binding to Her2 and PD-L1 double positive cells is stronger than binding to PD-L1 negative cells expressing Her2. This avidity binding increases the therapeutic window as it selectively improves potency on tumor cells (Her2/PD-L1 double positive), but not on PD-L1 negative Her2 expressing normal cells.

The term "tumor-associated immune checkpoint antigen" refers to a transmembrane protein expressed by the tumor that suppresses the activity of immune cells, particularly to an antigen taken from the group of: PD-L1, PD-L2, CD80, CD86, CD276 (B7-H3), and VTCN1 (B7-H4), more particularly PD-L1.

The term "low affinity" refers to a binding domain that binds to its cognate target with a dissociation constant (KD) of between 50 nM and 2000 nM, preferably between 100 nM and 1000 nM.

The term "tumor-associated antigen (TAA)" refers to an antigen that is expressed on the surface of a tumor cell. In particular embodiments, a TAA is an antigen that is preferentially expressed on a tumor cell when compared to non-tumor cells, particularly wherein expression of the TAA on a tumor cell is at least more than 5fold, at least more than 10fold, at least more than 20fold, at least more than 50fold, or at least more than 100fold higher than on non-tumor cells from the same organism or patient. In particular, the TAA is taken from the group of: EGFRvIII, mesothelin, GD2, Tn antigen, sTn antigen, Tn-O-Glycopeptides, sTn-O-Glycopeptides, PSMA, CD97, TAG72, CD44v6, CEA, EPCAM, KIT, IL-13Ra2, leguman, GD3, CD171, IL-11Ra, PSCA, MAD-CT-1, MAD-CT-2, VEGFR2, LewisY, CD24, PDGFR-beta, SSEA-4, folate receptor alpha, ERBBs (e.g., ERBB2), Her2/neu (HER2), MUC1, EGFR, NCAM, Ephrin B2, CAIX, LMP2, sLe, HMWMAA, o-acetyl-GD2, folate receptor beta, TEM1/CD248, TEM7R, FAP, Legumain, HPV E6 or E7, ML-IAP, CLDN6, TSHR, GPRC5D, ALK, Polysialic acid, Fos-related antigen, neutrophil elastase, TRP-2, CYP1B1, sperm protein 17, beta human chorionic gonadotropin, AFP, thyroglobulin, PLAC1, globoH, RAGE1, MN-CA IX, human telomerase reverse transcriptase, intestinal carboxyl esterase, mut hsp 70-2, NA-17, NY-BR-1, UPK2, HAVCR1, ADRB3, PANX3, NY-ESO-1, GPR20, Ly6k, OR51E2, TARP, and GFRalpha4..

The term "immune cell antigen" refers to an antigen present in an immune cell, particularly an immune cell selected from a T cell, an NK cell, and myeloid cells. In particular, the term relates to a protein, which is a stimulatory or co-stimulatory molecule of said immune cell.

In the context of the present invention, the term "stimulatory molecule of said immune cells" relates to molecules such as CD3 and CD16.

In the context of the present invention, the term "co-stimulatory molecule of said immune cells" relates to molecules such as the molecules comprised in the group of molecules consisting of CD137, CD28, ICOS, HVEM, CD27, OX40, DR3, GITR, CD30, SLAM, CD2, 2B4, TIM1, TIM2, and CD226.

In particular embodiments, the multispecific antibody of the present invention furthermore comprises (i) a binding domain for CD3, or (ii) a binding domain for CD137.

Suitable CD3-BDs for use in the multispecific antibody of the invention are binding domains provided in the present disclosure. The CD3-BDs of the invention include, but are not limited to, the humanized monoclonal antibodies whose sequences are listed in Table 3.

Suitable CD137-BDs for use in the multispecific antibody of the invention are binding domains provided in the present disclosure. The CD137-BDs of the invention include, but are not limited to, the humanized monoclonal antibodies whose sequences are listed in Table 5.

Suitably, the multispecific antibody of the invention has two different specificities (PD-L1 and HER2). Suitably, the multispecific antibody of the invention is a bispecific antibody. The multispecific antibody of the present invention may comprise a further specificity (trispecific) or specificities (tetraspecific, pentaspecific or hexaspecific antibody). In one embodiment, the multispecific antibody is trispecific. In another embodiment, the multispecific antibody is tetraspecific

In one embodiment, the multispecific antibody of the invention comprises an immunoglobulin Fc region polypeptide. The term "Fc region" herein is used to define a C-terminal region of an immunoglobulin heavy chain, including native-sequence Fc regions and variant Fc regions. Suitable native-sequence Fc regions include human IgG1, IgG2 (IgG2A, JgG2B), IgG3 and IgG4. "Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors, FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain, (see M. Daeron, Annu. Rev. Immunol. 5:203-234 (1997). FcRs are reviewed in Ravetch and Kinet, Annu. Rev. Immunol. 9: 457-92 (1991); Capet et al, Immunomethods 4: 25-34 (1994); and de Haas et al, J. Lab. Clin. Med. 126: 330-41 (1995). Other FcRs, including those to be identified in the future, are encompassed by the term "FcR" herein. The term "Fc receptor" or "FcR" also includes the neonatal receptor, FcRn, which is responsible for the transfer of maternal IgGs to the fetus. Guyer et al., J. Immunol. 117: 587 (1976) and Kim et al., J. Immunol. 24: 249 (1994). Methods of measuring binding to FcRn are known (see, e.g., Ghetie and Ward, Immunol. Today 18: (12): 592-8 (1997); Ghetie et al., Nature Biotechnology 15 (7): 637-40 (1997); Hinton et al., J. Biol. Chem. TJI (8): 6213-6 (2004); WO 2004/92219 (Hinton et al). Binding to FcRn in vivo and serum half-life of human FcRn high-affinity binding polypeptides can be assayed, e.g., in transgenic mice or transfected human cell lines expressing human FcRn, or in primates to which the polypeptides having a variant Fc region are administered. WO 2004/42072 (Presta) describes antibody variants which improved or diminished binding to FcRs. See also, e.g., Shields et al., J. Biol. Chem. 9(2): 6591-6604 (2001).

In another embodiment, the antibody of the invention does not comprise an immunoglobulin Fc region polypeptide.

In order to increase the number of specificities/functionalities at the same or lower molecular weight, it is advantageous to use antibodies comprising antibody fragments, such as Fv, Fab, Fab' and F(ab')2 fragments and other antibody fragments. These smaller molecules retain the antigen binding activity of the whole antibody and can also exhibit improved tissue penetration and pharmacokinetic properties in comparison to the whole immunoglobulin molecules. Whilst such fragments appear to exhibit a number of advantages over whole immunoglobulins, they also suffer from an increased rate of clearance from serum since they lack the Fc domain that imparts a long half-life in vivo (Medasan et al., 1997, J. Immunol. 158:2211-2217). Molecules with lower molecular weights penetrate more efficiently into target tissues (e.g. solid cancers) and thus hold the promise for improved efficacy at the same or lower dose.

The inventors have surprisingly found that an addition of human serum albumin binding domain (HSA-BD) to the multispecific antibody of the invention does not interfere with the ability of the other binding domains to bind to their respective targets This finding is insofar surprising as it cannot a priori be expected that all four binding domains remain functional without sterically or otherwise inhbiting each other in a complex multi-target, multi-cell in vivo situation.

Suitably, the multispecific antibody of the present invention may comprise a further binding domain having specificity to human serum albumin. In one embodiment, the multispecific antibody comprises: (i) at least one PD-L1-BD; (ii) at least one HER2-BD; and (iii) at least one HSA-BD. Suitably, the multispecific antibody of the present invention comprises: (i) one PD-L1-BD; (ii) at least one HER2-BD, preferably one PD-L1-BD or two PD-L1-BDs, more preferably one PD-L1-BD; and (iii) at least one HSA-BD, preferably one HSA-BD.

The term "HSA" refers in particular to human serum albumin with UniProt ID number P02768. Human Serum Albumin (HSA) is 66.4 kDa abundant protein in human serum (50 % of total protein) composing of 585 amino acids (Sugio, Protein Eng, Vol. 12, 1999, 439-446). Multifunctional HSA protein is associated with its structure that allowed to bind and transport a number of metabolizes such as fatty acids, metal ions, bilirubin and some drugs (Fanali, Molecular Aspects of Medicine, Vol. 33, 2012, 209-290). HSA concentration in serum is around 3.5-5 g/dL. Albumin binding antibodies and fragments thereof may be used for example, for extending the in vivo serum half-life of drugs or proteins conjugated thereto.

In some embodiments, the HSA-BD is derived from a monoclonal antibody or antibody fragment.

Suitable HSA-BDs for use in the multispecific antibody of the invention are binding domains provided in the present disclosure. The HSA-BDs of the invention include, but are not limited to, the humanized monoclonal antibodies whose sequences are listed in Table 4.

In particular, the HSA-BDs of the invention specifically bind to human serum albumin. The HSA-BDs of the invention comprise a VH CDR having an amino acid sequence of any one of the VH CDRs listed in Table 4. In particular, the invention provides HSA-BDs comprising (one, two, three, or more VH CDRs having an amino acid sequence of any of the VH CDRs listed in Table 4.

The invention also provides HSA-BDs comprising a VL CDR having an amino acid sequence of any one of the VL CDRs listed in Table 4. In particular, the invention provides HSA-BDs comprising one, two, three or more VL CDRs having an amino acid sequence of any of the VL CDRs listed in Table 4.

Other variable domains of the invention include amino acids that have been mutated, yet have at least 60, 70, 80, 90, 91, 92, 93, 94, 95, 96, 97, 98 or 99 percent identity in the CDR regions with the CDR regions depicted in the sequences described in Tables 1 to 5. Other variable domains of the invention include mutant amino acid sequences wherein no more than 1, 2, 3, 4 or 5 amino acids have been mutated in the CDR regions when compared with the CDR regions depicted in the sequence described in Tables 1 to 5.

In a further embodiment, the invention provides a HSA-BD that specifically binds human serum albumin, wherein said binding domain comprises a VH domain and a VL domain.

Other suitable HSA-BD for use in the multispecific antibody of the invention comprises or is derived from an antibody selected from the group consisting of: (i) polypeptides that bind serum albumin (see, for example, Smith et al., 2001, Bioconjugate Chem. 12:750-756; EP0486525; US6267964; WO 2004/001064; WO 2002/076489; and WO 2001/45746); (ii) anti-serum albumin binding single variable domains described in Holt et al., Protein Engineering, Design & Selection, vol 21, 5, pp283-288, WO 2004/003019, WO 2008/096158, WO 2005/118642, WO 2006/0591056 and WO 2011/006915; (iii) anti-serum albumin antibodies described in WO 2009/040562, WO 2010/035012 and WO 2011/086091.

Suitably, the VH domains of the binding domains of the invention belong to a VH3 or VH4 family. In one embodiment, a binding domain of the invention comprises a VH domain belonging to the VH3 family. In the context of the present invention, the term "belonging to VHx family (or VLx family)" means that the framework sequences FR1 to FR2 show the highest degree of homology to said VHx family (or VLx, respectively). Examples of VH and VL families are given in Knappik et al., J. Mol. Biol. 296 (2000) 57-86, or in WO 2019/057787. A specific example of a VH domain belonging to VH3 family is represented by SEQ ID NO: 120, and a specific example of a VH domain belonging to VH4 family is represented by SEQ ID NO: 121. In particular, framework regions FR1 to FR4 taken from SEQ ID NO: 120 belong to VH3 family (Table 7, regions marked in non-bold). Suitably, a VH belonging to VH3 family, as used herein, is a VH comprising FR1 to FR4 having at least 85 %, preferably at least 90 %, more preferably at least 95 % sequence identity to FR1 to FR4 of SEQ ID NO: 120. Alternative examples of VH3 sequences, and examples of VH4 sequences, may be found in Knappik et al., J. Mol. Biol. 296 (2000) 57-86 or in WO 2019/057787. Suitably, the HSA-BD of the invention comprises: Vκ frameworks FR1, FR2 and FR3, particularly Vκ1 or Vκ3 frameworks, preferably Vκ1 frameworks FR1 to 3, and a framework FR4, which is selected from a Vκ FR4, particularly Vκ1 FR4, Vκ3 FR4, and a Vλ FR4. Suitable Vκ1 frameworks FR1 to 3 are set forth in SEQ ID NO: 122 (Table 7, FR regions are marked in non-bold). Alternative examples of V κ1 sequences, and examples of Vκ2, Vκ3 or Vκ4 sequences, may be found in Knappik et al., J. Mol. Biol. 296 (2000) 57-86. Suitable Vκ1 frameworks FR1 to 3 comprise the amino acid sequences having at least 60, 70, 80, 90 percent identity to amino acid sequences corresponding to FR1 to 3 and taken from SEQ ID NO: 122 (Table 7, FR regions are marked in non-bold). Suitable Vλ FR4 are as set forth in SEQ ID NO: 123 to SEQ ID NO: 129. In one embodiment, the VL domains of the present invention comprises Vλ FR4 comprising the amino acid sequence having at least 60, 70, 80, 90 percent identity to an amino acid sequence selected from any of SEQ ID NO: 123 to SEQ ID NO: 129, preferably to SEQ ID NO: 124.

The binding domains of the invention comprises a VH domain listed in Tables 1 to 5. Suitably, a binding domain of the invention comprises a VH amino acid sequence listed in one of Tables 1 to 5, wherein no more than about 10 amino acids in a framework sequence (for example, a sequence which is not a CDR) have been mutated (wherein a mutation is, as various non-limiting examples, an addition, substitution or deletion). Suitably, a binding domain of the present invention comprises a VH amino acid sequence listed in one of Tables 1 to 5, wherein no more than about 20 amino acids in a framework sequence (for example, a sequence which is not a CDR) have been mutated (wherein a mutation is, as various non-limiting examples, an addition, substitution or deletion). Other binding domains of the invention include amino acids that have been mutated, yet have at least 60, 70, 80, 90, 91, 92, 93, 94, 95, 96, 97, 98 or 99 percent identity in the VH regions with the VH regions depicted in the corresponding sequences described in one of Tables 1 to 5.

In particular, a binding domain of the invention comprises a VL domain listed in one of Tables 1 to 5. Suitably, a binding domain of the invention comprises a VL amino acid sequence listed in one of Tables 1 to 5, wherein no more than about 10 amino acids in a framework sequence (for example, a sequence which is not a CDR) have been mutated (wherein a mutation is, as various non-limiting examples, an addition, substitution or deletion). Suitably, a binding domain of the invention comprises a VL amino acid sequence listed in one of Tables 1 to 5, wherein no more than about 20 amino acids in a framework sequence (for example, a sequence which is not a CDR) have been mutated (wherein a mutation is, as various non-limiting examples, an addition, substitution or deletion). Other binding domains of the invention include amino acids that have been mutated, yet have at least 60, 70, 80, 90, 91, 92, 93, 94, 95, 96, 97, 98 or 99 percent identity in the VL regions with a VL region depicted in the sequences described in Tables 1 to 5.

In the context of the present invention, the term "binding domain of the present invention" relates both to a binding domain a such, i.e. independent of a multispecific context, and, in particular, to a binding domain comprised in a multispecific construct, e.g. one of the binding domains comprised in a bispecific, trispecific or tetraspecific construct.

Suitably, a binding domain of the invention is selected from the group consisting of: a Fab, an Fv, an scFv, dsFv, a scAb, and STAB.

Suitably, a binding domain of the invention is an scFv antibody fragment..

The multispecific antibody of the invention may be in any suitable format.

Suitably, the binding domains of the multispecific antibody are operably linked. The binding domains of the multispecific antibody of the invention are capable of binding to their respective antigens or receptors simultaneously.

In one embodiment, the multispecific antibody of the invention comprises at least one PD-L1-BD, at least one HER2-BD, wherein: (i) said PD-L1-BD and said HER2-BD are both operably linked to each other. In one embodiment, the multispecific antibody of the invention comprises at least one PD-L1-BD, at least one HER2-BD, at least one HSA-BD, wherein: (i) said PD-L1-BD and said HER2-BD are both operably linked to said HSA-BD; or (ii) said PD-L1-BD and said HSA-BD are both operably linked to said HER2-BD; or (iii) said HER2-BD and said HSA-BD are both operably linked to said PD-L1-BD. In a preferred embodiment, the multispecific antibody of the invention comprises at least one PD-L1-BD, at least one HER2-BD, at least one HSA-BD, wherein said PD-L1-BD and said HSA-BD are both operably linked to said HER2-BD.

The term "operably linked", as used herein, indicates that two molecules (e.g., polypeptides, domains, binding domains) are attached so as to each retain functional activity. Two molecules can be "operably linked" whether they are attached directly or indirectly (e.g., via a linker, via a moiety, via a linker to a moiety). The term "linker" refers to a peptide or other moiety that is optionally located between binding domains or antibody fragments of the invention. A number of strategies may be used to covalently link molecules together. These include, but are not limited to polypeptide linkages between N- and C-termini of proteins or protein domains, linkage via disulfide bonds, and linkage via chemical cross-linking reagents. In one aspect of this embodiment, the linker is a peptide bond, generated by recombinant techniques or peptide synthesis. Choosing a suitable linker for a specific case where two polypeptide chains are to be connected depends on various parameters, including but not limited to the nature of the two polypeptide chains (e.g., whether they naturally oligomerize), the distance between the N- and the C-termini to be connected if known, and/or the stability of the linker towards proteolysis and oxidation. Furthermore, the linker may contain amino acid residues that provide flexibility.

In the context of the present invention, the term "polypeptide linker" refers to a linker consisting of a chain of amino acid residues linked by peptide bonds that is connecting two domains, each being attached to one end of the linker. The polypeptide linker should have a length that is adequate to link two molecules in such a way that they assume the correct conformation relative to one another so that they retain the desired activity. In particular embodiments, the polypeptide linker has a continuous chain of between 2 and 30 amino acid residues (e.g., 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acid residues). In addition, the amino acid residues selected for inclusion in the polypeptide linker should exhibit properties that do not interfere significantly with the activity of the polypeptide. Thus, the linker peptide on the whole should not exhibit a charge that would be inconsistent with the activity of the polypeptide, or interfere with internal folding, or form bonds or other interactions with amino acid residues in one or more of the monomers that would seriously impede the binding of receptor monomer domains. In particular embodiments, the polypeptide linker is non-structured polypeptide. Useful linkers include glycine-serine, or GS linkers. By "Gly-Ser" or "GS" linkers is meant a polymer of glycines and serines in series (including, for example, (Gly-Ser)n, (GSGGS)n (GGGGS)n and (GGGS)n, where n is an integer of at least one), glycine-alanine polymers, alanine-serine polymers, and other flexible linkers such as the tether for the shaker potassium channel, and a large variety of other flexible linkers, as will be appreciated by those in the art. Glycine-serine polymers are preferred since both of these amino acids are relatively unstructured, and therefore may be able to serve as a neutral tether between components. Secondly, serine is hydrophilic and therefore able to solubilize what could be a globular glycine chain. Third, similar chains have been shown to be effective in joining subunits of recombinant proteins such as single chain antibodies.

Suitably, the multispecific antibody is in a format selected from any suitable multispecific, e.g. bispecific, format known in the art, including, by way of non-limiting example, formats based on a single-chain diabody (scDb), a tandem scDb (Tandab), a linear dimeric scDb (LD-scDb), a circular dimeric scDb (CD-scDb), a bispecific T-cell engager (BiTE; tandem di-scFv), a tandem tri-scFv, a tribody (Fab-(scFv)2) or bibody (Fab-(scFv)1), Fab, , Fab-Fv2, Morrison (IgG CH3-scFv fusion (Morrison L) or IgG CL-scFv fusion (Morrison H)), triabody, scDb-scFv, bispecific Fab2, di-miniantibody, tetrabody, scFv-Fc-scFv fusion, scFv-HSA-scFv fusion, di-diabody, DVD-Ig, COVD, IgG-scFab, scFab-dsscFv, Fv2-Fc, IgG-scFv fusions, such as bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain), Ts2Ab (dsscFv linked to C-terminus of heavy chain), Bispecific antibodies based on heterodimeric Fc domains, such as Knob-into-Hole antibodies (KiHs) (bispecific IgGs prepared by the KiH technology); an Fv, scFv, scDb, tandem-di-scFv, tandem tri-scFv, Fab-(scFv)2, Fab-(scFv)1, Fab, Fab-Fv2, COVD fused to the N- and/or the C-terminus of either chain of a heterodimeric Fc domain or any other heterodimerization domain, a MATCH (described in WO 2016/0202457; Egan T., et al., mAbs 9 (2017) 68-84) and DuoBodies (bispecific IgGs prepared by the Duobody technology) (MAbs. 2017 Feb/Mar;9(2):182-212. doi: 10.1080/19420862.2016.1268307). Particularly suitable for use herein is a single-chain diabody (scDb) or scDb-scFv.

In one embodiment, the multispecific antibody of the invention is in a format selected from the list consisting of scDb (diabody), scDb-scFv, triabody, and tribody. Particularly suitable for use herein is a single-chain diabody (scDb), in particular a bispecific monomeric scDb. Also, particularly suitable for use herein is a scDb-scFv, in particular wherein said CD137-BD and said PD-L1-BD are in the form of a scDb and said HSA-BD is an scFv operably linked to said scDb.

The term "diabodies" refers to antibody fragments with two antigen-binding sites, which fragments comprise a VH connected to VL in the same polypeptide chain (VH-VL). By using a linker that is too short to allow pairing between the two domains on the same chain, the domains are forced to pair with the complementary domains of another chain to create two antigen-binding sites. Diabodies may be bivalent or bispecific. Diabodies are described more fully in, for example, EP404097, WO 93/01161, Hudson et al., Nat. Med. 9:129-134 (2003), and Hollinger et al., Proc. Natl. Acad. Sci. USA 90: 6444-6448 (1993). Triabodies and tetrabodies are also described in Hudson et al., Nat. Med. 9:129-134 (2003).

The bispecific scDb, in particular the bispecific monomeric scDb, particularly comprises two variable heavy chain domains (VH) or fragments thereof and two variable light chain domains (VL) or fragments thereof connected by linkers L1, L2 and L3 in the order VHA-L1-VLB-L2-VHB-L3-VLA, VHA-L1-VHB-L2-VLB-L3-VLA, VLA-L1-VLB-L2-VHB-L3-VHA, VLA-L1-VHB-L2-VLB-L3-VHA, VHB-L1-VLA-L2-VHA-L3-VLB, VHB-L1-VHA-L2-VLA-L3-VLB, VLB-L1-VLA-L2-VHA-L3-VHB or VLB-L1-VHA-L2-VLA-L3-VHB, wherein the VLA and VHA domains jointly form the antigen binding site for the first antigen, and VLB and VHB jointly form the antigen binding site for the second antigen.

The linker L1 particularly is a peptide of 2-10 amino acids, more particularly 3-7 amino acids, and most particularly 5 amino acids, and linker L3 particularly is a peptide of 1-10 amino acids, more particularly 2-7 amino acids, and most particularly 5 amino acids. In particular embodiments, the linker L1 and/or L3 comprises one or two units of four (4) glycine amino acid residues and one (1) serine amino acid residue (GGGGS)n, wherein n=1 or 2, preferably n=1.

The middle linker L2 particularly is a peptide of 10-40 amino acids, more particularly 15-30 amino acids, and most particularly 20-25 amino acids. In particular embodiments, said linker L2 comprises one or more units of four (4) glycine amino acid residues and one (1) serine amino acid residue (GGGGS)n, wherein n=1, 2, 3, 4, 5, 6, 7 or 8, preferably n=4.

In one embodiment, the multispecific antibody of the invention is a scDb-scFv. The term "scDb-scFv" refers to an antibody format, wherein a single-chain Fv (scFv) fragment is fused by a flexible Gly-Ser linker to a single-chain diabody (scDb). In one embodiment, said flexible Gly-Ser linker is a peptide of 2-40 amino acids, e.g., 2-35, 2-30, 2-25, 2-20, 2-15, 2-10 amino acids, particularly 10 amino acids. In particular embodiments, said linker comprises one or more units of four (4) glycine amino acid residues and one (1) serine amino acid residue (GGGGS)n, wherein n=1, 2, 3, 4, 5, 6, 7 or 8, preferably n=2.

In one embodiment of the present invention, the multispecific antibody of the invention is in a MATCH format described in WO 2016/202457; Egan T., et al., mAbs 9 (2017) 68-84.

The multispecific antibody of the invention can be produced using any convenient antibody manufacturing method known in the art (see, e.g., Fischer, N. & Leger, O., Pathobiology 74 (2007) 3-14 with regard to the production of bispecific constructs; Hornig, N. & Färber-Schwarz, A., Methods Mol. Biol. 907 (2012)713-727, and WO 99/57150 with regard to bispecific diabodies and tandem scFvs). Specific examples of suitable methods for the preparation of the bispecific construct of the invention further include, inter alia, the Genmab (see Labrijn et al., Proc. Natl. Acad. Sci. USA 110 (2013) 5145-5150) and Merus (see de Kruif et al., Biotechnol. Bioeng. 106 (2010) 741-750) technologies. Methods for production of bispecific antibodies comprising a functional antibody Fc part are also known in the art (see, e.g., Zhu et al., Cancer Lett. 86 (1994) 127-134); and Suresh et al., Methods Enzymol. 121 (1986) 210-228).

These methods typically involve the generation of monoclonal antibodies, for example by means of fusing myeloma cells with the spleen cells from a mouse that has been immunized with the desired antigen using the hybridoma technology (see, e.g., Yokoyama et al., Curr. Protoc. Immunol. Chapter 2, Unit 2.5, 2006) or by means of recombinant antibody engineering (repertoire cloning or phage display/yeast display) (see, e.g., Chames & Baty, FEMS Microbiol. Letters 189 (2000) 1-8), and the combination of the antigen-binding domains or fragments or parts thereof of two or more different monoclonal antibodies to give a bispecific or multispecific construct using known molecular cloning techniques.

The multispecific molecules of the invention can be prepared by conjugating the constituent binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-5-acetyl-thioacetate (SATA), 5,5'-dithiobis (2-nitrobenzoic acid) (DTNB), o-phenylenedimaleimide (oPDM), N-succinimidyl-3- (2-pyridyldithio)propionate (SPDP), and sulfosuccinimidyl 4- (N- maleimidomethyl)cyclohaxane-I-carboxylate (sulfo-SMCC) (see e.g., Karpovsky et al., 1984 J. Exp. Med. 160: 1686; Liu, M A et al., 1985 Proc. Natl. Acad. Sci. USA 82:8648). Other methods include those described in Paulus, 1985 Behring Ins. Mitt. No. 78, 118-132; Brennan et al., 1985 Science 229:81-83), and Glennie et al., 1987 J. Immunol. 139: 2367-2375). Conjugating agents are SATA and sulfo-SMCC, both available from Pierce Chemical Co. (Rockford, 111).

When the binding specificities are antibodies, they can be conjugated by sulfhydryl bonding of the C-terminus hinge regions of the two heavy chains. In a particularly embodiment, the hinge region is modified to contain an odd number of sulfhydryl residues, for example one, prior to conjugation.

Alternatively, two or more binding specificities can be encoded in the same vector and expressed and assembled in the same host cell. This method is particularly useful where the bispecific molecule is a mAb X mAb, mAb X Fab, Fab X F (ab')2 or ligand X Fab fusion protein. A multispecific antibody of the invention can be a single chain molecule comprising one single chain antibody and a binding determinant, or a single chain multispecific antibody comprising two binding determinants. Multispecific antibody may comprise at least two single chain molecules. Methods for preparing multispecific antibodies and molecules are described for example in U.S. Pat. No. 5,260,203; U.S. Pat. No. 5,455,030; U.S. Pat. No. 4,881,175; U.S. Pat. No. 5,132,405; U.S. Pat. No. 5,091,513; U.S. Pat. No. 5,476,786; U.S. Pat. No. 5,013,653; U.S. Pat. No. 5,258,498; and U.S. Pat. No. 5,482,858.

Binding of the multispecific antibodies to their specific targets can be confirmed by, for example, enzyme-linked immunosorbent assay (ELISA), radioimmunoassay (REA), FACS analysis, bioassay (e.g., growth inhibition), or Western Blot assay. Each of these assays generally detects the presence of protein-antibody complexes of particular interest by employing a labeled reagent (e.g., an antibody) specific for the complex of interest.

In a further aspect, the invention provides a nucleic acid encoding the multispecific antibody of the invention or fragments thereof or binding domains thereof. Such nucleic acid sequences can be optimized for expression in mammalian cells.

The term "nucleic acid" is used herein interchangeably with the term "polynucleotide(s)" and refers to one or more deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphorates, 2-O-methyl ribonucleotides, peptide-nucleic acids (PNAs). Unless otherwise indicated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes 8:91-98, 1994).

The invention provides substantially purified nucleic acid molecules which encode polypeptides comprising segments or domains of the multispecific antibody described above. When expressed from appropriate expression vectors, polypeptides encoded by these nucleic acid molecules are capable of exhibiting antigen binding capacity or capacities of the multispecific antibody of the present invention.

Also provided in the invention are polynucleotides which encode at least one CDR region and usually all three CDR regions of the binding domains of the multispecific antibody of the present invention set forth in Tables 1 to 4. Because of the degeneracy of the code, a variety of nucleic acid sequences will encode each of the immunoglobulin amino acid sequences.

The polynucleotide sequences can be produced by de novo solid-phase DNA synthesis or by PCR mutagenesis of an existing sequence (e.g., sequences as described in the Examples below) encoding the multispecific antibody of the invention or fragments thereof or binding domains thereof. Direct chemical synthesis of nucleic acids can be accomplished by methods known in the art, such as the phosphotriester method of Narang et al., 1979, Meth. Enzymol. 68:90; the phosphodiester method of Brown et al., Meth. Enzymol. 68: 109, 1979; the diethylphosphoramidite method of Beaucage et al., Tetra. Lett., 22: 1859, 1981; and the solid support method of U.S. Pat. No. 4,458,066. Introducing mutations to a polynucleotide sequence by PCR can be performed as described in, e.g., PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., Nucleic Acids Res. 19:967, 1991; and Eckert et al., PCR Methods and Applications 1:17, 1991.

Also provided in the invention are expression vectors and host cells for producing the multispecific antibody of the invention or fragments thereof or binding domains thereof.

The term "vector" is intended to refer to a polynucleotide molecule capable of transporting another polynucleotide to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome.

Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e.g., replication defective retroviruses, adenoviruses and adenoassociated viruses), which serve equivalent functions. In this particular context, the term "operably linked" refers to a functional relationship between two or more polynucleotide (e.g., DNA) segments. Typically, it refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, i.e., they are cis- acting. However, some transcriptional regulatory sequences, such as enhancers, need not be physically contiguous or located in close proximity to the coding sequences whose transcription they enhance.

Various expression vectors can be employed to express the polynucleotides encoding the multispecific antibody chains or binding fragments. Both viral-based and nonviral expression vectors can be used to produce the antibodies in a mammalian host cell. Nonviral vectors and systems include plasmids, episomal vectors, typically with an expression cassette for expressing a protein or RNA, and human artificial chromosomes (see, e.g., Harrington et al., Nat Genet. 15:345, 1997). For example, nonviral vectors useful for expression of the CD137-binding polynucleotides and polypeptides in mammalian (e.g., human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adenoassociated viruses, herpes viruses, vectors based on SV40, papilloma virus, HBP Epstein Barr virus, vaccinia virus vectors and Semliki Forest virus (SFV). See, Brent et al., supra; Smith, Annu. Rev. Microbiol. 49:807, 1995; and Rosenfeld et al., Cell 68: 143, 1992.

The choice of expression vector depends on the intended host cells in which the vector is to be expressed. Typically, the expression vectors contain a promoter and other regulatory sequences (e.g., enhancers) that are operably linked to the polynucleotides encoding a multispecific antibody chain or a fragment. In one embodiment, an inducible promoter is employed to prevent expression of inserted sequences except under inducing conditions. Inducible promoters include, e.g., arabinose, lacZ, metallothionein promoter or a heat shock promoter. Cultures of transformed organisms can be expanded under noninducing conditions without biasing the population for coding sequences whose expression products are better tolerated by the host cells. In addition to promoters, other regulatory elements may also be required or desired for efficient expression of a multispecific antibody chain or a fragment. These elements typically include an ATG initiation codon and adjacent ribosome binding site or other sequences. In addition, the efficiency of expression may be enhanced by the inclusion of enhancers appropriate to the cell system in use (see, e.g., Scharf et al., Results Probl. Cell Differ. 20: 125, 1994; and Bittner et al., Meth. Enzymol., 153:516, 1987). For example, the SV40 enhancer or CMV enhancer may be used to increase expression in mammalian host cells.

The expression vectors may also provide a secretion signal sequence position to form a fusion protein with polypeptides encoded by inserted the multispecific antibody of the invention or fragments thereof or binding domains thereof sequences. More often, the inserted the multispecific antibody of the invention or fragments thereof or binding domains thereof sequences are linked to signal sequences before inclusion in the vector. Vectors to be used to receive sequences encoding binding domains of the multispecific antibody light and heavy chain variable domains sometimes also encode constant regions or parts thereof. Such vectors allow expression of the variable regions as fusion proteins with the constant regions thereby leading to production of intact antibodies and antigen-binding fragments thereof. Typically, such constant regions are human.

The term "recombinant host cell" (or simply "host cell") refers to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The host cells for harboring and expressing the multispecific antibody of the invention or fragments thereof or binding domains thereof can be either prokaryotic or eukaryotic. E. coli is one prokaryotic host useful for cloning and expressing the polynucleotides of the present invention. Other microbial hosts suitable for use include bacilli, such as Bacillus subtilis, and other enterobacteriaceae, such as Salmonella, Serratia, and various Pseudomonas species. In these prokaryotic hosts, one can also make expression vectors, which typically contain expression control sequences compatible with the host cell (e.g., an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express CD137-binding polypeptides of the invention. Insect cells in combination with baculovirus vectors can also be used.

In one embodiment, mammalian host cells are used to express and produce the multispecific antibody of the invention or fragments thereof or binding domains thereof. For example, they can be either a hybridoma cell line expressing endogenous immunoglobulin genes or a mammalian cell line harboring an exogenous expression vector. These include any normal mortal or normal or abnormal immortal animal or human cell. For example, a number of suitable host cell lines capable of secreting intact immunoglobulins have been developed including the CHO cell lines, various Cos cell lines, HeLa cells, myeloma cell lines, transformed B-cells and hybridomas. The use of mammalian tissue cell culture to express polypeptides is discussed generally in, e.g., Winnacker, FROM GENES TO CLONES, VCH Publishers, N.Y., N.Y., 1987. Expression vectors for mammalian host cells can include expression control sequences, such as an origin of replication, a promoter, and an enhancer (see, e.g., Queen, et al., Immunol. Rev. 89:49-68, 1986), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP pollll promoter, the constitutive MPS V promoter, the tetracycline-inducible CMV promoter (such as the human immediate-early CMV promoter), the constitutive CMV promoter, and promoter- enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Sambrook, et al., supra). Other methods include, e.g., electroporation, calcium phosphate treatment, liposome-mediated transformation, injection and microinjection, ballistic methods, virosomes, immunoliposomes, polycatiomnucleic acid conjugates, naked DNA, artificial virions, fusion to the herpes virus structural protein VP22 (Elliot and O'Hare, Cell 88:223, 1997), agent-enhanced uptake of DNA, and ex vivo transduction. For long-term, high-yield production of recombinant proteins, stable expression will often be desired. For example, cell lines which stably express the multispecific antibody of the invention or fragments thereof or binding domains thereof can be prepared using expression vectors of the invention which contain viral origins of replication or endogenous expression elements and a selectable marker gene. Following the introduction of the vector, cells may be allowed to grow for 1-2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type. The present invention thus provides a method of producing the antibody of the invention or antigen-binding fragment thereof, wherein said method comprises the step of culturing a host cell comprising a nucleic acid or a vector encoding the antibody of the invention or antigen-binding fragment thereof, whereby said antibody of the disclosure or a fragment thereof is expressed.

In one aspect, the present invention relates to a method of producing the multispecific antibody of the invention or a binding domain thereof or a fragment thereof, the method comprising the step of culturing a host cell expressing a nucleic acid encoding the multispecific antibody of the invention or a binding domain thereof or a fragment thereof.

In a further aspect, the present invention relates to a pharmaceutical composition comprising the multispecific antibody of the invention, and a pharmaceutically acceptable carrier. Pharmaceutically acceptable carriers enhance or stabilize the composition, or facilitate preparation of the composition. Pharmaceutically acceptable carriers include solvents, dispersion media, coatings, antibacterial and antifungal agents, isotonic and absorption delaying agents, and the like that are physiologically compatible.

A pharmaceutical composition of the invention can be administered by a variety of methods known in the art. The route and/or mode of administration vary depending upon the desired results. Administration can be intravenous, intramuscular, intraperitoneal, or subcutaneous, or administered proximal to the site of the target. The pharmaceutically acceptable carrier should be suitable for intravenous, intramuscular, subcutaneous, parenteral, spinal or epidermal administration (e.g., by injection or infusion). Depending on the route of administration, the active compound, i.e., the multispecific antibody of the invention, may be coated in a material to protect the compound from the action of acids and other natural conditions that may inactivate the compound.

Pharmaceutical compositions of the invention can be prepared in accordance with methods well known and routinely practiced in the art. See, e.g., Remington: The Science and Practice of Pharmacy, Mack Publishing Co., 20th ed., 2000; and Sustained and Controlled Release Drug Delivery Systems, J. R. Robinson, ed., Marcel Dekker, Inc., New York, 1978. Pharmaceutical compositions are preferably manufactured under GMP conditions. Typically, a therapeutically effective dose or efficacious dose of the multispecific antibody of the invention is employed in the pharmaceutical compositions of the invention. The multispecific antibodies of the invention are formulated into pharmaceutically acceptable dosage forms by conventional methods known to those of skill in the art. Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions of the invention can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

The multispecific antibody of the invention is usually administered on multiple occasions. Intervals between single dosages can be weekly, monthly or yearly. Intervals can also be irregular as indicated by measuring blood levels of the multispecific antibody of the invention in the patient. Alternatively, the multispecific antibody of the invention can be administered as a sustained release formulation, in which case less frequent administration is required. Dosage and frequency vary depending on the half-life of the antibody in the patient. In general, humanized antibodies show longer half-life than that of chimeric antibodies and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

In one aspect, the present invention relates to the multispecific antibody of the invention or the pharmaceutical composition of the invention for use as a medicament. In a suitable embodiment, the present invention provides the multispecific antibody or the pharmaceutical composition for use in treatment of a proliferative disease, in particular a cancer in a subject in need thereof.

In another aspect, the present invention provides the multispecific antibody or the pharmaceutical composition for use in a manufacture of a medicament for treatment of a proliferative disease, in particular a cancer.

In another aspect, the present invention relates to use of the multispecific antibody or the pharmaceutical composition for treating a proliferative disease, in particular a cancer in a subject in need thereof.

In a further aspect, the present invention relates to use of the multispecific antibody or the pharmaceutical composition in the manufacture of a medicament for treatment of a proliferative disease, in particular a cancer, in a subject in need thereof.

In another aspect, the present invention relates to a method of treating a subject comprising administering to the subject a therapeutically effective amount of the multispecific antibody of the present invention. In a suitable embodiment, the present invention relates to a method of treating a proliferative disease, in particular a cancer in a subject comprising administering to the subject a therapeutically effective amount of the multispecific antibody of the present invention.

The term "subject" includes human and non-human animals. Non-human animals include all vertebrates, e.g., mammals and non-mammals, such as non-human primates, sheep, dog, cow, chickens, amphibians, and reptiles. Except when noted, the terms "patient" or "subject" are used herein interchangeably.

The terms "treatment", "treating", "treat", "treated", and the like, as used herein, refer to obtaining a desired pharmacologic and/or physiologic effect. The effect may be therapeutic in terms of a partial or complete cure for a disease and/or adverse effect attributable to the disease or delaying the disease progression. "Treatment", as used herein, covers any treatment of a disease in a mammal, e.g., in a human, and includes: (a) inhibiting the disease, i.e., arresting its development; and (b) relieving the disease, i.e., causing regression of the disease.

The term "therapeutically effective amount" or "efficacious amount" refers to the amount of an agent that, when administered to a mammal or other subject for treating a disease, is sufficient to effect such treatment for the disease. The "therapeutically effective amount" will vary depending on the agent, the disease and its severity and the age, weight, etc., of the subject to be treated.

In one embodiment, the proliferative disease is a cancer. The term "cancer" refers to a disease characterized by the rapid and uncontrolled growth of aberrant cells. Cancer cells can spread locally or through the bloodstream and lymphatic system to other parts of the body. The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors. The term "cancer" is used herein to mean a broad spectrum of tumors, including all solid and haematological malignancies. Examples of such tumors include, but are not limited to: a benign or especially malignant tumor, solid tumors, brain cancer, kidney cancer, liver cancer, adrenal gland cancer, bladder cancer, breast cancer, stomach cancer (e.g., gastric tumors), oesophageal cancer, ovarian cancer, cervical cancer, colon cancer, rectum cancer, prostate cancer, pancreatic cancer, lung cancer (e.g. non-small cell lung cancer and small cell lung cancer), vaginal cancer, thyroid cancer, melanoma (e.g., unresectable or metastatic melanoma), renal cell carcinoma, sarcoma, glioblastoma, multiple myeloma or gastrointestinal cancer, especially colon carcinoma or colorectal adenoma, a tumor of the neck and head, endometrial cancer, Cowden syndrome, Lhermitte-Duclos disease, Bannayan-Zonana syndrome, prostate hyperplasia, a neoplasia, especially of epithelial character, preferably mammary carcinoma or squamous cell carcinoma, chronic lymphocytic leukemia, chronic myelogenous leukemia (e.g., Philadelphia chromosome-positive chronic myelogenous leukemia), acute lymphoblastic leukemia (e.g., Philadelphia chromosome-positive acute lymphoblastic leukemia), non-Hodgkin's lymphoma, plasma cell myeloma, Hodgkin's lymphoma, a leukemia, and any combination thereof. In a preferred embodiment, the cancer is a lung cancer, preferably non-small cell lung cancer (NSCLC). In another embodiment, said cancer is a colorectal cancer.

The multispecific antibody of the present invention, or the composition of the present invention, inhibits the growth of solid tumors, but also liquid tumors. In a further embodiment, the proliferative disease is a solid tumor. The term "solid tumor" especially means a breast cancer, ovarian cancer, colon cancer, rectum cancer, prostate cancer, stomach cancer (especially gastric cancer), cervical cancer, lung cancer (e.g., non-small cell lung cancer and small cell lung cancer), and a tumor of the head and neck. Further, depending on the tumor type and the particular combination used, a decrease of the tumor volume can be obtained. The multispecific antibody of the present invention, or the composition of the present invention, is also suited to prevent the metastatic spread of tumors and the growth or development of micrometastases in a subject having a cancer.

In one embodiment, said cancer is PD-L1-positive, preferably wherein said cancer expresses high levels of PD-L1 in comparison to a healthy tissue, in particular wherein said cancer expresses PD-L1 (mRNA or protein) at least 2 times, at least 3 times, at least 4 times, at least 5 times, at least 6 times, at least 7 times, at least 8 times, at least 9 times, at least 10 times, at least 15 times, at least 20 times, at least 30 times, at least 40 times, at least 50 times, at least 60 times, at least 70 times, at least 80 times, at least 90 times, at least 100 times higher level in comparison to PD-L1 expression (mRNA or protein respectively) in a healthy tissue. In some embodiments, said cancer is malignant. In some embodiments, said cancer is benign. In some embodiments, said cancer is primary. In some embodiments, said cancer is secondary. In one embodiment, said cancer is lung cancer, preferably non-small cell lung cancer (NSCLC). In another embodiment, said cancer is colorectal cancer.

In one aspect, the present invention relates to a kit comprising the multispecific antibody of the invention or the pharmaceutical composition of the invention. The kit can include one or more other elements including: instructions for use; other reagents, e.g., a label, a therapeutic agent, or an agent useful for chelating, or otherwise coupling, an antibody to a label or therapeutic agent, or a radioprotective composition; devices or other materials for preparing the antibody molecule for administration; pharmaceutically acceptable carriers; and devices or other materials for administration to a subject. In a specific embodiment, the kit comprises the multispecific antibody of the invention in a pharmaceutically effective amount. In a further embodiment, the kit comprises a pharmaceutically effective amount of the multispecific antibody of the invention in lyophilized form and a diluent and, optionally, instructions for use. Said kit may further comprise a filter needle for reconstitution and a needle for injecting

**Sequence listing (mutations designated according to AHo numbering scheme, CDRs defined according to Numab CDR definition)**

TABLE 1. Examples of PDL1 binding domains of the present invention.

| SEQ ID NO: | Ab region | Sequence |
|---|---|---|
| 1 | HCDR1 | GFSFSSGYDMC |
| 2 | HCDR2 | CVVAGSVDITYYASWAKG |
| 3 | HCDR3 $_{33-03-G02}$ | RKDAYSDAFNL |
| 4 | HCDR3 $_{33-03-G02, VH-Y112A}$ | RKDAASDAFNL |
| 5 | LCDR1 | QASQSINDYLA |
| 6 | LCDR2 | KASTLAS |
| 7 | LCDR3 $_{33-03-G02}$ | QQGYIITDIDNV |
| 8 | LCDR3 $_{33-03-G02, VL-Q108A}$ | QAGYIITDIDNV |
| 9 | LCDR3 $_{33-03-G02, VL-G109A}$ | QQAYIITDIDNV |
| 10 | LCDR3 $_{33-03-G02, VL-Q108A, VL-G109A}$ | QAAYIITDIDNV |
| 11 | VH $_{33-03-G02}$ (PRO830; PRO1075; PRO1076; PRO1434) | QVQLQESGPGLVKPSETLSLTCKVSGFSFSSGYDMCWIRQPPGKGLEWIGCVVAGSVDITYYASWAKGRVTISVDSSKNQFSLKLSSVTAADTAVYYCARKDAYSDAFNLWGQGTLVTVSS |
| 12 | VH $_{33-03-G02, VH-Y112A}$ (PRO1089; PRO1494) | QVQLQESGPGLVKPSETLSLTCKVSGFSFSSGYDMCWIRQPPGKGLEWIGCVVAGSVDITYYASWAKGRVTISVDSSKNQFSLKLSSVTAADTAVYYCARKDAASDAFNLWGQGTLVTVSS |
| 13 | VL$_{33-03-G02}$ (PRO830; PRO1089) | DIQMTQSPSSLSASVGDRVTITCQASQSINDYLAWYQQKPGKAPKLLIYKASTLASGVPSRFSGSGSGTDFTLTISSLQPEDFATYYCQQGYIITDIDNVFGTGTKVTVLG |
| 14 | VL $_{33-03-G02, VL-Q108A}$ (PRO1075; PRO1494) | DIQMTQSPSSLSASVGDRVTITCQASQSINDYLAWYQQKPGKAPKLLIYKASTLASGVPSRFSGSGSGTDFTLTISSLQPEDFATYYCQAGYIITDIDNVFGTGTKVTVLG |
| 15 | VL $_{33-03-G02, VL-G109A}$ (PRO1076) | DIQMTQSPSSLSASVGDRVTITCQASQSINDYLAWYQQKPGKAPKLLIYKASTLASGVPSRFSGSGSGTDFTLTISSLQPEDFATYYCQQAYIITDIDNVFGTGTKVTVLG |

(continued)

| SEQ ID NO: | Ab region | Sequence |
|---|---|---|
| 16 | VL 33-03-G02, VL-Q108A, VL-G109A (PRO1434) | DIQMTQSPSSLSASVGDRVTITCQASQSINDYLAWYQQKPGKAPKLLIYKASTLASGVPSRFSGSGSGTDFTLTISSLQPEDFATYYCQAAYIITDIDNVFGTGTKVTVLG |

*Table2. Examples of HER2 binding domains of the present invention.*

| SEQ ID NO: | Description | Sequence |
|---|---|---|
| 17 | HCDR1 Trastuzumab | GFNIKDTYIH |
| 18 | HCDR2 Trastuzumab | RIYPTNGYTRYADSVKG |
| 19 | HCDR3 Trastuzumab | RWGGDGFYAMDY |
| 20 | LCDR 1 Trastuzumab | RASQDVNTAVA |
| 21 | LCDR2 Trastuzumab | SASFLYS |
| 22 | LCDR3 Trastuzumab | QQHYTTPPT |
| 23 | VH Trastuzumab | EVQLVESGGGLVQPGGSLRLSCAASGFNIKDTYIHWVRQAPGK**G**LEWVARIYPTNGYTRYADSVKGRFTISADTSKNTAYLQMNSLRAEDTAVYYCSRWGGDGFYAMDYWGQGTLVTVSS |
| 24 | VH Trastuzumab (G51C) | EVQLVESGGGLVQPGGSLRLSCAASGFNIKDTYIHWVRQAPGK**C**LEWVARIYPTNGYTRYADSVKGRFTISADTSKNTAYLQMNSLRAEDTAVYYCSRWGGDGFYAMDYWGQGTLVTVSS |
| 25 | VL Trastuzumab (λ-capped) | DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFG**G**GTKLTVLG |
| 26 | VL Trastuzumab (G141C) (λ-capped) | DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFG**C**GTKLTVLG |
| 27 | HCDR1 Pertuzumab | GFTFTDYTMD |
| 28 | HCDR2 Pertuzumab | DVNPNSGGSIYNQRFKG |
| 29 | HCDR3 Pertuzumab | RNLGPSFYFDY |
| 30 | LCDR 1 Pertuzumab | KASQDVSIGVA |
| 31 | LCDR2 Pertuzumab | SASYRYT |
| 32 | LCDR3 Pertuzumab | QQYYIYPYT |
| 33 | VH Pertuzumab | EVQLVESGGGLVQPGGSLRLSCAASGFTFTDYTMDWVRQAPGKGLEWVADVNPNSGGSIYNQRFKGRFTLSVDRSKNTLYLQMNSLRAEDTAVYYCARNLGPSFYFDYWGQGTLVTVSS |

| SEQ ID NO: | Description | Sequence |
|---|---|---|
| 34 | VH Pertuzumab (G51C) | EVQLVESGGGLVQPGGSLRLSCAASGFTFTDYTMDWVRQAPGKCLEWVADVN PNSGGSIYNQRFKGRFTLSVDRSKNTLYLQMNSLRAEDTAVYYCARNLGPSFYF DYWGQGTLVTVSS |
| 35 | VL Pertuzumab (λ-capped) | DIQMTQSPSSLSASVGDRVTITCKASQDVSIGVAWYQQKPGKAPKLLIYSASYR YTGVPSRFSGSGSGTDFTLTISSLQPEDFATYYCQQYYIYPYTFGTGTKVTVLG |
| 36 | VL Pertuzumab (G141C) (λ-capped) | DIQMTQSPSSLSASVGDRVTITCKASQDVSIGVAWYQQKPGKAPKLLIYSASYR YTGVPSRFSGSGSGTDFTLTISSLQPEDFATYYCQQYYIYPYTFGCGTKVTVLG |

*Table3. Examples of CD3 binding domains of the present invention.*

| SEQ ID NO: | Description | Sequence |
|---|---|---|
| 37 | HCDR1 | GFSLSSYDMS |
| 38 | HCDR2 | ASYASGPTYYASWAKG |
| 39 | HCDR3 | RGGWTGTSHSNI |
| 40 | LCDR1 | QSSQSVFSNNYLA |
| 41 | LCDR2 | SASTLAS |
| 42 | LCDR3 | LGSYACSSADCYV |
| 43 | VH 28-21-D09 sc04 | EVQLVESGGGLVQPGGSLRLSCAASGFSLSSYDMSWVRQAPGKGLAWIGASYASG PTYYASWAKGRFTISRDNSKNTVYLQMNSLRAEDTATYFCARGGWTGTSHSNIWG QGTLVTVSS |
| 44 | VL 28-21-D09 sc04 | DIQMTQSPSSLSASVGDRVTITCQSSQSVFSNNYLAWFQQKPGQSPKRLIYSASTLA SGVPSRFSGSGSGTDFTLTISSLQPEDFATYYCLGSYACSSADCYVFGTGTKVTVLG |

*Table 4. Examples of human serum albumin (HSA) binding domains of the present invention.*

| SEQ ID NO: | Description | Sequence |
|---|---|---|
| Anti-HSA domain 19-01-H04-sc03 | | |
| 45 | HCDR1 | GFSLSSNAMG |
| 46 | HCDR2 | IISVGGFTYYASWAKG |
| 47 | HCDR3 | RDRHGGDSSGAFYL |
| 48 | LCDR1 | QSSESVYSNNQLS |
| 49 | LCDR2 | DASDLAS |
| 50 | LCDR3 | AGGFSSSSDTA |
| 51 | VH$_{19-01-H04-sc03}$ | EVQLVESGGGLVQPGGSLRLSCAASGFSLSSNAMGWVRQAPGKGLEYIGIISVGG FTYYASWAKGRFTISRDNSKNTVYLQMNSLRAEDTATYFCARDRHGGDSSGAFY LWGQGTLVTVSS |
| 52 | VL$_{19-01-H04-sc03}$ | DIQMTQSPSSLSASVGDRVTITCQSSESVYSNNQLSWYQQKPGQPPKLLIYDASDL ASGVPSRFSGSGSGTDFTLTISSLQPEDFATYYCAGGFSSSSDTAFGGGTKLTVLG |
| Anti-HSA domain 23-13-A01-sc03 | | |
| 53 | HCDR1 | GFSFSSSYWIC |
| 54 | HCDR2 | CVFTGDGTTYYASWAKG |
| 55 | HCDR3 | RPVSVYYYGMDL |
| 56 | LCDR1 | QASQIISSRSA |
| 57 | LCDR2 | QASKLAS |
| 58 | LCDR3 | QCTYIDSNFGA |
| 59 | VH$_{23-13-A01-sc03}$ | EVQLVESGGGLVQPGGSLRLSCAASGFSFSSSYWICWVRQAPGKGLEWVGCVF TGDGTTYYASWAKGRFTISRDNSKNTVYLQMNSLRAEDTATYFCARPVSVYYY GMDLWGQGTLVTVSS |
| 60 | VL$_{23-13-A01-sc03}$ | DVVMTQSPSSLSASVGDRVTITCQASQIISSRSAWYQQKPGQPPKLLIYQASKLA SGVPSRFSGSGSGTDFTLTISSLQPEDFATYYCQCTYIDSNFGAFGGGTKLTVLG |

Table 5. Examples of CD137 binding domains of the present invention.

| SEQ ID NO: | Description | Sequence |
|---|---|---|
| Anti-CD137 domain 38-27-A11 sc07 | | |
| 61 | HCDR1 | GFSFSANYYPC |
| 62 | HCDR2 | CIYGGSSDITYDANWTKG |
| 63 | HCDR3 | RSAWYSGWGGDL |
| 64 | LCDR1 | QASQSISNRLA |
| 65 | LCDR2 | SASTLAS |
| 66 | LCDR3 | QSTYYGNDGNA |
| 67 | VH 38-27-A11 sc07 (G51C) | EVQLVESGGGLVQPGGSLRLSCAASGFSFSANYYPCWVRQAPGKCLEWIGCIYGGSSDITYDANWTKGRFTISRDNSKNTVYLQMNSLRAEDTAVYYCARSAWYSGWGGDLWGQGTLVTVSS |
| 68 | VL 38-27-A11 sc07 (G141C) | DIQMTQSPSSLSASVGDRVTITCQASQSISNRLAWYQQKPGKAPKLLIYSASTLASGVPSRFSGSGSGTDFTLTISSLQPEDFATYYCQSTYYGNDGNAFGCGTKVTVLG |

Table 6. Examples of multispecific molecules of the present invention.

| SEQ ID NO: | Description | Sequence |
|---|---|---|
| **Constructs of Example 2: anti-HER2xPDL1(high KD)xCD3 (xHSA)** | | |
| **PRO1454 (Tribody)** | | |
| 69 | CHAIN_1PRO1454 | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGQGTKVEIKRTVAAPSVF IFPPSDEQLKSGTASVVCLLNNFYPREAKVQWKVDNALQSGNSQESVTEQDSKDST YSLSSTLTLSKADYEKHKVYACEVTHQGLSSPVTKSFNRGECGGGGSGGGGSDIQM TQSPSSLSASVGDRVTITCQSSQSVFSNNYLAWFQQKPGQSPKRLIYSASTLASGVPS RFSGSGSGTDFTLTISSLQPEDFATYYCLGSYACSSADCYVFGTGTKVTVLGGGGGS GGGGSGGGGSGGGGSEVQLVESGGGLVQPGGSLRLSCAASGFSLSSYDMSWVRQA PGKGLAWIGASYASGPTYYASWAKGRFTISRDNSKNTVYLQMNSLRAEDTATYFCA RGGWTGTSHSNIWGQGTLVTVSS |
| 70 | CHAIN_2PRO1454 | EVQLVESGGGLVQPGGSLRLSCAASGFNIKDTYIHWVRQAPGKGLEWVARIYPTNG YTRYADSVKGRFTISADTSKNTAYLQMNSLRAEDTAVYYCSRWGGDGFYAMDYW GQGTLVTVSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPKSCG GGGSGGGGSDIQMTQSPSSLSASVGDRVTITCQASQSINDYLAWYQQKPGKAPKLLI YKASTLASGVPSRFSGSGSGTDFTLTISSLQPEDFATYYCQQAYIITDIDNVFGTGTKV TVLGGGGGSGGGGSGGGGSGGGGSQVQLQESGPGLVKPSETLSLTCKVSGFSFSSG YDMCWIRQPPGKGLEWIGCVVAGSVDITYYASWAKGRVTISVDSSKNQFSLKLSSV TAADTAVYYCARKDAYSDAFNLWGQGTLVTVSS |
| **PRO1455 (Tribody)** | | |
| 71 | CHAIN_1PRO1455 | DIQMTQSPSSLSASVGDRVTITCQASENIYSFLAWYQQKPGKAPKLLIYSASKLAAGV PSRFSGSGSGTDFTLTISSLQPEDFATYYCQQTNRYSNPDIYNVFGTGTKVTVLGTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGECGGGGSGGGGS |

| PRO1455 (Tribody) | | |
|---|---|---|
| | | DIQMTQSPSSLSASVGDRVTITCQSSQSVFSNNYLAWFQQKPGQSPKRLIYSASTLAS GVPSRFSGSGSGTDFTLTISSLQPEDFATYYCLGSYACSSADCYVFGTGTKVTVLGGG GGSGGGGSGGGGSGGGGSEVQLVESGGGLVQPGGSLRLSCAASGFSLSSYDMSWV RQAPGKGLAWIGASYASGPTYYASWAKGRFTISRDNSKNTVYLQMNSLRAEDTAT YFCARGGWTGTSHSNIWGQGTLVTVSS |
| 72 | CHAIN_2<sub>PRO1455</sub> | EVQLVESGGGLVQPGGSLRLSCAASGIDFNSNYYMCWVRQAPGKGLEWIGCIYVGS HVNTYYANWAKGRFTISRDNSKNTVYLQMNSLRAEDTAVYYCATSGSSVLYFKFW GQGTLVTVSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPKSCG GGGSGGGGSDIQMTQSPSSLSASVGDRVTITCQASQSINDYLAWYQQKPGKAPKLLI YKASTLASGVPSRFSGSGSGTDFTLTISSLQPEDFATYYCQQAYIITDIDNVFGTGTKV TVLGGGGGSGGGGSGGGGSGGGGSQVQLQESGPGLVKPSETLSLTCKVSGFSFSSG YDMCWIRQPPGKGLEWIGCVVAGSVDITYYASWAKGRVTISVDSSKNQFSLKLSSV TAADTAVYYCARKDAYSDAFNLWGQGTLVTVSS |
| PRO1456 (Tribody) | | |
| 73 | CHAIN_1<sub>PRO1456</sub> | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGQGTKVEIKRTVAAPSVF IFPPSDEQLKSGTASVVCLLNNFYPREAKVQWKVDNALQSGNSQESVTEQDSKDST YSLSSTLTLSKADYEKHKVYACEVTHQGLSSPVTKSFNRGECGGGGSGGGGSDIQM TQSPSSLSASVGDRVTITCQSSQSVFSNNYLAWFQQKPGQSPKRLIYSASTLASGVPS RFSGSGSGTDFTLTISSLQPEDFATYYCLGSYACSSADCYVFGTGTKVTVLGGGGGS GGGGSGGGGSEVQLVESGGGLVQPGGSLRLSCAASGFSLSSYDMSWVRQA PGKGLAWIGASYASGPTYYASWAKGRFTISRDNSKNTVYLQMNSLRAEDTATYFCA RGGWTGTSHSNIWGQGTLVTVSS |

**PRO1456 (Tribody)**

| 74 | CHAIN_2PRO1456 | EVQLVESGGGLVQPGGSLRLSCAASGFNIKDTYIHWVRQAPGKGLEWVARIYPTNG YTRYADSVKGRFTISADTSKNTAYLQMNSLRAEDTAVYYCSRWGGDGFYAMDYW GQGTLVTVSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPKSCG GGGSGGGGSDIQMTQSPSSLSASVGDRVTITCQASENIYSFLAWYQQKPGKAPKLLI YSASKLAAGVPSRFSGSGSGTDFTLTISSLQPEDFATYYCQQTNRYSNPDIYNVFGTG TKVTVLGGGGSGGGGSGGGGSGGGGSEVQLVESGGGLVQPGGSLRLSCAASGIDF NSNYYMCWVRQAPGKGLEWIGCIYGSHVNTYYANWAKGRFTISRDNSKNTVYLQ MNSLRAEDTAVYYCATSGSSVLYFKFWGQGTLVTVSS |

**PRO1497 (Tribody)**

| 75 | CHAIN_1PRO1497 | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGQGTKVEIKRTVAAPSVF IFPPSDEQLKSGTASVVCLLNNFYPREAKVQWKVDNALQSGNSQESVTEQDSKDST YSLSSTLTLSKADYEKHKVYACEVTHQGLSSPVTKSFNRGECGGGGSGGGGSDIQM TQSPSSLSASVGDRVTITCQSSQSVFSNNYLAWFQQKPGQSPKRLIYSASTLASGVPS RFSGSGSGTDFTLTISSLQPEDFATYYCLGSYACSSADCYVFGTGTKVTVLGGGGGS GGGGSGGGGSGGGGSEVQLVESGGGLVQPGGSLRLSCAASGFSLSSYDMSWVRQA PGKGLAWIGASYASGPTYYASWAKGRFTISRDNSKNTVYLQMNSLRAEDTATYFCA RGGWTGTSHSNIWGQGTLVTVSS |
| 76 | CHAIN_2PRO1497 | EVQLVESGGGLVQPGGSLRLSCAASGFNIKDTYIHWVRQAPGKGLEWVARIYPTNG YTRYADSVKGRFTISADTSKNTAYLQMNSLRAEDTAVYYCSRWGGDGFYAMDYW GQGTLVTVSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPKSCG GGGSGGGGSDIQMTQSPSSLSASVGDRVTITCQASQSINDYLAWYQQKPGKAPKLLI YKASTLASGVPSRFSGSGSGTDFTLTISSLQPEDFATYYCQAAYIITDIDNVFGTGTKV TVLGGGGSGGGGSGGGGSGGGGSQVQLQESGPGLVKPSETLSLTCKVSGFSFSSG YDMCWIRQPPGKGLEWIGCVVAGSVDITYYASWAKGRVTISVDSSKNQFSLKLSSV TAADTAVYYCARKDAYSDAFNLWGQGTLVTVSS |

EP 3 816 185 A1

| PRO1498 (Tribody) | | |
|---|---|---|
| 77 | CHAIN_1<sub>PRO1498</sub> | DIQMTQSPSSLSASVGDRVTITCQASENIYSFLAWYQQKPGKAPKLLIYSASKLAAGV PSRFSGSGSGTDFTLTISSLQPEDFATYYCQQTNRYSNPDIYNVFGTGTKVTVLGTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGECGGGGSGGGGS DIQMTQSPSSLSASVGDRVTITCQSSQSVFSNNYLAWFQQKPGQSPKRLIYSASTLAS GVPSRFSGSGSGTDFTLTISSLQPEDFATYYCLGSYACSSADCYVFGTGTKVTVLGGG GGSGGGGSGGGGSGGGGSEVQLVESGGGLVQPGGSLRLSCAASGFSLSSYDMSWV RQAPGKGLAWIGASYASGPTYYASWAKGRFTISRDNSKNTVYLQMNSLRAEDTAT YFCARGGWTGTSHSNIWGQGTLVTVSS |
| 78 | CHAIN_2<sub>PRO1498</sub> | EVQLVESGGGLVQPGGSLRLSCAASGIDFNSNYYMCWVRQAPGKGLEWIGCIYVGS HVNTYYANWAKGRFTISRDNSKNTVYLQMNSLRAEDTAVYYCATSGSSVLYFKFW GQGTLVTVSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPKSCG GGGSGGGGSDIQMTQSPSSLSASVGDRVTITCQASQSINDYLAWYQQKPGKAPKLLI YKASTLASGVPSRFSGSGSGTDFTLTISSLQPEDFATYYCQAAYIITDIDNVFGTGTKV TVLGGGGGSGGGGSGGGGSGGGGSQVQLQESGPGLVKPSETLSLTCKVSGFSFSSG YDMCWIRQPPGKGLEWIGCVVAGSVDITYYASWAKGRVTISVDSSKNQFSLKLSSV TAADTAVYYCARKDAYSDAFNLWGQGTLVTVSS |
| PRO1543 (MATCH4) | | |
| 79 | CHAIN_1<sub>PRO1543</sub> | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKLTVLGGGGGSGG GGSGGGGSEVQLVESGGGLVQPGGSLRLSCAASGFNIKDTYIHWVRQAPGK CLEWVARIYPTNGYTRYADSVKGRFTISADTSKNTAYLQMNSLRAEDTAVYYCSR WGGDGFYAMDYWGQGTLVTVSSGGGGSGGGGSDVVMTQSPSSLSASVGDRVTITC QASQIISSRSAWYQQKPGQPPKLLIYQASKLASGVPSRFSGSGSGTDFTLTISSLQPED FATYYCQCTYIDSNFGAFGCGTKLTVLGGGSGGSDIQMTQSPSSLSASVGDRVTITC QSSQSVFSNNYLAWFQQKPGQSPKRLIYSASTLASGVPSRFSGSGSGTDFTLTISSLQP EDFATYYCLGSYACSSADCYVFGTGTKVTVLG |

**PRO1543 (MATCH4)**

| 80 | CHAIN_2<sub>PRO1543</sub> | DIQMTQSPSSLSASVGDRVTITCQASQSINDYLAWYQQKPGKAPKLLIYKASTLASG VPSRFSGSGSGTDFTLTISSLQPEDFATYYCQAAYIITDIDNVFGTGTKVTVLGGGGGS GGGGSGGGGSGGGGSQVQLQESGPGLVKPSETLSLTCKVSGFSFSSGYDMCWIRQPP GKGLEWIGCVVAGSVDITYYASWAKGRVTISVDSSKNQFSLKLSSVTAADTAVYYC ARKDAYSDAFNLWGQGTLVTVSSGGGGSGGGGSEVQLVESGGGLVQPGGSLRLSC AASGFSLSSYDMSWVRQAPGKGLAWIGASYASGPTYYASWAKGRFTISRDNSKNTV YLQMNSLRAEDTATYFCARGGWTGTSHSNIWGQGTLVTVSSGGGSGGGGSEVQLVE SGGGLVQPGGSLRLSCAASGFSFSSSYWICWVRQAPGKCLEWVGCVFTGDGTTYYA SWAKGRFTISRDNSKNTVYLQMNSLRAEDTATYFCARPVSVYYYGMDLWGQGTLV TVSS |

**PRO1544 (MATCH4)**

| 81 | CHAIN_1<sub>PRO1544</sub> | DIQMTQSPSSLSASVGDRVTITCQASQSINDYLAWYQQKPGKAPKLLIYKASTLASG VPSRFSGSGSGTDFTLTISSLQPEDFATYYCQAAYIITDIDNVFGTGTKVTVLGGGGGS GGGGSGGGGSGGGGSQVQLQESGPGLVKPSETLSLTCKVSGFSFSSGYDMCWIRQPP GKGLEWIGCVVAGSVDITYYASWAKGRVTISVDSSKNQFSLKLSSVTAADTAVYYC ARKDAYSDAFNLWGQGTLVTVSSGGGGSGGGGSDVVMTQSPSSLSASVGDRVTITC QASQIISSRSAWYQQKPGQPPKLLIYQASKLASGVPSRFSGSGSGTDFTLTISSLQPED FATYYCQCTYIDSNFGAFGCGTKLTVLGGGSGGSDIQMTQSPSSLSASVGDRVTITC QSSQSVFSNNYLAWFQQKPGQSPKRLIYSASTLASGVPSRFSGSGSGTDFTLTISSLQP EDFATYYCLGSYACSSADCYVFGTGTKVTVLG |

EP 3 816 185 A1

| PRO1544 (MATCH4) | | |
|---|---|---|
| 82 | CHAIN_2<sub>PRO1544</sub> | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKLTVLGGGGGSGG GGSGGGGSGGGGSEVQLVESGGGLVQPGGSLRLSCAASGFNIKDTYIHWVRQAPGK CLEWVARIYPTNGYTRYADSVKGRFTISADTSKNTAYLQMNSLRAEDTAVYYCSR WGGDGFYAMDYWGQGTLVTVSSGGGGSGGGGSEVQLVESGGGLVQPGGSLRLSC AASGFSLSSYDMSWVRQAPGKGLAWIGASYASGPTYYASWAKGRFTISRDNSKNTV YLQMNSLRAEDTATYFCARGGWTGTSHSNIWGQGTLVTVSSGGGSGGGSEVQLVE SGGGLVQPGGSLRLSCAASGFSFSSSYWICWVRQAPGKCLEWVGCVFTGDGTTYYA SWAKGRFTISRDNSKNTVYLQMNSLRAEDTATYFCARPVSVYYYGMDLWGQGTLV TVSS |
| PRO1545 (MATCH4) | | |
| 83 | CHAIN_1<sub>PRO1545</sub> | DIQMTQSPSSLSASVGDRVTITCQASQSINDYLAWYQQKPGKAPKLLIYKASTLASG VPSRFSGSGSGTDFTLTISSLQPEDFATYYCQAAYIITDIDNVFGTGTKVTVLGGGGGS GGGGSGGGGSGGGGSQVQLQESGPGLVKPSETLSLTCKVSGFSFSSGYDMCWIRQPP GKGLEWIGCVVAGSVDITYYASWAKGRVTISVDSSKNQFSLKLSSVTAADTAVYYC ARKDAYSDAFNLWGQGTLVTVSSGGGGSGGGGSDVVMTQSPSSLSASVGDRVTITC QASQIISSRSAWYQQKPGQPPKLLIYQASKLASGVPSRFSGSGSGTDFTLTISSLQPED FATYYCQCTYIDSNFGAFGCGTKLTVLGGGSGGSDIQMTQSPSSLSASVGDRVTITC QSSQSVFSNNYLAWFQQKPGQSPKRLIYSASTLASGVPSRFSGSGSGTDFTLTISSLQP EDFATYYCLGSYACSSADCYVFGTGTKVTVLG |
| 84 | CHAIN_2<sub>PRO1545</sub> | DIQMTQSPSSLSASVGDRVTITCQASENIYSFLAWYQQKPGKAPKLLIYSASKLAAGV PSRFSGSGSGTDFTLTISSLQPEDFATYYCQQTNRYSNPDIYNVFGTGTKVTVLGGGG GSGGGGSGGGGSEVQLVESGGGLVQPGGSLRLSCAASGIDFNSNYYMCWV RQAPGKGLEWIGCIYVGSHVNTYYANWAKGRFTISRDNSKNTVYLQMNSLRAEDT AVYYCATSGSSVLYFKFWGQGTLVTVSSGGGGSGGGGSEVQLVESGGGLVQPGGS LRLSCAASGFSLSSYDMSWVRQAPGKGLAWIGASYASGPTYYASWAKGRFTISRDN SKNTVYLQMNSLRAEDTATYFCARGGWTGTSHSNIWGQGTLVTVSSGGGSGGGSE |

| PRO1545 (MATCH4) | | |
| --- | --- | --- |
| | | VQLVESGGGLVQPGGSLRLSCAASGFSFSSSYWICWVRQAPGKCLEWVGCVFTGDGTTYYASWAKGRFTISRDNSKNTVYLQMNSLRAEDTATYFCARPVSVYYYGMDLWGQGTLVTVSS |
| PRO1546 (MATCH4) | | |
| 85 | CHAIN_1$_{PRO1546}$ | DIQMTQSPSSLSASVGDRVTITCQASENIYSFLAWYQQKPGKAPKLLIYSASKLAAGVPSRFSGSGSGTDFTLTISSLQPEDFATYYCQQTNRYSNPDIYNVFGTGTKVTVLGGGGGSGGGGSGGGGSGGGGSEVQLVESGGGLVQPGGSLRLSCAASGIDFNSNYYMCWVRQAPGKGLEWIGCIYVGSHVNTYYANWAKGRFTISRDNSKNTVYLQMNSLRAEDTAVYYCATSGSSVLYFKFWGQGTLVTVSSGGGGSGGGGSDVVMTQSPSSLSASVGDRVTITCQASQIISSRSAWYQQKPGQPPKLLIYQASKLASGVPSRFSGSGSGTDFTLTISSLQPEDFATYYCQCTYIDSNFGAFGCGTKLTVLGGGSGGSDIQMTQSPSSLSASVGDRVTITCQSSQSVFSNNYLAWFQQKPGQSPKRLIYSASTLASGVPSRFSGSGSGTDFTLTISSLQPEDFATYYCLGSYACSSADCYVFGTGTKVTVLG |
| 86 | CHAIN_2$_{PRO1546}$ | DIQMTQSPSSLSASVGDRVTITCQASQSINDYLAWYQQKPGKAPKLLIYKASTLASGVPSRFSGSGSGTDFTLTISSLQPEDFATYYCQAAYIITDIDNVFGTGTKVTVLGGGGGSGGGGSGGGGSQVQLQESGPGLVKPSETLSLTCKVSGFSFSSGYDMCWIRQPPGKGLEWIGCVVAGSVDITYYASWAKGRVTISVDSSKNQFSLKLSSVTAADTAVYYCARKDAYSDAFNLWGQGTLVTVSSGGGGSGGGGSEVQLVESGGGLVQPGGSLRLSCAASGFSLSSYDMSWVRQAPGKGLAWIGASYASGPTYYASWAKGRFTISRDNSKNTVYLQMNSLRAEDTATYFCARGGWTGTSHSNIWGQGTLVTVSSGGGSGGGGSEVQLVESGGGLVQPGGSLRLSCAASGFSFSSSYWICWVRQAPGKCLEWVGCVFTGDGTTYYASWAKGRFTISRDNSKNTVYLQMNSLRAEDTATYFCARPVSVYYYGMDLWGQGTLVTVSS |

EP 3 816 185 A1

**PRO1547 (DVD-Tribody)**

| 87 | CHAIN_1<sub>PRO1547</sub> | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGQGTKVEIKRTVAAPSVF IFPPDVVMTQSPSSLSASVGDRVTITCQASQIISSRSAWYQQKPGQPPKLLIYQASKLA SGVPSRFSGSGSGTDFTLTISSLQPEDFATYYCQCTYIDSNFGAFGCGTKLTVLGTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGECGGGGSGGGGS DIQMTQSPSSLSASVGDRVTITCQSSQSVFSNNYLAWFQQKPGQSPKRLIYSASTLAS GVPSRFSGSGSGTDFTLTISSLQPEDFATYYCLGSYACSSADCYVFGTGTKVTVLGGG<br><br>GGSGGGGSGGGGSGGGGSEVQLVESGGGLVQPGGSLRLSCAASGFSLSSYDMSWV RQAPGKGLAWIGASYASGPTYYASWAKGRFTISRDNSKNTVYLQMNSLRAEDTAT YFCARGGWTGTSHSNIWGQGTLVTVSS |
|---|---|---|
| 88 | CHAIN_2<sub>PRO1547</sub> | EVQLVESGGGLVQPGGSLRLSCAASGFNIKDTYIHWVRQAPGKGLEWVARIYPTNG YTRYADSVKGRFTISADTSKNTAYLQMNSLRAEDTAVYYCSRWGGDGFYAMDYW GQGTLVTVSSASTKGPSVFPLAPEVQLVESGGGLVQPGGSLRLSCAASGFSFSSSYWI CWVRQAPGKCLEWVGCVFTGDGTTYYASWAKGRFTISRDNSKNTVYLQMNSLRA EDTATYFCARPVSVYYYGMDLWGQGTLVTVSSASTKGPSVFPLAPSSKSTSGGTAA LGCLVKDYFPEPVTVSWNSGALTSGVHTFPAVLQSSGLYSLSSVVTVPSSSLGTQTYI CNVNHKPSNTKVDKKVEPKSCGGGGSGGGGSIQMTQSPSSLSASVGDRVTITCQAS QSINDYLAWYQQKPGKAPKLLIYKASTLASGVPSRFSGSGSGTDFTLTISSLQPEDFA TYYCQAAYIITDIDNVFGTGTKVTVLGGGGGSGGGGSGGGGSGGGGSQVQLQESGP GLVKPSETLSLTCKVSGFSFSSGYDMCWIRQPPGKGLEWIGCVVAGSVDITYYASWA KGRVTISVDSSKNQFSLKLSSVTAADTAVYYCARKDAYSDAFNLWGQGTLVTVSS |

EP 3 816 185 A1

EP 3 816 185 A1

**PRO1548 (DVD-Tribody)**

| 89 | CHAIN_1PRO1548 | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGQGTKVEIKRTVAAPSVF IFPPDVVMTQSPSSLSASVGDRVTITCQASQIISSRSAWYQQKPGQPPKLLIYQASKLA SGVPSRFSGSGSGTDFTLTISSLQPEDFATYYCQCTYIDSNFGAFGCGTKLTVLGTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGECGGGGSGGGGS DIQMTQSPSSLSASVGDRVTITCQSSQSVFSNNYLAWFQQKPGQSPKRLIYSASTLAS GVPSRFSGSGSGTDFTLTISSLQPEDFATYYCLGSYACSSADCYVFGTGTKVTVLGGG GGSGGGGSGGGGSGGGGSEVQLVESGGGLVQPGGSLRLSCAASGFSLSSYDMSWV RQAPGKGLAWIGASYASGPTYYASWAKGRFTISRDNSKNTVYLQMNSLRAEDTAT YFCARGGWTGTSHSNIWGQGTLVTVSS |
|----|----------------|---|
| 90 | CHAIN_2PRO1548 | EVQLVESGGGLVQPGGSLRLSCAASGFNIKDTYIHWVRQAPGKGLEWVARIYPTNG YTRYADSVKGRFTISADTSKNTAYLQMNSLRAEDTAVYYCSRWGGDGFYAMDYW GQGTLVTVSSASTKGPSVFPLAPEVQLVESGGGLVQPGGSLRLSCAASGFSFSSSYWI CWVRQAPGKCLEWVGCVFTGDGTTYYASWAKGRFTISRDNSKNTVYLQMNSLRA EDTATYFCARPVSVYYYGMDLWGQGTLVTVSSASTKGPSVFPLAPSSKSTSGGTAA LGCLVKDYFPEPVTVSWNSGALTSGVHTFPAVLQSSGLYSLSSVVTVPSSSLGTQTYI CNVNHKPSNTKVDKKVEPKSCGGGGSGGGGSIQMTQSPSSLSASVGDRVTITCQASE NIYSFLAWYQQKPGKAPKLLIYSASKLAAGVPSRFSGSGSGTDFTLTISSLQPEDFAT YYCQQTNRYSNPDIYNVFGTGTKVTVLGGGGGSGGGGSGGGGSGGGGSEVQLVES GGGLVQPGGSLRLSCAASGIDFNSNYYMCWVRQAPGKGLEWIGCIYVGSHVNTYY ANWAKGRFTISRDNSKNTVYLQMNSLRAEDTAVYYCATSGSSVLYFKFWGQGTLV TVSS |

**PRO1557 (MATCH4)**

| 91 | CHAIN_1<sub>PRO1557</sub> | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKLTVLGGGGGSGG GGSGGGGSGGGGSEVQLVESGGGLVQPGGSLRLSCAASGFNIKDTYIHWVRQAPGK CLEWVARIYPTNGYTRYADSVKGRFTISADTSKNTAYLQMNSLRAEDTAVYYCSR WGGDGFYAMDYWGQGTLVTVSSGGGGSGGGGSDVVMTQSPSSLSASVGDRVTITC QASQIISSRSAWYQQKPGQPPKLLIYQASKLASGVPSRFSGSGSGTDFTLTISSLQPED FATYYCQCTYIDSNFGAFGCGTKLTVLGGGSGGSDIQMTQSPSSLSASVGDRVTITC QSSQSVFSNNYLAWFQQKPGQSPKRLIYSASTLASGVPSRFSGSGSGTDFTLTISSLQP EDFATYYCLGSYACSSADCYVFGTGTKVTVLG |
|---|---|---|
| 92 | CHAIN_2<sub>PRO1557</sub> | DIQMTQSPSSLSASVGDRVTITCQASENIYSFLAWYQQKPGKAPKLLIYSASKLAAGV PSRFSGSGSGTDFTLTISSLQPEDFATYYCQQTNRYSNPDIYNVFGTGTKVTVLGGGG GSGGGGSGGGGSEVQLVESGGGLVQPGGSLRLSCAASGIDFNSNYYMCWV RQAPGKGLEWIGCIYVGSHVNTYYANWAKGRFTISRDNSKNTVYLQMNSLRAEDT AVYYCATSGSSVLYFKFWGQGTLVTVSSGGGGSGGGGSEVQLVESGGGLVQPGGS LRLSCAASGFSLSSYDMSWVRQAPGKGLAWIGASYASGPTYYASWAKGRFTISRDN SKNTVYLQMNSLRAEDTATYFCARGGWTGTSHSNIWGQGTLVTVSSGGGGSGGGGSE VQLVESGGGLVQPGGSLRLSCAASGFSFSSSYWICWVRQAPGKCLEWVGCVFTGDG TTYYASWAKGRFTISRDNSKNTVYLQMNSLRAEDTATYFCARPVSVYYYGMDLWG QGTLVTVSS |

**PRO1558 (MATCH4)**

| 93 | CHAIN_1<sub>PRO1558</sub> | DIQMTQSPSSLSASVGDRVTITCQASENIYSFLAWYQQKPGKAPKLLIYSASKLAAGVPS RFSGSGSGTDFTLTISSLQPEDFATYYCQQTNRYSNPDIYNVFGTGTKVTVLGGGGGSG GGGSGGGGSEVQLVESGGGLVQPGGSLRLSCAASGIDFNSNYYMCWVRQAPG KGLEWIGCIYVGSHVNTYYANWAKGRFTISRDNSKNTVYLQMNSLRAEDTAVYYCAT SGSSVLYFKFWGQGTLVTVSSGGGGSGGGGSDVVMTQSPSSLSASVGDRVTITCQASQI |

EP 3 816 185 A1

| | | |
|---|---|---|
| **PRO1558 (MATCH4)** | | |
| | | ISSRSAWYQQKPGQPPKLLIYQASKLASGVPSRFSGSGSGTDFTLTISSLQPEDFATYYCQ CTYIDSNFGAFGCGTKLTVLGGGSGGSDIQMTQSPSSLSASVGDRVTITCQSSQSVFSNN YLAWFQQKPGQSPKRLIYSASTLASGVPSRFSGSGSGTDFTLTISSLQPEDFATYYCLGS YACSSADCYVFGTGTKVTVLG |
| 94 | CHAIN_2<sub>PRO1558</sub> | DIQMTQSPSSLSASVGDRVTITCRASQDVNTAVAWYQQKPGKAPKLLIYSASFLYSGVP SRFSGSRSGTDFTLTISSLQPEDFATYYCQQHYTTPPTFGCGTKLTVLGGGGGSGGGGSG GGGSGGGGSEVQLVESGGGLVQPGGSLRLSCAASGFNIKDTYIHWVRQAPGKCLEWV ARIYPTNGYTRYADSVKGRFTISADTSKNTAYLQMNSLRAEDTAVYYCSRWGGDGFY AMDYWGQGTLVTVSSGGGGSGGGGSEVQLVESGGGLVQPGGSLRLSCAASGFSLSSY DMSWVRQAPGKGLAWIGASYASGPTYYASWAKGRFTISRDNSKNTVYLQMNSLRAED TATYFCARGGWTGTSHSNIWGQGTLVTVSSGGGGSGGGSEVQLVESGGGLVQPGGSLRL SCAASGFSFSSSYWICWVRQAPGKCLEWVGCVFTGDGTTYYASWAKGRFTISRDNSKN TVYLQMNSLRAEDTATYFCARPVSVYYYGMDLWGQGTLVTVSS |
| **Constructs of Example 3: anti-HER2xPDL1<sub>(high KD)</sub>xCD137xHSA** | | |
| **PRO1778 (MATCH4)** | | |
| 95 | CHAIN_1<sub>PRO1778</sub> | DIQMTQSPSSLSASVGDRVTITCRASQDVNTAVAWYQQKPGKAPKLLIYSASFLYSGVPS RFSGSRSGTDFTLTISSLQPEDFATYYCQQHYTTPPTFGCGTKLTVLGGGGGSGGGGSGG GGSGGGGSEVQLVESGGGLVQPGGSLRLSCAASGFNIKDTYIHWVRQAPGKCLEWVARI YPTNGYTRYADSVKGRFTISADTSKNTAYLQMNSLRAEDTAVYYCSRWGGDGFYAMD YWGQGTLVTVSSGGGGSGGGGSDIQMTQSPSSLSASVGDRVTITCQASQSISNRLAWYQ QKPGKAPKLLIYSASTLASGVPSRFSGSGSGTDFTLTISSLQPEDFATYYCQSTYYGNDGN AFGCGTKVTVLGGGSGGSDIQMTQSPSSLSASVGDRVTITCQASQSINDYLAWYQQKPG KAPKLLIYKASTLASGVPSRFSGSGSGTDFTLTISSLQPEDFATYYCQAAYIITDIDNVFGT GTKVTVLG |

**Constructs of Example 3: anti-HER2xPDL1(high KD)xCD137xHSA**

**PRO1778 (MATCH4)**

| 96 | CHAIN_2PRO1778 | DVVMTQSPSSLSASVGDRVTITCQASQIISSRSAWYQQKPGQPPKLLIYQASKLASGVPSR FSGSGSGTDFTLTISSLQPEDFATYYCQCTYIDSNFGAFGGGTKLTVLGGGGGSGGGGSG GGGSGGGGSEVQLVESGGGLVQPGGSLRLSCAASGFSFSSSYWICWVRQAPGKGLEWV GCVFTGDGTTYYASWAKGRFTISRDNSKNTVYLQMNSLRAEDTATYFCARPVSVYYYG MDLWGQGTLVTVSSGGGGSGGGGSQVQLQESGPGLVKPSETLSLTCKVSGFSFSSGYDM CWIRQPPGKGLEWIGCVVAGSVDITYYASWAKGRVTISVDSSKNQFSLKLSSVTAADTA VYYCARKDAYSDAFNLWGQGTLVTVSSGGGSGGGSEVQLVESGGGLVQPGGSLRLSCA ASGFSFSANYYPCWVRQAPGKCLEWIGCIYGGSSDITYDANWTKGRFTISRDNSKNTVYL<br><br>QMNSLRAEDTAVYYCARSAWYSGWGGDLWGQGTLVTVSS |
|---|---|---|

**PRO1780 (MATCH4)**

| 97 | CHAIN_1PRO1780 | DIQMTQSPSSLSASVGDRVTITCRASQDVNTAVAWYQQKPGKAPKLLIYSASFLYSGVPS RFSGSRSGTDFTLTISSLQPEDFATYYCQQHYTTPPTFGCGTKLTVLGGGGGSGGGGSGG GGSGGGGSEVQLVESGGGLVQPGGSLRLSCAASGFNIKDTYIHWVRQAPGKCLEWVARI YPTNGYTRYADSVKGRFTISADTSKNTAYLQMNSLRAEDTAVYYCSRWGGDGFYAMD YWGQGTLVTVSSGGGGSGGGGSDVVMTQSPSSLSASVGDRVTITCQASQIISSRSAWYQ QKPGQPPKLLIYQASKLASGVPSRFSGSGSGTDFTLTISSLQPEDFATYYCQCTYIDSNFGA FGCGTKLTVLGGGSGGSDIQMTQSPSSLSASVGDRVTITCQASQSINDYLAWYQQKPGK APKLLIYKASTLASGVPSRFSGSGSGTDFTLTISSLQPEDFATYYCQAAYIITDIDNVFGTG TKVTVLG |
|---|---|---|
| 98 | CHAIN_2PRO1780 | DIQMTQSPSSLSASVGDRVTITCQASQSISNRLAWYQQKPGKAPKLLIYSASTLASGVPSR FSGSGSGTDFTLTISSLQPEDFATYYCQSTYYGNDGNAFGCGTKVTVLGGGGGSGGGGS GGGGSGGGGSEVQLVESGGGLVQPGGSLRLSCAASGFSFSANYYPCWVRQAPGKCLEWI GCIYGGSSDITYDANWTKGRFTISRDNSKNTVYLQMNSLRAEDTAVYYCARSAWYSGW GGDLWGQGTLVTVSSGGGGSGGGGSQVQLQESGPGLVKPSETLSLTCKVSGFSFSSGYD MCWIRQPPGKGLEWIGCVVAGSVDITYYASWAKGRVTISVDSSKNQFSLKLSSVTAADT AVYYCARKDAYSDAFNLWGQGTLVTVSSGGGSGGGSEVQLVESGGGLVQPGGSLRLSC AASGFSFSSSYWICWVRQAPGKCLEWVGCVFTGDGTTYYASWAKGRFTISRDNSKNTV YLQMNSLRAEDTATYFCARPVSVYYYGMDLWGQGTLVTVSS |

**PRO1992 (MATCH4)**

| 99 | CHAIN_1PRO1992 | DVVMTQSPSSLSASVGDRVTITCQASQIISSRSAWYQQKPGQPPKLLIYQASKLASGVPSR FSGSGSGTDFTLTISSLQPEDFATYYCQCTYIDSNFGAFGTGTKLTVLGGGGGSGGGGSG GGGSGGGGSEVQLVESGGGLVQPGGSLRLSCAASGFSFSSSYWICWVRQAPGKGLEWV GCVFTGDGTTYYASWAKGRFTISRDNSKNTVYLQMNSLRAEDTATYFCARPVSVYYYG MDLWGQGTLVTVSSGGGGSGGGGSDIQMTQSPSSLSASVGDRVTITCKASQDVSIGVAW YQQKPGKAPKLLIYSASYRYTGVPSRFSGSGSGTDFTLTISSLQPEDFATYYCQQYYIYPY TFGCGTKVTVLGGGSGGGSDIQMTQSPSSLSASVGDRVTITCQASQSINDYLAWYQQKPG KAPKLLIYKASTLASGVPSRFSGSGSGTDFTLTISSLQPEDFATYYCQAAYIITDIDNVFGT GTKVTVLG |
| --- | --- | --- |
| 100 | CHAIN_2PRO1992 | DIQMTQSPSSLSASVGDRVTITCQASQSISNRLAWYQQKPGKAPKLLIYSASTLASGVPSR FSGSGSGTDFTLTISSLQPEDFATYYCQSTYYGNDGNAFGCGTKVTVLGGGGGSGGGGS GGGGSGGGGSEVQLVESGGGLVQPGGSLRLSCAASGFSFSANYYPCWVRQAPGKCLEWI GCIYGGSSDITYDANWTKGRFTISRDNSKNTVYLQMNSLRAEDTAVYYCARSAWYSGW GGDLWGQGTLVTVSSGGGGSGGGGSQVQLQESGPGLVKPSETLSLTCKVSGFSFSSGYD MCWIRQPPGKGLEWIGCVVAGSVDITYYASWAKGRVTISVDSSKNQFSLKLSSVTAADT AVYYCARKDAYSDAFNLWGQGTLVTVSSGGGSGGGSEVQLVESGGGLVQPGGSLRLSC AASGFTFTDYTMDWVRQAPGKCLEWVADVNPNSGGSIYNQRFKGRFTLSVDRSKNTLY LQMNSLRAEDTAVYYCARNLGPSFYFDYWGQGTLVTVSS |

**PRO1993 (MATCH4) and PRO1993 variant (MATCH4)**

| 101 | CHAIN_1PRO1993 | DIQMTQSPSSLSASVGDRVTITCKASQDVSIGVAWYQQKPGKAPKLLIYSASYRYTG VPSRFSGSGSGTDFTLTISSLQPEDFATYYCQQYYIYPYTFGCGTKVTVLGGGGGSGG GGSGGGGSGGGGSEVQLVESGGGLVQPGGSLRLSCAASGFTFTDYTMDWVRQAPG KCLEWVADVNPNSGGSIYNQRFKGRFTLSVDRSKNTLYLQMNSLRAEDTAVYYCA RNLGPSFYFDYWGQGTLVTVSSGGGGSGGGGSDVVMTQSPSSLSASVGDRVTITCQ ASQIISSRSAWYQQKPGQPPKLLIYQASKLASGVPSRFSGSGSGTDFTLTISSLQPEDF ATYYCQCTYIDSNFGAFGTGTKLTVLGGGSGGGSDIQMTQSPSSLSASVGDRVTITCQ ASQSISNRLAWYQQKPGKAPKLLIYSASTLASGVPSRFSGSGSGTDFTLTISSLQPEDF ATYYCQSTYYGNDGNAFGCGTKVTVLG |
| --- | --- | --- |

| PRO1993 (MATCH4) and PRO1993 variant (MATCH4) | | |
|---|---|---|
| 102 | CHAIN_2PRO1993 | DIQMTQSPSSLSASVGDRVTITCQASQSINDYLAWYQQKPGKAPKLLIYKASTLASG VPSRFSGSGSGTDFTLTISSLQPEDFATYYCQAAYIITDIDNVFGTGTKVTVLGGGGGS GGGGSGGGGSGGGGSQVQLQESGPGLVKPSETLSLTCKVSGFSFSSGYDMCWIRQPP GKGLEWIGCVVAGSVDITYYASWAKGRVTISVDSSKNQFSLKLSSVTAADTAVYYC ARKDAYSDAFNLWGQGTLVTVSSGGGGSGGGGSEVQLVESGGGLVQPGGSLRLSC AASGFSFSANYYPCWVRQAPGKCLEWIGCIYGGSSDITYDANWTKGRFTISRDNSKN TVYLQMNSLRAEDTAVYYCARSAWYSGWGGDLWGQGTLVTVSSGGGGSGGGGSEVQ LVESGGGLVQPGGSLRLSCAASGFSFSSSYWICWVRQAPGKGLEWVGCVFTGDGTT YYASWAKGRFTISRDNSKNTVYLQMNSLRAEDTATYFCARPVSVYYYGMDLWGQ GTLVTVSS |
| 103 | CHAIN_1PRO1993 variant | DIQMTQSPSSLSASVGDRVTITCKASQDVSIGVAWYQQKPGKAPKLLIYSASYRYTGV PSRFSGSGSGTDFTLTISSLQPEDFATYYCQQYYIYPYTFGCGTKVTVLGGGGGSGGGG SGGGGSGGGGSEVQLVESGGGLVQPGGSLRLSCAASGFTFTDYTMDWVRQAPGKCL EWVADVNPNSGGSIYNQRFKGRFTLSVDRSKNTLYLQMNSLRAEDTAVYYCARNLG PSFYFDYWGQGTLVTVSSGGGGSGGGGSDIQMTQSPSSLSASVGDRVTITCQSSESVY SNNQLSWYQQKPGQPPKLLIYDASDLASGVPSRFSGSGSGTDFTLTISSLQPEDFATYY CAGGFSSSSDTAFGGGTKLTVLGGGSGGSDIQMTQSPSSLSASVGDRVTITCQASQSIS NRLAWYQQKPGKAPKLLIYSASTLASGVPSRFSGSGSGTDFTLTISSLQPEDFATYYCQ STYYGNDGNAFGCGTKVTVLG |
| 104 | CHAIN-2PRO1993 variant | DIQMTQSPSSLSASVGDRVTITCQASQSINDYLAWYQQKPGKAPKLLIYKASTLASGVP SRFSGSGSGTDFTLTISSLQPEDFATYYCQAAYIITDIDNVFGTGTKVTVLGGGGGSGG GGSGGGGSGGGGSQVQLQESGPGLVKPSETLSLTCKVSGFSFSSGYDMCWIRQPPGKG LEWIGCVVAGSVDITYYASWAKGRVTISVDSSKNQFSLKLSSVTAADTAVYYCARKD AYSDAFNLWGQGTLVTVSSGGGGSGGGGSEVQLVESGGGLVQPGGSLRLSCAASGFS FSANYYPCWVRQAPGKCLEWIGCIYGGSSDITYDANWTKGRFTISRDNSKNTVYLQM NSLRAEDTAVYYCARSAWYSGWGGDLWGQGTLVTVSSGGGGSGGGGSEVQLVESGGG LVQPGGSLRLSCAASGFSLSSNAMGWVRQAPGKGLEYIGIISVGGFTYYASWAKGRFT ISRDNSKNTVYLQMNSLRAEDTATYFCARDRHGGDSSGAFYLWGQGTLVTVSS |

(continued)

| Constructs of Example 4: anti-HER2xPDL1 ($_{high\ KD}$)xHSA | | |
|---|---|---|
| 105 | PRO1678 (scDb-scFv, scMATCH3) | DIQMTQSPSSLSASVGDRVTITCQASQSINDYLAWYQQKPGKAPKLLIYKASTLASG VPSRFSGSGSGTDFTLTISSLQPEDFATYYCQAAYIITDIDNVFGTGTKVTVLGGGGGS EVQLVESGGGLVQPGGSLRLSCAASGFNIKDTYIHWVRQAPGKCLEWVARIYPTNG YTRYADSVKGRFTISADTSKNTAYLQMNSLRAEDTAVYYCSRWGGDGFYAMDYW GQGTLVTVSSGGGGSGGGGSGGGGSGGGGSIQMTQSPSSLSASVGDRVTITCRASQD VNTAVAWYQQKPGKAPKLLIYSASFLYSGVPSRFSGSRSGTDFTLTISSLQPEDFATY YCQQHYTTPPTFGCGTKLTVLGGGGGSQVQLQESGPGLVKPSETLSLTCKVSGFSFS SGYDMCWIRQPPGKGLEWIGCVVAGSVDITYYASWAKGRVTISVDSSKNQFSLKLS SVTAADTAVYYCARKDAYSDAFNLWGQGTLVTVSSGGGGSGGGGSVVMTQSPSSL SASVGDRVTITCQASQIISSRSAWYQQKPGQPPKLLIYQASKLASGVPSRFSGSGSGT DFTLTISSLQPEDFATYYCQCTYIDSNFGAFGGGTKLTVLGGGGGSGGGGSGGGGSG GGGSEVQLVESGGGLVQPGGSLRLSCAASGFSFSSSYWICWVRQAPGKGLEWVGCV FTGDGTTYYASWAKGRFTISRDNSKNTVYLQMNSLRAEDTATYFCARPVSVYYYG MDLWGQGTLVTVSS |
| 106 | PRO1679 (scDb-scFv, scMATCH3) | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKLTVLGGGGGSQV QLQESGPGLVKPSETLSLTCKVSGFSFSSGYDMCWIRQPPGKGLEWIGCVVAGSVDI TYYASWAKGRVTISVDSSKNQFSLKLSSVTAADTAVYYCARKDAYSDAFNLWGQG TLVTVSSGGGGSGGGGSGGGGSGGGGSIQMTQSPSSLSASVGDRVTITCQASQSIND YLAWYQQKPGKAPKLLIYKASTLASGVPSRFSGSGSGTDFTLTISSLQPEDFATYYCQ AAYIITDIDNVFGTGTKVTVLGGGGGSEVQLVESGGGLVQPGGSLRLSCAASGFNIK DTYIHWVRQAPGKCLEWVARIYPTNGYTRYADSVKGRFTISADTSKNTAYLQMNSL RAEDTAVYYCSRWGGDGFYAMDYWGQGTLVTVSSGGGGSGGGGSVVMTQSPSSL SASVGDRVTITCQASQIISSRSAWYQQKPGQPPKLLIYQASKLASGVPSRFSGSGSGT DFTLTISSLQPEDFATYYCQCTYIDSNFGAFGGGTKLTVLGGGGGSGGGGSGGGGSG GGGSEVQLVESGGGLVQPGGSLRLSCAASGFSFSSSYWICWVRQAPGKGLEWVGCV FTGDGTTYYASWAKGRFTISRDNSKNTVYLQMNSLRAEDTATYFCARPVSVYYYG MDLWGQGTLVTVSS |

(continued)

| Constructs of Example 4: anti-HER2xPDL1 ($_{high\ KD}$)xHSA | | |
|---|---|---|
| 107 | PRO1680 (scDb-scFv, scMATCH3) | DIQMTQSPSSLSASVGDRVTITCQASQSINDYLAWYQQKPGKAPKLLIYKASTLASG VPSRFSGSGSGTDFTLTISSLQPEDFATYYCQAGYIITDIDNVFGTGTKVTVLGGGGGS EVQLVESGGGLVQPGGSLRLSCAASGFNIKDTYIHWVRQAPGKCLEWVARIYPTNG YTRYADSVKGRFTISADTSKNTAYLQMNSLRAEDTAVYYCSRWGGDGFYAMDYW GQGTLVTVSSGGGGSGGGGSGGGGSGGGGSIQMTQSPSSLSASVGDRVTITCRASQD VNTAVAWYQQKPGKAPKLLIYSASFLYSGVPSRFSGSRSGTDFTLTISSLQPEDFATY YCQQHYTTPPTFGCGTKLTVLGGGGGSQVQLQESGPGLVKPSETLSLTCKVSGFSFS SGYDMCWIRQPPGKGLEWIGCVVAGSVDITYYASWAKGRVTISVDSSKNQFSLKLS SVTAADTAVYYCARKDAASDAFNLWGQGTLVTVSSGGGGSGGGGSVVMTQSPSSL SASVGDRVTITCQASQIISSRSAWYQQKPGQPPKLLIYQASKLASGVPSRFSGSGSGT DFTLTISSLQPEDFATYYCQCTYIDSNFGAFGGGTKLTVLGGGGGSGGGGSGGGGSG GGGSEVQLVESGGGLVQPGGSLRLSCAASGFSFSSSYWICWVRQAPGKGLEWVGCV FTGDGTTYYASWAKGRFTISRDNSKNTVYLQMNSLRAEDTATYFCARPVSVYYYG MDLWGQGTLVTVSS |
| 108 | PRO1681 (scDb-scFv, scMATCH3) | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKLTVLGGGGGSQV QLQESGPGLVKPSETLSLTCKVSGFSFSSGYDMCWIRQPPGKGLEWIGCVVAGSVDI TYYASWAKGRVTISVDSSKNQFSLKLSSVTAADTAVYYCARKDAASDAFNLWGQG TLVTVSSGGGGSGGGGSGGGGSGGGGSIQMTQSPSSLSASVGDRVTITCQASQSIND YLAWYQQKPGKAPKLLIYKASTLASGVPSRFSGSGSGTDFTLTISSLQPEDFATYYCQ AGYIITDIDNVFGTGTKVTVLGGGGGSEVQLVESGGGLVQPGGSLRLSCAASGFNIK DTYIHWVRQAPGKCLEWVARIYPTNGYTRYADSVKGRFTISADTSKNTAYLQMNSL RAEDTAVYYCSRWGGDGFYAMDYWGQGTLVTVSSGGGGSGGGGSVVMTQSPSSL SASVGDRVTITCQASQIISSRSAWYQQKPGQPPKLLIYQASKLASGVPSRFSGSGSGT DFTLTISSLQPEDFATYYCQCTYIDSNFGAFGGGTKLTVLGGGGGSGGGGSGGGGSG GGGSEVQLVESGGGLVQPGGSLRLSCAASGFSFSSSYWICWVRQAPGKGLEWVGCV FTGDGTTYYASWAKGRFTISRDNSKNTVYLQMNSLRAEDTATYFCARPVSVYYYG MDLWGQGTLVTVSS |

EP 3 816 185 A1

| Constructs of Example 4: anti-HER2xPDL1 (high KD)xHSA | | |
|---|---|---|
| 109 | PRO1814 (scDb-scFv, scMATCH3) | DIQMTQSPSSLSASVGDRVTITCQASQSINDYLAWYQQKPGKAPKLLIYKASTLASG VPSRFSGSGSGTDFTLTISSLQPEDFATYYCQAAYIITDIDNVFGTGTKVTVLGGGGGS EVQLVESGGGLVQPGGSLRLSCAASGFTFTDYTMDWVRQAPGKGLEWVADVNPNS GGSIYNQRFKGRFTLSVDRSKNTLYLQMNSLRAEDTAVYYCARNLGPSFYFDYWGQ GTLVTVSSGGGGSGGGGSGGGGSGGGGSDIQMTQSPSSLSASVGDRVTITCKASQD VSIGVAWYQQKPGKAPKLLIYSASYRYTGVPSRFSGSGSGTDFTLTISSLQPEDFATY YCQQYYIYPYTFGTGTKVTVLGGGGGSQVQLQESGPGLVKPSETLSLTCKVSGFSFS SGYDMCWIRQPPGKGLEWIGCVVAGSVDITYYASWAKGRVTISVDSSKNQFSLKLS SVTAADTAVYYCARKDAYSDAFNLWGQGTLVTVSSGGGGSGGGGSIQMTQSPSSLS ASVGDRVTITCQSSESVYSNNQLSWYQQKPGQPPKLLIYDASDLASGVPSRFSGSGS GTDFTLTISSLQPEDFATYYCAGGFSSSSDTAFGGGTKLTVLGGGGGSGGGGSGGGG SGGGGSEVQLVESGGGLVQPGGSLRLSCAASGFSLSSNAMGWVRQAPGKGLEYIGII SVGGFTYYASWAKGRFTISRDNSKNTVYLQMNSLRAEDTATYFCARDRHGGDSSG AFYLWGQGTLVTVSS |
| 110 | PRO1852 (scDb-scFv, scMATCH3) | DIQMTQSPSSLSASVGDRVTITCQASQSINDYLAWYQQKPGKAPKLLIYKASTLASG VPSRFSGSGSGTDFTLTISSLQPEDFATYYCQAAYIITDIDNVFGTGTKVTVLGGGGGS EVQLVESGGGLVQPGGSLRLSCAASGFTFTDYTMDWVRQAPGKCLEWVADVNPNS GGSIYNQRFKGRFTLSVDRSKNTLYLQMNSLRAEDTAVYYCARNLGPSFYFDYWGQ GTLVTVSSGGGGSGGGGSGGGGSGGGGSDIQMTQSPSSLSASVGDRVTITCKASQD VSIGVAWYQQKPGKAPKLLIYSASYRYTGVPSRFSGSGSGTDFTLTISSLQPEDFATY YCQQYYIYPYTFGCGTKVTVLGGGGGSQVQLQESGPGLVKPSETLSLTCKVSGFSFS SGYDMCWIRQPPGKGLEWIGCVVAGSVDITYYASWAKGRVTISVDSSKNQFSLKLS SVTAADTAVYYCARKDAYSDAFNLWGQGTLVTVSSGGGGSGGGGSIQMTQSPSSLS ASVGDRVTITCQSSESVYSNNQLSWYQQKPGQPPKLLIYDASDLASGVPSRFSGSGS GTDFTLTISSLQPEDFATYYCAGGFSSSSDTAFGGGTKLTVLGGGGGSGGGGSGGGG SGGGGSEVQLVESGGGLVQPGGSLRLSCAASGFSLSSNAMGWVRQAPGKGLEYIGII SVGGFTYYASWAKGRFTISRDNSKNTVYLQMNSLRAEDTATYFCARDRHGGDSSG AFYLWGQGTLVTVSS |

| Constructs of Example 4: anti-HER2xPDL1 (high KD)xHSA | | |
|---|---|---|
| 111 | PRO1853 (scDb-scFv, scMATCH3) | DIQMTQSPSSLSASVGDRVTITCQASQSINDYLAWYQQKPGKAPKLLIYKASTLASG VPSRFSGSGSGTDFTLTISSLQPEDFATYYCQAGYIITDIDNVFGTGTKVTVLGGGGGS EVQLVESGGGLVQPGGSLRLSCAASGFTFTDYTMDWVRQAPGKGLEWVADVNPNS GGSIYNQRFKGRFTLSVDRSKNTLYLQMNSLRAEDTAVYYCARNLGPSFYFDYWGQ GTLVTVSSGGGGSGGGGSGGGGSGGGGSDIQMTQSPSSLSASVGDRVTITCKASQD VSIGVAWYQQKPGKAPKLLIYSASYRYTGVPSRFSGSGSGTDFTLTISSLQPEDFATY YCQQYYIYPYTFGTGTKVTVLGGGGGSQVQLQESGPGLVKPSETLSLTCKVSGFSFS SGYDMCWIRQPPGKGLEWIGCVVAGSVDITYYASWAKGRVTISVDSSKNQFSLKLS SVTAADTAVYYCARKDAASDAFNLWGQGTLVTVSSGGGGSGGGGSIQMTQSPSSLS ASVGDRVTITCQSSESVYSNNQLSWYQQKPGQPPKLLIYDASDLASGVPSRFSGSGS GTDFTLTISSLQPEDFATYYCAGGFSSSSDTAFGGGTKLTVLGGGGGSGGGGSGGGG SGGGGSEVQLVESGGGLVQPGGSLRLSCAASGFSLSSNAMGWVRQAPGKGLEYIGII SVGGFTYYASWAKGRFTISRDNSKNTVYLQMNSLRAEDTATYFCARDRHGGDSSG AFYLWGQGTLVTVSS |
| 112 | PRO1854 (scDb-scFv, scMATCH3) | DIQMTQSPSSLSASVGDRVTITCQASQSINDYLAWYQQKPGKAPKLLIYKASTLASG VPSRFSGSGSGTDFTLTISSLQPEDFATYYCQAGYIITDIDNVFGTGTKVTVLGGGGGS EVQLVESGGGLVQPGGSLRLSCAASGFTFTDYTMDWVRQAPGKCLEWVADVNPNS GGSIYNQRFKGRFTLSVDRSKNTLYLQMNSLRAEDTAVYYCARNLGPSFYFDYWGQ GTLVTVSSGGGGSGGGGSGGGGSGGGGSDIQMTQSPSSLSASVGDRVTITCKASQD VSIGVAWYQQKPGKAPKLLIYSASYRYTGVPSRFSGSGSGTDFTLTISSLQPEDFATY YCQQYYIYPYTFGCGTKVTVLGGGGGSQVQLQESGPGLVKPSETLSLTCKVSGFSFS SGYDMCWIRQPPGKGLEWIGCVVAGSVDITYYASWAKGRVTISVDSSKNQFSLKLS SVTAADTAVYYCARKDAASDAFNLWGQGTLVTVSSGGGGSGGGGSIQMTQSPSSLS ASVGDRVTITCQSSESVYSNNQLSWYQQKPGQPPKLLIYDASDLASGVPSRFSGSGS GTDFTLTISSLQPEDFATYYCAGGFSSSSDTAFGGGTKLTVLGGGGGSGGGGSGGGG SGGGGSEVQLVESGGGLVQPGGSLRLSCAASGFSLSSNAMGWVRQAPGKGLEYIGII SVGGFTYYASWAKGRFTISRDNSKNTVYLQMNSLRAEDTATYFCARDRHGGDSSG AFYLWGQGTLVTVSS |

**PRO1543 variant (MATCH4)**

| 113 | CHAIN_1<sub>PRO1543_variant</sub> | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKLTVLGGGGGSGG GGSGGGGSGGGGSEVQLVESGGGLVQPGGSLRLSCAASGFNIKDTYIHWVRQAPGK CLEWVARIYPTNGYTRYADSVKGRFTISADTSKNTAYLQMNSLRAEDTAVYYCSR WGGDGFYAMDYWGQGTLVTVSSGGGGSGGGGSDIQMTQSPSSLSASVGDRVTITC QSSESVYSNNQLSWYQQKPGQPPKLLIYDASDLASGVPSRFSGSGSGTDFTLTISSLQ PEDFATYYCAGGFSSSSDTAFGCGTKLTVLGGGSGGSDIQMTQSPSSLSASVGDRVTI TCQSSQSVFSNNYLAWFQQKPGQSPKRLIYSASTLASGVPSRFSGSGSGTDFTLTISSL QPEDFATYYCLGSYACSSADCYVFGTGTKVTVLG |
|-----|-----------------------------------|---------------------------------------------------------------|
| 114 | CHAIN_2<sub>PRO1543_variant</sub> | DIQMTQSPSSLSASVGDRVTITCQASQSINDYLAWYQQKPGKAPKLLIYKASTLASG VPSRFSGSGSGTDFTLTISSLQPEDFATYYCQAAYIITDIDNVFGTGTKVTVLGGGGGS GGGGSGGGGSGGGGSQVQLQESGPGLVKPSETLSLTCKVSGFSFSSGYDMCWIRQPP GKGLEWIGCVVAGSVDITYYASWAKGRVTISVDSSKNQFSLKLSSVTAADTAVYYC ARKDAYSDAFNLWGQGTLVTVSSGGGGSGGGGSEVQLVESGGGLVQPGGSLRLSC AASGFSLSSYDMSWVRQAPGKGLAWIGASYASGPTYYASWAKGRFTISRDNSKNTV YLQMNSLRAEDTATYFCARGGWTGTSHSNIWGQGTLVTVSSGGGSGGGGSEVQLVE SGGGLVQPGGSLRLSCAASGFSLSSNAMGWVRQAPGKCLEYIGIISVGGFTYYASWA KGRFTISRDNSKNTVYLQMNSLRAEDTATYFCARDRHGGDSSGAFYLWGQGTLVT VSS |

*Table 7. Other sequences related to the present invention.*

| SEQ ID NO: | Description | Sequence |
|---|---|---|
| *IL23R domain 14-11-D07 sc03 (dummy)* | | |
| 115 | VH$_{14\text{-}11\text{-}D07\ sc03}$ | EVQLVESGGGLVQPGGSLRLSCAASGIDFNSNYYMCWVRQAPGKGLEWIGCIYVGS HVNTYYANWAKGRFTISRDNSKNTVYLQMNSLRAEDTAVYYCATSGSSVLYFKFW GQGTLVTVSS |
| 116 | VL$_{14\text{-}11\text{-}D07\ sc03}$ | DIQMTQSPSSLSASVGDRVTITCQASENIYSFLAWYQQKPGKAPKLLIYSASKLAAGV PSRFSGSGSGTDFTLTISSLQPEDFATYYCQQTNRYSNPDIYNVFGTGTKVTVLG |
| *Linkers* | | |
| 117 | Linker | GGGGSGGGGSGGGGSGGGGS |
| 118 | Linker sequence unit | GGGGS |
| 119 | Generic linker sequence | (GmS)n, with m being selected from 2, 3 and 4 and with n being selected from 2, 3, 4, 5 and 6 |
| *VH and VL sequences* | | |
| 120 | VH3 | EVQLVESGGGLVQPGGSLRLSCAAS*GFSFSANYYPC*WVRQAPGKGLEWIG*CIYGGSS DITYDANWTK*GRFTISRDNSKNTVYLQMNSLRAEDTAVYYCA*RSAWYSGWGGDL*W GQGTLVTVSS |
| 121 | VH4 | QVQLQESGPGLVKPSETLSLTCKVS*GFSFSNSYWIC*WIRQPPGKGLEWIG*CTFVGSSD STYYANWAKG*RVTISVDSSKNQFSLKLSSVTAADTAVYYCA*RHPSDAVYGYANNL*W GQGTLVTVSS |
| 122 | Vkappa1 | DIQMTQSPSSLSASVGDRVTITC*QASQSINNVLA*WYQQKPGKAPKLLIY*RASTLAS*GV PSRFSGSGSGTDFTLTISSLQPEDFATYYC*QSSYGNYGD*FGTGTKVTVLG |
| *Vlambda FR4 sequences* TFGCGTKLTVLG | | |
| 123 | Vlambda germline-based FR4_Sk17 | FGTGTKVTVLG |
| 124 | Vlambda germline-based FR4_Sk12 | FGGGTKLTVLG |
| 125 | Vlambda germline-based FR4_1 | FGGGTQLIILG |
| 126 | Vlambda germline-based FR4_2 | FGEGTELTVLG |
| 127 | Vlambda germline-based FR4_3 | FGSGTKVTVLG |

| Vlambda FR4 sequences | TFGCGTKLTVLG | |
|---|---|---|
| 128 | Vlambda germline-based FR4_4 | FGGGTQLTVLG |
| 129 | Vlambda germline-based FR4_5 | FGGGTQLTALG |
| Antigens | | |
| 130 | CD137 UniProt ID NO: Q07011 | MGNSCYNIVATLLLVLNFERTRSLQDPCSNCPAGTFCDNNRNQICSPCPPNSFSSAGG QRTCDICRQCKGVFRTRKECSSTSNAECDCTPGFHCLGAGCSMCEQDCKQGQELTK KGCKDCCFGTFNDQKRGICRPWTNCSLDGKSVLVNGTKERDVVCGPSPADLSPGAS SVTPPAPAREPGHSPQIISFFLALTSTALLFLLFFLTLRFSVVKRGRKKLLYIFKQPFMR PVQTTQEEDGCSCRFPEEEEGGCEL |

Throughout the text of this application, should there be a discrepancy between the text of the specification (e.g., Tables 1 to 6) and the sequence listing, the text of the specification shall prevail.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the invention are specifically embraced by the present invention and are disclosed herein just as if each and every combination was individually and explicitly disclosed. In addition, all sub-combinations of the various embodiments and elements thereof are also specifically embraced by the present invention and are disclosed herein just as if each and every such sub-combination was individually and explicitly disclosed herein.

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

To the extent possible under the respective patent law, all patents, applications, publications, test methods, literature, and other materials cited herein are hereby incorporated by reference.

The following Examples illustrates the invention described above, but is not, however, intended to limit the scope of the invention in any way. Other test models known as such to the person skilled in the pertinent art can also determine the beneficial effects of the claimed invention.

**Examples**

**Example 1: Generation and testing of low affinity anti-PD-L1 molecules:**

**Aim of the project**

The goal of the project is to identify a low affinity anti-PD-L1 antibody fragment that neutralizes the interaction between PD-L1 and PD-1. Ultimately, this domain will be combined in a multispecific molecule with a high affinity domain against a selected tumor associated antigen (TAA) coexpressed with PD-L1 on tumor cells, allowing the targeting and neutralization of PD-L1 specifically on those cancer cells. Two groups of long acting molecules, corresponding to two projects, were designed. Each group of molecules is mainly differing in the number of specificities (3 or 4) as well as in their format. Both groups of molecules contain a Her2 domain as TAA, a low affinity PD-L1 domain and a human serum albumin binding domain for half-life extension, but one group contains in addition an anti-CD3ε binding domain in order to trigger T cell activation. This example describes the production and characterization of the low affinity domains as well as of the multispecific molecules that were designed in these projects.

**Design and production of scFv**

In order to generate a low affinity PD-L1 antibody that neutralizes the interaction between PD-L1 and PD-1, single alanine substitutions were introduced in the CDR regions of a high affinity neutralizing anti-PD-L1 domain previously identified, clone 33-03-G02. As a first step each amino acid of the CDR3 region (highest amino acid variability) of the high affinity domain were mutated to alanine resulting in 21 mutants. In comparison to the original domain, the affinity to PD-L1 was reduced by more than 100-fold for three single mutants. Therefore, single mutations were combined go generate two double mutants. In parallel, nine additional single mutants of the most variable residues of the CDR1 and CDR2 regions were designed as well as two mutants containing combination of other single mutations of the CD3 region which only slightly reduced the affinity of the parental domain but are presumably exposed residues. Furthermore, three mutants containing up to three alanine substitutions of predicted exposed residues were expressed in addition. Data obtained for the five most interesting molecules with a 100-fold to 6'500-fold reduced affinity are presented below.

Methods:

*scFv production*

Heterologous expression of the proteins was performed in *E.coli* as insoluble inclusion bodies by induced overnight expression in small scale (as indicated in Table 8 below). Inclusion bodies were isolated from the homogenized cell pellet by a centrifugation protocol that included several washing steps to remove cell debris and other host cell impurities. The purified inclusion bodies were solubilized in a denaturing buffer and the scFvs were refolded by a scalable refolding protocol that generated milligram amounts of natively folded, monomeric scFv. At this point a standardized protocol was employed to purify the scFvs. The product after refolding was captured by an affinity chromatography to yield the purified scFvs. Table 8 summarizes manufacture of scFv molecules. Expression of mammalian constructs was performed in CHO-S cells using CHOgro transient transfection kit (Mirus) (see in Table 8). Cultures were harvested after 5-7 days (cell viability <70 %) of expression at 37°C by centrifugation and proteins were purified from clarified culture supernatants by Protein L affinity chromatography followed, if needed, by a polishing step by size-exclusion chromatography. For the quality control of the manufactured material standard analytical methods, such as SE-HPLC, UV280 and SDS-PAGE were used.

*Freeze-thaw stability study*

Compatibility of the top performing scFv molecules was assessed with respect to freeze-thawing (F/T) cycles (colloidal stability). For the F/T stability assessment the same analytical methods (SE-HPLC, UV absorbance at 280 nm) and parameters as for the storage stability study were applied to monitor the quality of the molecules over multiple F/T cycles. As no dedicated freeze-thaw study was performed, freeze-thaw data was extracted from -80°C samples of storage stability study which was acquired over 28 days (up to 7d storage in between F/T cycles).

*Differential Scanning Fluorimetry (DSF)*

The midpoint of transition for the thermal unfolding of the scFv constructs was determined by Differential Scanning Fluorimetry using the fluorescence dye SYPRO® Orange. Samples were prepared at a final protein concentration of 50 μg/mL in final buffer (50 mM NaCiP, 150 mM NaCl, pH6.4) containing a final concentration of 5x SYPRO® Orange in a total volume of 100 μl. Twenty-five microliters of prepared samples were added in triplicate to white-walled AB gene PCR plates. The assay was performed in a qPCR machine used as a thermal cycler, and the fluorescence emission was detected using the software's custom dye calibration routine. The PCR plate containing the test samples was subjected to a temperature ramp from 25°C to 96°C in increments of 1°C with 30 s pauses after each temperature increment. The total assay time was about two hours. The Tm was calculated by the software GraphPad Prism using a mathematical second derivative method to calculate the inflection point of the curve. The reported Tm is an average of three measurements.

**Table 8: Manufacture of scFv domains based on clone with Clone ID 33-03-G02.**

| PRO ID | Mutations (AHO numbering) | Expression, mg/L | Purity by SE-HPLC, StM, % mc | Purity by SE-HPLC post concentration to 10 mg/mL, % mc | Purity by SDS-PAGE | Thermodynamic Stability by DSF, Tm | Thermodynamic Stability by DSF, Tonset | Freeze / Thaw Stability, 4 cycles |
|---|---|---|---|---|---|---|---|---|
| PRO1075 | VL-Q108A | 4.2 (E. coli) | 97 | 93.2 | > 95 % | 76.9 | 71.0 | <1 % |
| PRO1076 | VL-G109A | 13.2(E. coli) | 97 | 95.8 | > 95 % | 76.3 | 71.0 | <1 % |
| PRO1089 | VH-Y112A | 8.4(E. coli) | 99 | 99.1 | > 95 % | 79.6 | 74.0 | <1 % |
| PRO1434 | VL-Q108A/G109A | 8.2 (CHO) | 100 | 99.6 | > 95 % | 73.0 | 66.3 | <1 % |
| PRO1494 | VL-Q108A/VH-Y112A | 17 (CHO) | 99 | 99.3 | > 95 % | 75.2 | 68.0 | <1 % |

**Affinities to PD-L1 by SPR**

Method:

Affinity to PD-L1 was determined by surface plasmon resonance (SPR) measurements using a Biacore T200 device (GE Healthcare). All measurements were performed at 25°C. In this experiment, Fc-tagged human PD-L1 extracellular domain (ECD, SinoBiological, cat. 10084-H02H) was captured using the Human Antibody Capture kit from GE Healthcare (cat. BR-1008-39). After each analyte injection cycle, the anti-human Fc-specific IgG was regenerated and new antigen was captured. For affinity determination, the scFvs were injected as analyte using a dose response multicycle kinetic assay with concentrations of the analyte ranging from 6.86 to 5000 nM (three-fold dilutions steps) diluted in running buffer (10 mM HEPES, 150 mM NaCl, and 0.05 % Tween 20, pH 7.4). Association and dissociation time were set to 300 s and 720 s, respectively. The apparent dissociation ($k_d$) and association ($k_a$) rate constants and the apparent dissociation equilibrium constant ($K_D$) were calculated with the Biacore analysis software (BIAevaluation, GE Healthcare) using one-to-one Langmuir binding model and quality of the fits was monitored based on Chi2 and U-value, which is a measure for the quality of the curve fitting. In addition to the kinetic measurement, the value of the $K_D$ was obtained by fitting a plot of response at equilibrium against the concentration (steady-state affinity measurement).

Results:

As shown in Table 9, binding to human PD-L1 was confirmed for the humanized scFvs tested. Affinity to PD-L1 was reduced by 6'500 fold for PRO1434 compared to the parental scFv PRO830.

**Table 9: Summary of affinities of scFv based on clone with Clone ID 33-03-G02 to hPD-L1.**

| PRO ID | Mutations (AHO numbering) | Affinity to human PD-L1 | | | |
|---|---|---|---|---|---|
| | | kinetic analysis | | | steady state analysis |
| | | $k_a$ (M$^{-1}$s$^{-1}$) | $k_d$ (s$^{-1}$) | KD (M) | KD (M) |
| PRO830 | NA | 1.78E+06 | 1.39E-04 | 7.81E-11 | ND |
| PRO1075 | VL-Q108A | 1.67E+06 | 2.05E-02 | 1.22E-08 | ND |
| PRO1076 | VL-G109A | 1.80E+06 | 1.35E-02 | 7.52E-09 | ND |
| PRO1089 | VH-Y112A | 1.99E+06 | 3.28E-02 | 1.65E-08 | ND |
| PRO1434 | VL-Q108A/G109A | 1.09E+06 | 0.5346 | 4.92E-07 | 4.87E-07 |
| PRO1494 | VL-Q108A/VH-Y112A | | | ND | |

NA: not applicable
ND: not determined

**Binding to PD-L1 expressing cells by FC**

Method:

CHO-K1 (control cells that do not express PD-L1) and CHO-PD-L1 (Amsbio) with a high PD-L1 expression level were harvested and cell number was determined. Cell suspensions were centrifuged for 5 min at 400xg and 100 µl of cell suspensions (10'000 cells) diluted in PBS-EB (1x DPBS, 2 % BCS H.I., 2 mM EDTA) were added to designated wells in a non-binding plate. After three washing steps with PBS-EB, cells were centrifuged and washing buffer was aspirated. 100 µl of the serial dilutions of samples to be tested as well as the positive control were then directly added to the plate. Positive control samples (PRO830, 33-03-G02) were diluted in PBS-EB with concentrations ranging from 3500 to 0.22 ng/ml and samples dilutions ranged from 1000 to 0.064 µg/ml. After incubation at 4°C for 1 h, plates were washed three times using 100 µl of PBS-EB. Cell pellets were re-suspended with 100 µl Protein L-APC at a concentration of 2 µg/ml and incubated for 1 h at 4°C. Next, cells were washed again three times using 100 µl of PBS-EB. The cell pellets were re-suspended with 50 µl PBS-EB and analyzed with NovoCyte 2060 flow cytometer device. Fluorescence intensity of APC for 5'000 events was recorded for each sample and the geometric mean of fluorescence intensity MFI was calculated. The data were corrected for unspecific antibody binding (blank and CHO-K1 cell binding).

Results:

Potency to bind cells expressing PD-L1 was assessed using flow cytometry as described above. Serial dilutions of the respective molecules to be tested as well as the reference PRO830, were added to the plates. Individual $IC_{50}$ values on each plate were calibrated against the $IC_{50}$ of the reference molecule PRO830 (high affinity PD-L1 domain) that was taken along on each plate (relative $EC_{50}$: $EC_{50}$, PRO830/$IC_{50}$, test scFv). Potencies are summarized in Table 10 which shows that PRO1434 and PRO1494 have the weakest binding. Dose-response curves obtained for PRO1434 and PRO1494 are presented in Figure 1.

**Table 10: Potencies of scFvs based on clone with Clone ID 33-03-G02 to bind cells expressing hPD-L1.**

| | | Binding to cells expressing PD-L1 | |
| --- | --- | --- | --- |
| | | CHO-K1 with high PD-L1 expression | |
| PRO ID | Mutations | $EC_{50\ (ng/ml)}$ | rel. $EC_{50}$* |
| PRO830 | NA | 5.5 | 1 |
| PRO1075 | VL-Q108A | 333314 | 1.42E-05 |
| PRO1076 | VL-G109A | | ND |
| PRO1089 | VH-Y112A | 51168 | 9.30E-05 |
| PRO1434 | VL-Q108A/G109A | NB | |
| PRO1494 | VL-Q108A/VH-Y112A | very low binding level | |

NA: not applicable ND: not determined NB: no binding at the highest tested concentration

*: $EC_{50,\ PRO830}$ (ng/ml)/$EC_{50,\ test\ molecule}$ (ng/ml)

## PD-L1/PD-1 neutralization by NFAT reporter gene assay

Method:

The ability of the scFvs to neutralize the PD-L1/PD-1 interaction when both interacting molecules were expressed on the cell surface was tested using CHO/PD-L1/TCR (T cell receptor) activator and Jurkat/PD-1 cells. In this assay, CHO cells stably expressing PD-L1 and a TCR activator are incubated with Jurkat T cells stably expressing PD-1 and as a reporter gene to monitor T cell activation, firefly luciferase under the control of the NFAT response elements. Upon binding of the TCR activator on CHO cells to the Jurkat T cells, TCR signaling triggers NFAT-induced expression of firefly luciferase. The interaction between PD-L1 and PD-1 however negatively regulates such TCR signaling and thus diminishes firefly luciferase expression. Therefore, blockade of the PD-L1/PD-1 interaction in this system restores luciferase activity.

35'000 CHO/PDL1/TCR activator cells in 100 $\mu$l of cell culture medium (DMEM/F12, 10 % FCS) were added to the inner wells of a white cell culture plate and incubated for 16-20 h at 37°C and 5 % $CO_2$. Next day, cell culture medium was removed from each well and 50 $\mu$l of 2-fold concentrated serial dilutions of the respective molecules to be tested (final concentrations from 162 to 0.025 $\mu$g/ml) and the reference molecule Avelumab (final concentrations from 3'000 to 0.46 ng/ml) were added. Then, 50 $\mu$l of effector Jurkat cells diluted at 400'000 cell/ml in assay buffer (RPMI1640 with 10 % FCS) were added to each well and plates were incubated for 6h at 37°C and 5 % $CO_2$. Finally, 50 $\mu$L luciferase substrate (BPS Bioscience) prepared according to manufacturer's protocol, was added per well and plates were incubated for 30 min in the dark, luminescence was measured using Flexstation III multi-mode microplate reader.

Results:

Potency to neutralize PD-L1 binding to PD-1 was assessed in the cell based reporter gene assay as described above. Serial dilutions of the respective molecules to be tested as well as the reference Avelumab, were added to the plates. Individual IC50 values on each plate were calibrated against the $IC_{50}$ of the reference molecule Avelumab (high affinity PD-L1 domain) that was taken along on each plate (relative $IC_{50}$: $IC_{50}$, Avelumab/$IC_{50}$, test scFv). Potencies are summarized in Table 11 which shows that PRO1434 and PRO1494 have the lowest potencies. Titration curves obtained for PRO1434 and PRO1494 are presented Figure 2.

**Table11: Potencies of scFvs based on clone with Clone ID 33-03-G02 to neutralize PD-L1/PD-1 interaction in reporter gene assay.**

| PRO ID | Mutations | Neutralization of PD-L1 in NF-AT potency assay | |
|---|---|---|---|
| | | IC$_{50}$ (ng/ml) | rel. IC$_{50}$* |
| PRO830 | NA | 20.36 | 1 |
| PRO1075 | VL-Q108A | 827.7 | 0.021 |
| PRO1076 | VL-G109A | ND | |
| PRO1089 | VH-Y112A | 1284 | 0.013 |
| PRO1434 | VL-Q108A/G109A | very low potency | |
| PRO1494 | VL-Q108A/VH-Y112A | 36761 | 5.89E-04 |

NA: not applicable ND: not determined

*: IC$_{50, \text{Avelumab}}$ (ng/ml)/IC$_{50, \text{test molecule}}$ (ng/ml)

**Storage stability study**

Method:

Humanized scFvs were subjected to stability studies such as a four-week stability study, in which the scFvs were formulated in an aqueous buffer (final buffer, 50 mM NaCiP, 150 mM NaCl, pH 6.4) at 10 mg/ml and stored at -80°C, 4°C and 40°C for four weeks. At the minimum, protein concentration by measurement of UV absorbance at 280 nm was measured and the fraction of monomers and oligomers in the formulation were evaluated by integration of SE-HPLC peak areas after one week, two weeks and at the end of each study. Parameters, such as % monomer content, % monomer loss, content and % content loss were recorded over time.

Results:

Table 12 and 13 compare d0, d7, d14 and endpoint measurements obtained at d28 of the study at 4°C and 40 °C. At 4°C all molecules show less then 10 % loss of monomeric content over 28 days except PRO1075 and PRO1076. At 40°C only two molecules showed a monomeric content above 85 % after 28 days, PRO1434 (86 %) and PRO1494 (90 %).

**Example 2: Generation and testing of multispecific constructs targeting HER2 (as an example of a TAA), PD-L1 and CD3 (as example of an immune cell antigen):**

This approach is directed at a next generation multi-specific immuno-oncology program targeting HER2-expressing malignant tumors. HER2 is a clinically validated target in several cancer types with unmet medical need, most prominently breast and gastric cancer. However, the spectrum of tumors (over-) expressing HER2 is much broader but - for mechanistic reasons - not accessible to conventional antibodies like trastuzumab while amenable for the present approach, which is designed to not only effectively mediate T cell-induced lysis of HER2-expressing tumors but at the same time to avoid tumor immune-escape by concomitant blockade of immune-suppressive PD-L1 signaling. The local restriction of the two additive, likely simultaneously acting mechanisms of action to tumor tissue, is expected to provide compounds in accordance with the present approach a substantially expanded efficacy profile with anticipated clinical efficacy even in HER2 expressing tumors primarily or secondarily refractory to standard anti-HER2 therapies. The present approach should lead to i) at least as potent, and more selective blockade of PD-1/PD-L1 interaction than Avelumab/Bavencio®. Specifically, compounds in accordance with the present approach should efficiently block PD-1 binding to PD-L1 on HER2/PD-L1 co-expressing target cells, while blockade of the PD-1/PD-L1 interaction on cells not expressing Her2 should be much less potent than with Avelumab/Bavencio®, ii) at least as potent and more selective lysis of HER2/PD-L1 co-expressing cells by peripheral blood mononuclear cells, when compared to Trastuzumab/Herceptin®, Avelumab/Bavencio® and the combination of the two. More specifically, compounds in accordance with the present approach should potently lyse cells co-expressing HER2 and PD-L1 while no lysis of cells expressing PD-L1 only should occur.

**Design and production of Tribodies, DVD-Tribodies and MATCH-4 molecules**

A description of the different formats (Tribody, DVD-Tribody and MATCH-4) designed within the present approach is presented in Figure 3. The data regarding the production of all the molecules are detailed in Table 14, which shows a description of the domain compositions of the different molecules produced and their positioning within the molecules. The targets for the different domains are: Trastuzumab: Her2; clone 14-11-D07: IL23R; clone 23-13-A01: human/mouse SA; clone 28-21-D09: CD3e; clone 33-02-G02 and mutants thereof: PD-L1.

**Table12: 4w stability study scFv fragments based on clone with Clone ID 33-03-G02 at 4°C of the scFv.**

| PRO ID | Mutations | Stress Stability, % mc at 4°C | | | |
|---|---|---|---|---|---|
| | | d0 | d7 | d14 | d28 |
| PRO1075 | VL-Q108A | 93.2 | 76.3 | 76.1 | 74.0 |
| PRO1076 | VL-G109A | 95.8 | 81.7 | 78.4 | 77.1 |
| PRO1089 | VH-Y112A | 99.1 | 98.0 | 97.6 | 96.5 |
| PRO1434 | VL-Q108A/G109A | 99.6 | 98.9 | 98.2 | 96.7 |
| PRO1494 | VL-Q108A/VH-Y112A | 99.3 | 98.9 | 98.8 | 98.1 |

**Table 13: 4w stability study at 40°C of the scFv.**

| PRO ID | Mutations | Stress Stability, % mc at 4°C | | | |
|---|---|---|---|---|---|
| | | d0 | d7 | d14 | d28 |
| PRO1075 | VL-Q108A | 93.2 | 80.0 | 80.1 | 78.8 |
| PRO1076 | VL-G109A | 95.8 | 81.9 | 81.7 | 81.4 |
| PRO1089 | VH-Y112A | 99.4 | 91.7 | 88.0 | 84.5 |
| PRO1434 | VL-Q108A/G109A | 99.6 | 87.1 | 86.6 | 86.0 |
| PRO1494 | VL-Q108A/VH-Y112A | 99.3 | 96.4 | 93.8 | 90.1 |

**Table 14: Composition of multispecific molecules according to the present approach**

| PRO ID | Format | Domain 1 | Specificity 1 | Domain 2 | Specificity 2 | Domain 3 | Specificity 3 | Domain 4 | Specificity 4 |
|---|---|---|---|---|---|---|---|---|---|
| PRO1454 | Tribody | Trastuzumab | Her2 | 28-21-D09 sc04 | CD3 | 33-03-G02, VL-G109A | PD-L1 | NA | NA |
| PRO1455 | Tribody | 14-11-D07-sc03 | IL23R | 28-21-D09 sc04 | CD3 | 33-03-G02, VL-G109A | PD-L1 | NA | NA |
| PRO1456 | Tribody | Trastuzumab | Her2 | 28-21-D09 sc04 | CD3 | 14-11-D07-sc03 | IL23R | NA | NA |
| PRO1497 | Tribody | Trastuzumab | Her2 | 28-21-D09 sc04 | CD3 | 33-03-G02, VL-Q108A, VL-G109A** | PD-L1 | NA | NA |
| PRO1498 | Tribody | 14-11-D07-sc03 | IL23R | 28-21-D09 sc04 | CD3 | 33-03-G02, VL-Q108A, VL-G109A** | PD-L1 | NA | NA |
| PRO1543 | MATCH-4 | Trastuzumab, VL-G141C/VH-G51C* | Her2 | 23-13-A01-sc02, VL-G141C/VH-G51C* | HSA | 28-21-D09 sc04 | CD3 | 33-03-G02, VL-Q108A, VL-G109A** | PD-L1 |
| PRO1543 variant | MATCH-4 | Trastuzumab, VL-G141C/VH-G51C* | Her2 | 19-01-H04-sc03 | HSA | 28-21-D09 sc04 | CD3 | 33-03-G02, VL-Q108A, VL-G109A** | PD-L1 |
| PRO1544 | MATCH-4 | 33-03-G02, VL-Q108A,VL-G109A** | PD-L1 | 23-13-A01-sc02, VL-G141C/VH-G51C* | HSA | 28-21-D09 sc04 | CD3 | Trastuzumab, VL-G141C/VH-G51C* | Her2 |
| PRO1545 | MATCH-4 | 33-03-G02, VL-Q108A, VL-G109A** | PD-L1 | 23-13-A01-sc02, VL-G141C/VH-G51C* | HSA | 28-21-D09 sc04 | CD3 | 14-11-D07-sc03 | IL23R |
| PRO1546 | MATCH-4 | 14-11-D07-sc03 | IL23R | 23-13-A01-sc02, VL-G141C/VH-G51C* | HSA | 28-21-D09 sc04 | CD3 | 33-03-G02, VL-Q108A, VL-G109A** | PD-L1 |
| PRO1547 | DVD-Tribody | Trastuzumab | Her2 | 23-13-A01-sc02 | HSA | 28-21-D09 sc04 | CD3 | 33-03-G02, VL-Q108A, VL-G109A** | PD-L1 |

(continued)

| PRO ID | Format | Domain 1 | Specificity 1 | Domain 2 | Specificity 2 | Domain 3 | Specificity 3 | Domain 4 | Specificity 4 |
|---|---|---|---|---|---|---|---|---|---|
| PRO1548 | DVD-Tribody | Trastuzumab | Her2 | 23-13-A01-sc02 | HSA | 28-21-D09 sc04 | CD3 | 14-11-D07-sc03 | IL23R |
| PRO1557 | MATCH-4 | Trastuzumab, VL-G141C/VH-G51C* | Her2 | 23-13-A01-sc02, VL-G141C/VH-G51C* | HSA | 28-21-D09 sc04 | CD3 | 14-11-D07-sc03 | IL23R |
| PRO1558 | MATCH-4 | 14-11-D07-sc03 | IL23R | 23-13-A01-sc02, VL-G141C/VH-G51C* | HSA | 28-21-D09 sc04 | CD3 | Trastuzumab, VL-G141C/VH-G51C* | Her2 |

*VL/VH interdomain disulfide bond, numbering according to A. Honegger
**mutations, numbering according to A. Honegger

Method:

Expression of Tribodies, DVD-Tribodies and MATCH-4 constructs was performed in CHO-S cells using CHOgro transient transfection kit (Mirus). Cultures were harvested after 5-7 days (cell viability <70 %) of expression at 37°C by centrifugation and proteins were purified from clarified culture supernatants by Protein L affinity chromatography followed, if needed, by a polishing step by size-exclusion chromatography. For the quality control of the manufactured material standard analytical methods, such as SE-HPLC, UV280 and SDS-PAGE were used.

**Affinities to human PD-L1, IL-23R, Her2, CD3$\varepsilon$ and human and mouse serum albumin (SA) by SPR**

Method:

Affinity to PD-L1 was determined by surface plasmon resonance (SPR) measurements using a Biacore T200 device (GE Healthcare) as described above. The apparent dissociation ($k_d$) and association ($k_a$) rate constants and the apparent dissociation equilibrium constant ($K_D$) were calculated with the Biacore analysis software (BIAevaluation, GE Healthcare) using one-to-one Langmuir binding model. The quality of the fits was monitored based on Chi2 and U-value. In addition to the kinetic measurement, the value of the $K_D$ was obtained by fitting a plot of response at equilibrium against the concentration (steady-state affinity measurement).

Binding affinities of multi-specific constructs towards recombinant human CD3$\varepsilon$ ECD (SinoBiological, cat. 10977-H08H), recombinant human IL-23R (custom-made by Trenzyme) and recombinant human Her2 ECD (SinoBiological, cat. 10004-HCCH) were measured by SPR using a Biacore T200 instrument. All measurements were performed at 25 °C. The different proteins were immobilized on a sensor chip (CM5 sensor chip, GE healthcare) by amine-coupling to reach an immobilization level of approximately 100 response units (RUs). Serial dilutions of the multi-specific molecules ranging from 0.35 to 90 nM (two-fold dilutions steps) in running buffer were injected into the flow cells at a flow rate of 30 $\mu$l/min for 5 min. Dissociation of the multispecific constructs from the CD3$\varepsilon$, IL-23R and Her2 on the CM5 chip was allowed to proceed for 12 min. After each injection cycle, surfaces were regenerated with one injection of 10 mM glycine HCl, pH 2. Obtained binding curves were double-referenced (empty reference channel and zero analyte injection) and the values of kd, ka and $K_D$ were calculated with the Biacore analysis software using one-to-one Langmuir binding model and quality of the fits was monitored based on Chi2 and U-value. Since the fits using the one-to-one Langmuir binding model showed suboptimal quality of curve fitting for CD3$\varepsilon$, the $K_D$ was in addition calculated using a two state reaction model. This model describes a 1:1 binding of analyte to immobilized ligand followed by a conformational change that stabilizes the complex.

Affinity of molecules to human serum albumin (HSA) and mouse serum albumin (MSA) was determined by SPR measurements using a Biacore T200 device (GE Healthcare). SA was directly coupled to a CM5 sensor chip (GE Healthcare) using amine coupling chemistry. After performing a regeneration scouting and surface performance test to find best assay conditions, a dose response of the molecules of interest with concentrations ranging from 0.7 to 180 nM was tested. The assay was run in a PBS-Tween buffer at pH 5.5. Association and dissociation time were set to 180 s and 720 s, respectively. Obtained binding curves were double-referenced (empty reference channel and zero analyte injection) and fitted using BiaEvaluation software (GE Healthcare) and the 1:1 Langmuir binding model. Retrieved kinetic parameters were used to calculate the apparent dissociation equilibrium constant ($K_D$).

Results:

As shown in Table 15 and Table 16, binding to CD3$\varepsilon$, human and mouse serum albumin was similar for all molecules tested, and molecules containing the anti-IL23R domain showed comparable affinities to IL23R. Affinity determination by SPR of low affinity domains is very difficult due to the high amount of protein that has to be injected in order to cover the concentration range corresponding to the affinity of the molecules. Therefore, a reliable measurement of the affinity to PD-L1 could not be obtained for some molecules. In addition, in case of low affinity domains steady state analysis of the SPR measurement might be more adequate due to the high bulk shifts observed, which introduce artefacts in the kinetic analysis. Affinity measurement for human PD-L1 was valid for only one Tribody, PRO1498. For the MATCH-4 molecules, valid measurements were obtained using the steady state analysis. The lowest affinities to PD-L1 were around 900 nM for PRO1544, PRO1545 and PRO1547. PRO1543 and PRO1546 showed similar affinities around 300 nM. MATCH-4 proteins carrying the lambda capped Trastuzumab G100C/G172C mutant anti-Her2 domain (PRO1543, PRO1544, PRO1557 and PRO1558) showed similar affinities to Her2 (300 to 400 pM), which again is comparable to the affinities of Trastuzumab anti-Her2 domain incorporated in Tribody (PRO1497) and DVD-Tribody (PRO1547 and PRO1548).

**Table 15: Summary of affinities of constructs of the present approach for binding to human PD-L1, Her2 and IL23R by SPR.**

| | Affinity to human Her2 | | | Affinity to human PD-L1 | | | | Affinity to human IL23R | | |
| | | | | kinetic analysis | | | steady state analysis | | | |
| PRO ID | $k_a$(M$^{-1}$s$^{-1}$) | $k_d$(s$^{-1}$) | $K_D$(M) | $k_a$(M$^{-1}$s$^{-1}$) | $k_d$ (s$^{-1}$) | $K_D$(M) | $K_D$(M) | $k_a$(M$^{-1}$s$^{-1}$) | $k_d$(s$^{-1}$) | $K_D$(M) |
|---|---|---|---|---|---|---|---|---|---|---|
| PRO1454 | not measured | | | not measured | | | | not measured | | |
| PRO1455 | not measured | | | not measured | | | | not measured | | |
| PRO1456 | not measured | | | not measured | | | | not measured | | |
| PRO1497 | 2.07E+05 | 5.40E-05 | 2.61E-10 | not determined | | | | no binding | | |
| PRO1498 | no binding | | | 1.14E+03 | 3.79E-04 | 3.32E-07 | not measured | 4.95E+05 | 2.78E-04 | 5.63E-10 |
| PRO1543 | 1.54E+05 | 6.26E-05 | 4.06E-10 | 1.76E+04 | 3.67E-04 | 2.09E-08 | 3.47E-07 | no binding | | |
| PRO1544 | 1.57E+05 | 5.56E-05 | 3.54E-10 | 4.12E+03 | 1.17E-03 | 2. 84E-07 | 8.09E-07 | no binding | | |
| PRO1545 | no binding | | | 1.95E+03 | 2.20E-03 | 1.13E-06 | 8.79E-07 | 3.09E+05 | 4.71E-04 | 1.52E-09 |
| PRO1546 | no binding | | | 2.41E+04 | 3.45E-04 | 1.43E-08 | 3.80E-07 | 4.58E+05 | 3.88E-04 | 8.48E-10 |
| PRO1547 | 1.66E+05 | 6.00E-05 | 3.61E-10 | no determined | | | 9.16E-07 | no binding | | |
| PRO1548 | 1.87E+05 | 6.06E-05 | 3.23E-10 | no binding | | | | 2.40E+05 | 2.36E-04 | 9.85E-10 |
| PRO1557 | 2.00E+05 | 6.35E-05 | 3.18E-10 | no binding | | | | 5.52E+05 | 5.14E-04 | 9.31E-10 |
| PRO1558 | 2.02E+05 | 6.07E-05 | 3.00E-10 | no binding | | | | 5.68E+05 | 4.93E-04 | 8.68E-10 |

**Table 16: Summary of affinities of constructs of the present approach for binding to human CD3$\varepsilon$, human and mouse serum albumin (SA) by SPR.**

| PRO ID | Affinity to human CD3 | | | | | | | Affinity to human SA (at pH5.5) | | | Affinity to mouse SA (at pH5.5) | | |
|---|---|---|---|---|---|---|---|---|---|---|---|---|---|
| | $k_a1(M^{-1}s^{-1})$ | $k_d1\ (s^{-1})$ | $K_D1\ (M)$ | $k_a2(M^{-1}s^{-1})$ | $k_d2\ (s^{-1})$ | $K_D2\ (M)$ | $K_D(M)$ | $k_a(M^{-1}s^{-1})$ | $k_d\ (s^{-1})$ | $K_D(M)$ | $k_a(M^{-1}s^{-1})$ | $k_d\ (s^{-1})$ | $K_D(M)$ |
| PRO1454 | not measured | | | | | | | NA | | | NA | | |
| PRO1455 | not measured | | | | | | | NA | | | NA | | |
| PRO1456 | not measured | | | | | | | NA | | | NA | | |
| PRO1497 | 2.31E+05 | 5.94E-03 | 2.57E-08 | 7.20E-04 | 2.86E-03 | 3.97E+00 | 2.06E-08 | NA | | | NA | | |
| PRO1498 | 1.97E+05 | 5.84E-03 | 2.96E-08 | 1.05E-03 | 1.98E-03 | 1.89E+00 | 1.95E-08 | NA | | | NA | | |
| PRO1543 | 1.90E+05 | 5.42E-03 | 2.85E-08 | 4.08E-03 | 5.03E-03 | 1.23E+00 | 1.57E-08 | 1.45E+05 | 1.18E-04 | 8.10E-10 | 6.02E+04 | 1.17E-03 | 1.95E-08 |
| PRO1544 | 2.22E+05 | 5.16E-03 | 2.32E-08 | 3.49E-03 | 4.20E-03 | 1.20E+00 | 1.27E-08 | 1.39E+05 | 1.21E-04 | 8.70E-10 | 5.75E+04 | 1.12E-03 | 1.95E-08 |
| PRO1545 | 1.69E+05 | 6.45E-03 | 3.82E-08 | 5.51E-03 | 4.32E-03 | 7.84E-01 | 1.67E-08 | 1.71E+05 | 1.19E-04 | 6.93E-10 | 6.79E+04 | 1.08E-03 | 1.59E-08 |
| PRO1546 | 1.97E+05 | 7.52E-03 | 3.82E-08 | 5.77E-03 | 5.33E-03 | 9.24E-01 | 1.83E-08 | 1.68E+05 | 1.34E-04 | 7.97E-10 | 6.70E+04 | 1.16E-03 | 1.73E-08 |
| PRO1547 | 1.97E+05 | 4.97E-03 | 2.52E-08 | 6.04E-04 | 8.19E-04 | 1.36E+00 | 1.45E-08 | n ot measured | | | not measured | | |
| PRO1548 | 2.82E+05 | 4.06E-03 | 1.44E-08 | 9.47E-04 | 1.29E-03 | 1.36E+00 | 8.28E-09 | n ot measured | | | not measured | | |
| PRO1557 | 2.83E+05 | 5.29E-03 | 1.87E-08 | 4.35E-03 | 3.56E-03 | 8.18E-01 | 8.43E-09 | 2.40E+05 | 1.20E-04 | 4.98E-10 | 9.56E+04 | 1.07E-03 | 1.12E-08 |
| PRO1558 | 3.15E+05 | 4.96E-03 | 1.57E-08 | 3.27E-03 | 3.35E-03 | 1.02E+00 | 7.95E-09 | 2.45E+05 | 1.23E-04 | 5.01E-10 | 9.58E+04 | 1.10E-03 | 1.15E-08 |
| NA: not applicable | | | | | | | | | | | | | |

**PD-L1/PD-1 neutralization by FC**

Method:

These assays were conducted to assess the ability of the multi-specific low affinity PD-L1x/Her2 constructs to neutralize the interaction between PD-1 and PD-L1 expressed on HCC1954 cells, which also express Her2. Specifically, the molecules should efficiently block PD-1 binding to PD-L1 on Her2/PD-L1 co-expressing target cells (HCC1954), while blockade of the PD-1/PD-L1 interaction on cells not expressing Her2 (HCC827) should be much less potent than with Avelumab/Bavencio® or Nivolumab/Obdivo®. Blockade of PD-1 binding to the cells was analyzed by FC and compared to the reference IgG Avelumab. Moreover, HCC827 cells were used as an additional control as these cells express PD-L1 at comparable levels than HCC1954 cells but lack significant expression of Her2.

HCC1954 and HCC827 cells were stimulated with 10 ng/ml human IFN$\gamma$ for 24 h to further induce the expression of PD-L1. Next day, HCC827 and HCC1954 cells were detached, centrifuged for 4 min with 200 g, re-suspended in PBS/2 % FCS/2 mM EDTA (staining buffer) and seeded into 96 well PP microplates (50 $\mu$l/well). Dilution series of three-fold steps of the multi-specific molecules and of Avelumab starting at 20 $\mu$g/ml and 5 $\mu$g/ml, respectively, were prepared in staining buffer containing 500 ng/ml biotinylated PD 1. Plates of HCC827 and HCC1954 were centrifuged for 4 min with 200 g and the dilution series were added to the cells (100 $\mu$l/well) and incubated for 30 min at RT. Next, plates were washed once with 150 $\mu$l staining buffer and Streptavidin-PE solution was added to the cells (100 $\mu$l/well) and incubated for 30 min at 4°C. As a next step, cells were washed again as indicated above and then cells were re-suspended in 100 $\mu$l of staining buffer. Re-suspended cells were then processed for fluorescence measurement using NovoCyte flow cytometer (ACEA Bioscience Inc.). Mean fluorescence intensity of PE-labeled PD-1 was reported and data were fitted using sigmoidal 4PL fit (GraphPad Prism). Individual $IC_{50}$ values on each plate were calibrated against the $IC_{50}$ of the reference molecule Avelumab that was taken along on each plate (relative $IC_{50}$: $IC_{50}$, Avelumab/$IC_{50}$, test molecule). In addition, the ratio between the relative $IC_{50}$ values found on high Her2 expressing HCC1954 and low Her2 expressing HCC827 cells was calculated.

Results:

Obtained potencies of constructs according to the present approach to neutralize PD-1/PD-L1 interaction are summarized in Table 17. On HCC1954 cells expressing high levels of Her2 and PD-L1, the Tribody PRO1454 carrying the single alanine mutant PD-L1 domain (33-03-G02 G109A) showed a similar potency as Avelumab whereas on cells expressing only PD-L1 (HCC827) the potency of PRO1454 was 14-fold lower. The Tribody PRO1497 carrying the double alanine mutant 33-03-G02 Q108A/G109A neutralized PD-1/PD-L1 interaction on Her2/PD-L1 positive cells (HCC1954) almost as efficient as PRO1454 as shown by similar relative $IC_{50}$ values (PRO1454: 0.66, PRO1497: 0.38). However, in contrast to PRO1454, PRO1497 demonstrated only very weak neutralization potency on PD-L1 expressing cells (HCC827) (Figure 4). This finding shows the potentially larger therapeutic window of molecules such as PRO1497 containing a PD-L1 domain with an affinity equivalent to the domain carrying both mutations (Q108A/G109A) versus a domain with an affinity similar to the single alanine mutant (G109A).

All MATCH-4 molecules containing Trastuzumab anti-Her2 domain and the anti-PD-L1 domain carrying both mutations (Q108A/G109A) had similar potencies (rel. $IC_{50}$ values, PRO1543: 0.28, PRO1544: 0.24) when compared to the Tribody PRO1497. Titration curves obtained for PRO1543 and PRO1546 are presented in Figure 5. Along that line, the DVD-Tribody PRO1547 containing Trastuzumab anti-Her2 domain and the anti-PD-L1 domain carrying both mutations (Q108A/G109A) neutralized PD-1/PD-L1 interaction with a potency similar to PRO1497 (rel. IC50 values, PRO1547: 0.25).

**Table17: Potencies of multi-specific molecules to neutralize PD-L1/PD-1 interaction in flow cytometry assay.**

| | Neutralization of PD-L1/PD-1 interaction in FC based assay | | | | |
| --- | --- | --- | --- | --- | --- |
| | High Her2 expressing cells (HCC1954) | | Low Her2 expressing cells (HCC827) | | Ratio |
| **PRO ID** | $IC_{50}$ (ng/ml) | rel. $IC_{50}$* | $IC_{50}$ (ng/ml) | rel. $IC_{50}$* | rel. $IC_{50}$ (high Her2) / rel. $IC_{50}$ (low Her2) |
| PRO1454 | 205.5 | 0.66 | 1240 | 0.05 | 14.04 |
| PRO1455 | 2846 | 0.06 | 2796 | 0.01 | 4.83 |
| PRO1456 | no inhibition | | no inhibition | | NA |

(continued)

| Neutralization of PD-L1/PD-1 interaction in FC based assay | | | | | |
|---|---|---|---|---|---|
| | High Her2 expressing cells (HCC1954) | | Low Her2 expressing cells (HCC827) | | Ratio |
| PRO ID | $IC_{50}$ (ng/ml) | rel. $IC_{50}$* | $IC_{50}$ (ng/ml) | rel. $IC_{50}$* | rel. $IC_{50}$ (high Her2) / rel. $IC_{50}$ (low Her2) |
| PRO1497 | 352.2 | 0.38 | 719036 | 0.00 | 4886 |
| PRO1498 | 7046 | 0.02 | 8537 | 0.01 | 2.9 |
| PROL1543 | 600.5 | 0.28 | 59224 | 0.00 | 272.4 |
| PROL1544 | 527.7 | 0.24 | 45995 | 0.00 | 232.6 |
| PROL1545 | 4136 | 0.03 | 88075 | 0.00 | 55.66 |
| PROL1546 | 51902 | 0.00 | 254254 | 0.00 | 10.31 |
| PROL1547 | 522.3 | 0.25 | no inhibition | | NA |
| PROL1548 | not measured | | | | NA |
| PRO1557 | not measured | | | | NA |
| PROL1558 | not measured | | | | NA |
| Avelumab | 127.7 | 1 | 46.03 | 1 | 1 |
| Nivolumab | 43.48 | 2.94 | 39.77 | 1.16 | 2.54 |
| NA: not applicable *$IC_{50}$, Avelumab (ng/ml)/$IC_{50}$, test molecule (ng/ml) | | | | | |

## CD3 activation and PD-L1/PD-1 interaction blockade by NFAT reporter gene assay

Method:

T-cell activation was tested in an NFAT (nuclear factor of activated T-cells) assay to assess simultaneous effects of the molecules on CD3 cross-linking and PD-1/PD-L1 blockade. Specifically, the molecules should efficiently induce CD3 activation and block PD-1 binding to PD-L1 on HER2/PD-L1 co-expressing target cells (HCC1954), while CD3 activation and blockade of the PD-1/PD-L1 interaction on cells not expressing Her2 (CHO-PD-L1) should be much less potent. The Jurkat PD-1 NFAT reporter T cell line expresses the luciferase reporter gene under control of the NFAT response elements from the IL-2 promoter. The transcription factor NFAT is activated upon cross-linking of CD3ε and induces a number of genes involved in T cell activation. In this system, cross-linking of CD3ε induces expression of the luciferase reporter gene. In addition, the interaction between PD-L1 and PD-1 negatively regulates such CD3ε signaling and thus diminishes firefly luciferase expression. Therefore, blockade of the PD-L1/PD-1 interaction in this system leads to increased luciferase activity. HCC1954 cells stimulated for 24 h with 10 ng/ml IFNy to increase PD-L1 expression and PD-L1 expressing CHO-K1 cells (clone A2) were used as target cells and seeded at 25'000 cells per well in 50 μl on 96-well culture plates. Serial dilutions of the molecules to be tested were prepared in assay medium containing 50 mg/ml hSA and 25 μl were added to the cells (final concentrations range from 250 nM to 0.026 pM). PD-1 expressing Jurkat NFAT reporter cells were prepared in assay medium containing hSA at 50 mg/ml and added at a cell density of 50'000 cells per well. Luciferase expression was detected by addition of Luciferase reagent and was read by a luminescence reader 5 or 22 h after addition of Jurkat PD-1 NFAT reporter cells. Relative luminescence units (RLU) are presented. Potency of PRO1497 which is a HER2xPD-L1 $_{high\ KD}$xCD3 scDb-scFv was used as a reference for calculation of the relative potency of the extended half-life molecules.

Results:

Potency to trigger CD3 activation and PD-L1/PD-1 interaction blockade concomitantly was assessed by NFAT reporter gene assay and the results are presented in Table 18. Serial dilutions of the respective molecules to be tested as well as the reference scDb-scFv Her2xCD3xPD-L1$_{low\ affinity}$ (PRO1497) were added to the plates. Individual $IC_{50}$ values and maximal activation were normalized to the $IC_{50}$ and maximal activation of the reference molecule PRO1497 in presence of Her2 and PD-L1 expressing cells HCC1954 (relative $IC_{50}$ or Max. act.: $IC_{50}$ or Max. Act., PRO1497 on HCC1954/$IC_{50}$ or Max act., test molecule). In the presence of PD-L1/ Her2 expressing cells (HCC1954), Tribodies PRO1454, PRO1497 and PRO1456 activated CD3 signaling in Jurkat cells with similar $EC_{50}$ but the maximal activation was higher for PRO1454 and PRO1497, i.e. for molecules carrying the low affinity anti-PD-L1 domain, compared to PRO1456 containing an anti-IL-23R dummy domain instead of the anti-PD-L1 domain (Figure 6). This suggests that PRO1454 and PRO1497 simultaneously block PD-L1 and activate CD3 within the immunological synapse in presence of cells co-expressing Her2 and PD-L1. 27-fold weaker activation was observed with PRO1455 compared to PRO1454 and even 475-fold weaker with PRO1498 compared to PRO1497, i.e. molecules carrying no anti-Her2 domain but the low affinity anti-PD-L1 domain 33-03-G02 G109A or 33-03-G02 Q108A/G109A, respectively. This finding shows the potentially larger therapeutic window of molecules containing a PD-L1 domain with an affinity equivalent to the domain carrying both mutations (Q108A/G109A) versus a domain with an affinity similar to the single alanine mutant (G109A) both molecules being of similar potency as the molecule carrying no PD-L1 domain (PRO1456). Potencies are summarized in Table 18 which shows that PRO1543 had the best potency on Her 2/PD-L1 expressing cells (HCC1954) and one of the lowest in presence of PD-L1/very low Her2 expressing cells (HCC827). Titration curves obtained for PRO1543 and the control molecules PRO1546 and PRO1557 in presence or absence of 1 μg/ml Nivolumab and PRO1557 in combination with the low affinity PD-L1 domain PRO1434 are presented in Figure 7.

**Table18. Potencies of molecules to trigger CD3 activation and neutralize PD-L1/PD-1 interaction in NFAT reporter gene assay.**

| | Neutralization of PD-L1/PD-1 interaction in NFAT reporter gene assay | | | | | |
| | High Her2 expressing cells (HCC1954) | | | negative Her2 cells (CHO-PD-L1; A2 clone) | | |
| PRO ID | $IC_{50}$ (pM) | rel. $IC_{50}$* | rel. max. activation (%)$ | $IC_{50}$ (pM) | rel. $IC_{50}$* | rel. max. activation (%)$ |
|---|---|---|---|---|---|---|
| PRO1454 | 148 | 1** | 100$$ | not measured | | |
| PRO1455 | 4071 | 0.036** | 59$$ | not measured | | |
| PRO1456 | 291 | 1.24 | 75 | not measured | | |
| PRO1497 | 361 | 1 | 100 | not measured | | |
| PRO1498 | 172108 | 0.002 | 71 | not measured | | |
| PRO1543 | 402 | 0.32 | 102 | 29196 | 0.004 | 62 |
| PRO1543 + nivolumab | 125 | 4.75*** | 107$$$ | no activation | | |
| PRO1544 | 2290 | 0.08 | 78 | 10544 | 0.02 | 42 |
| PRO1545 | 12246 | 0.010 | 67 | 25996 | 0.006 | 59 |
| PRO1546 | 95436 | 0.001 | 28 | 31494 | 0.004 | 60 |
| PRO1546 + nivolumab | no activation | | | no activation | | |
| PRO1547 | 779 | 0.2 | 106 | 142091 | 0.001 | 19 |
| PRO1548 | 325 | 0.5 | 86 | no activation | | |
| PRO1557 | 391 | 0.30 | 82 | no activation | | |
| PRO1557 + nivolumab | 319 | 1.86*** | 115$$$ | no activation | | |
| PRO1557 + PRO1434 | 671 | 0.88*** | 75$$$ | no activation | | |

(continued)

### Neutralization of PD-L1/PD-1 interaction in NFAT reporter gene assay

| | High Her2 expressing cells (HCC1954) | | | negative Her2 cells (CHO-PD-L1; A2 clone) | | |
|---|---|---|---|---|---|---|
| PRO ID | $IC_{50}$ (pM) | rel. $IC_{50}$* | rel. max. activation (%)$ | $IC_{50}$ (pM) | rel. $IC_{50}$* | rel. max. activation (%)$ |
| RO1558 | 2714 | 0.06 | 53 | no activation | | |

NA: not applicable

*: $IC_{50, \text{PRO1497 on HCC1954}}$ (pM)/$IC_{50, \text{test molecule}}$ (pM)

** : $IC_{50, \text{PRO1454 on HCC1954}}$ (pM)/$IC_{50, \text{test molecule}}$ (pM)

***: $IC_{50, \text{PRO1543 on HCC1954}}$ (pM)/$IC_{50, \text{test molecule}}$ (pM)

$: Max. activation, $_{\text{PRO1497 on HCC1954}}$ (RLU)/Max. activation, $_{\text{test molecule}}$ (RLU)

$$: Max. activation, $_{\text{PRO1454 on HCC1954}}$ (RLU)/Max. $_{\text{activation, test molecule}}$ (RLU)

$$$: Max. activation, $_{\text{PRO1543 on HCC1954}}$ (RLU)/Max. $_{\text{activation, test molecule}}$ (RLU)

## Cytotoxicity assay (T-cell driven target cell depletion)

To assess the ability of the compounds of the present approach to selectively direct T cells to Her2 and PD-L1 co-expressing cells, a cytotoxicity assay using Her2 and PD-L1 positive cell lines (HCC1954) were performed, in the presence of human PBMCs. Simultaneous binding to both targets by the compounds of the present approach lead to cross-linking of CD3ε on T cells and activated a signaling cascade that triggers T cell activation (CD69 upregulation, cytokine secretion) and the release of cytotoxic granules, which ultimately resulted in target cell killing. In contrast to the combination of Avelumab/Bavencio® and Trastuzumab/Herceptin®, the compounds of the present approach should selectively lyse cells co-expressing PD-L1 and HER2 while no lysis of cells solely expressing PD-L1 (HCC827) should be observed.

Methods:

*Blood cells fractionation:*

Peripheral blood mononuclear cells (PBMC) were isolated from fresh blood of healthy volunteers using the lymphocyte separation medium Lymphoprep (Stemcell technologies) according to manufacturer's instructions. Briefly, blood was diluted 1:2 with human PBMC isolation buffer (PBS, 2 % FCS, 2 mM EDTA) or cynomolgus PBMCs isolation buffer (PBS, 5 % FCS, 2 mM EDTA) and applied to Leucosep tubes containing recommended amount of Lymphoprep medium. LeucoSep tubes were centrifuged 30 min at 800 g (human blood) or 2000 g (cynomolgus blood) without brakes at RT. Then, the cell layer containing PBMCs was collected and washed twice with human PBMCs isolation buffer and red blood cells were lysed using red blood cells lysis buffer for 5 min at RT. Isolated human cells were then washed once with their respective isolation buffer and once with assay medium (RPMI-1640, 10% FCS). After platelet removal, isolated PBMCs were resuspended in assay medium at a density of $3 \times 10^6$ viable cells per ml.

*Flow cytometry-based in vitro cytotoxicity assay (FC assay), CD8+ T cells activation and CD11c+, CD4+ T cells, CD8+ T cells viability:*

Three cell lines were used as target cells, HCC1954 (co-expressing high levels of HER2 and PD-L1) stimulated for 24h with 10 ng/ml IFNy to increase PD-L1 expression as well as CHO-PD-L1 (no Her2 and high PD-L1) and CHO-K1 cell line used as negative control cell line. 5'000 viable target cells previously labelled with PKH67 and diluted in 75 µl of assay medium (RPMI-1640, 10% FCS) were added to 96-well plates. 25 µl of 6 times concentrated test proteins diluted in assay medium were added to appropriate wells. 150'000 viable effector cells (PBMCs) diluted in 50 µl assay medium were added to each well (E:T ratio of 30:1) and plates were mixed on a nutating mixer at RT prior to their incubation at 37°C, 5 % CO2. After 16 h or 40 h, cells were trypsinized, resuspended in staining buffer (PBS, 2 % BCS, 2 mM EDTA) and transferred into non-binding plates.

Cells were stained for different markers such as CD69, CD8, CD4, CD11c and Annexin-V. For analysis, the focus was on apoptotic and dead target cells and activated CD8+ T cells. Thereby, target cells were identified by green fluorescence (PKH67) and their viability was analyzed by Annexin-V APC. Effector cells (CD8+ cells) were identified by detecting CD8 on their surface (anti-CD8 PerCP-Cy5.5). Activation of CD8+ T cells was finally detected by quantification of CD69 expression (anti-CD69 PE). CD4 was used to better discriminate CD8+ and CD4+ T cells. CD11c was used to stain monocytes and dendritic cells. For each marker except Annexin-V antibodies were incubated 30 min at RT under gentle agitation. Cells were washed once with staining buffer, once with Annexin binding buffer and Annexin-V staining was carried on for 30 min at RT under agitation. Cells were washed once with Annexin-V binding buffer and flow cytometry analysis was done on a Novocyte Flow Cytometer.

The percentage of specific target cells lysis was calculated according to the following equation:

$$Specific\ lysis\ of\ target\ cells\ [in\ \%]$$
$$= \left[1 - \frac{Viability\ target\ cells\ of\ sample}{average\ viability\ of\ control\ samples}\right] x\ 100$$

The percentage of activated CD8+ T cells corresponds to the proportion of CD69+ CD8+ T cells.

The percentage of viable CD4+, CD8+ T cells and CD11c+ cells correspond to the proportion of Annexin-V negative cells within the different cell populations.

Results:

Cytotoxic potential of the selected MATCH-4 molecule PRO1543 was assessed using the flow cytometry based cytotoxicity assay. Data obtained are presented in Table 19 and titration curves for PRO1543 and both control molecules PRO1546 and PRO1557 are presented in Figure 8. PRO1543 bearing the anti-Her2 and the low affinity anti-PD-L1 domain is more potent than PRO1557 which carries the anti-Her2 domain but contains the anti-IL-23R dummy domain instead of the low affinity anti-PD-L1 domain. This data show the additional effect of PD-L1 targeting within the immunlogical synapse leading to improved target cell lysis.

CD8+ T cell activation correlates with the ranking of the cytotoxic potencies of the different molecules PRO1543 being the most potent molecule, and Nivolumab treatment showing not additional effect. Potencies and maximal activation of PRO1543 are presented in Table 20 and titration curves obtained are showed in Figure 9.

**Table19: Potencies of PRO1543 for target cell lysis.**

| PRO ID | High Her2 expressing cells (HCC1954) | | | | Her2-/PD-L1+ CHO-PD-L1 (A2 clone) | | | | Her2-/PD-L1- cells (CHO-K1) | | | |
| | $IC_{50}$(pM) | | max. lysis (%) | | $IC_{50}$ (pM) | | max. lysis (%) | | $IC_{50}$(pM) | | max. lysis (%) | |
| | 16h | 40h | 16h | 40h | 16h | 40h | 16h | 40h | 16h | 40h | 16h | 40h |
|---|---|---|---|---|---|---|---|---|---|---|---|---|
| PRO1454 | | | | | Not measured | | | | | | | |
| PRO1455 | | | | | Not measured | | | | | | | |
| PRO1456 | | | | | Not measured | | | | | | | |
| PRO1497 | | | | | Not measured | | | | | | | |
| PRO1498 | | | | | Not measured | | | | | | | |
| PRO1543 | 1.34 | 1.01 | 98.1 | 98.40 | 1726 | 1837 | 85.80 | 83.30 | no target cell killing | | | |
| PRO1543 + Nivolumab | 0.66 | 0.874 | 97.2 | 96.80 | 2238 | 1640 | 94.10 | 83.50 | | | | |
| PRO1546 | 1926 | 1799 | 89.4 | 97.00 | 4759 | 2771 | 90.80 | 84.40 | no target cell killing | | | |
| PRO1546 + Nivolumab | 1040 | 942 | 92.1 | 97.90 | 1913 | 2719 | 91.60 | 88.80 | | | | |
| PRO1557 | 13.4 | 7.33 | 96.5 | 97.10 | no target cell killing | | | | no target cell killing | | | |
| PRO1557 + Nivolumab | 2.02 | 4.48 | 98 | 97.10 | no target cell killing | | | | | | | |
| PRO1557 + PRO1434 | 5.92 | 6.15 | 96.9 | 7.4 | no target cell killing | | | | | | | |

**Table20. Potencies of PRO1543 for CD8+ T cell activation**

| | High Her2 expressing cells (HCC1954) | | | | Her2-/PD-L1+ CHO-PD-L1 (A2 clone) | | | | Her2-/PD-L1- cells (CHO-K1) | | | |
|---|---|---|---|---|---|---|---|---|---|---|---|---|
| | IC$_{50}$(pM) | | max. act. (%) | | IC$_{50}$(pM) | | max. act. (%) | | IC$_{50}$(pM) | | max. act. (%) | |
| PRO ID | 16h | 40h | 16h | 40h | 16h | 40h | 16h | 40h | 16h | 40h | 16h | 40h |
| PRO1454 | | | | | | Not measured | | | | | | |
| PRO1455 | | | | | | Not measured | | | | | | |
| PRO1456 | | | | | | Not measured | | | | | | |
| PRO1497 | | | | | | Not measured | | | | | | |
| PRO1498 | | | | | | Not measured | | | | | | |
| PRO1543 | 16.35 | 12.09 | 87.48 | 89.48 | 6753.00 | 3716.00 | 75.67 | 89.53 | | no activiation | | |
| PRO1543 + Nivolumab | 14.59 | 10.83 | 81.98 | 90.87 | 5005.00 | 6628.00 | 79.42 | 89.43 | | | | |
| PRO1544 | | | | | | Not measured | | | | | | |
| PRO1545 | | | | | | Not measured | | | | | | |
| PRO1546 | 82153.00 | 5466.00 | 60.35 | 56.54 | 9807.00 | 43622.00 | 67.93 | 83.00 | | no activiation | | |
| PRO1546 + Nivolumab | 355563.00 | 9535.00 | 57.21 | 58.42 | 9471.00 | 7741.00 | 70.64 | 80.37 | | | | |
| PRO1547 | | | | | | Not measured | | | | | | |
| PRO1548 | | | | | | Not measured | | | | | | |
| PRO1557 | 100.20 | 110.70 | 80.67 | 88.96 | | no activation | | | | no activiation | | |
| PRO1557 + Nivolumab | 98.47 | 133.20 | 81.75 | 88.90 | | no activation | | | | | | |
| PRO1557 + PRO1434 | 99.60 | 95.76 | 81.41 | 87.53 | | no activation | | | | | | |
| PRO1558 | | | | | | Not measured | | | | | | |

CD4+ and CD8+ T cell viability was analyzed after 16 and 40 h. This gives a safety read-out as activated CD4+ and CD8+ T cells are expressing PD-L1 but not Her2 and might be targeted as well by PRO1543. As summarized in Table 21 and illustrated in Figure 10, PRO1543 decreased CD4+ and CD8+ T cell viability by 5 to 10 % and only at the highest concentrations tested. This can be considered as a minor effect on cell viability.

**Cytotoxicity assay on cell expressing different Her2 and PD-L1 levels (T-cell driven target cell depletion)**

Method:

Same method as in the example above was used. Four cell lines were used as target cells, HCC1954 (co-expressing high levels of HER2 and PD-L1), HCC1827 (expressing very low levels of HER2 and high levels of PD-L1) stimulated for 24 h with 10 ng/ml IFNy to increase PD-L1 expression, MCF-7 (expressing low levels of HER2 and very low PD-L1 levels) as well as CHO-PD-L1 (no Her2 and high PD-L1, purchased at BPS Bioscience. Please note that this cell line expresses PD-L1 at a about 9 times lower level compared to the CHO-PD-L1 clone A2 cell line used for the experiments shown in Tables 18 and 19, Figures 8 and 9).

Results:

The results of the cytotoxicity assay are shown in Table 22 and in Figures 11 and 12. The results demonstrate that the molecule comprising the low affinity PD-L1 domain, PRO1543, has superior potency over PRO1557 and PRO957 to kill cells expressing HER2 and PD-L1 while it does not kill cells expressing no HER2. Co-expression of HER2 and PD-L1 occurs only on cancer cells. PRO1543 furthermore mediates lysis of HER2/PD-L1 double-positive cancer cells more potently than _normal cells expressing only HER2. Thus PRO1543 in contrast to a molecule not containing a PD-L1 binding domain, reveals selectivity for double-positive cells and therefore spares normal cells expressing HER2. Due to the absence of a PD-L1 binding domain, PRO1557 and PRO957 have no such selectivity and their potency is solely determined by the HER2 expression level of the target cell.

**Table21. Percentage of viable CD4+ and CD8+ T cells after 40 h**

| PRO ID | High her2 expressing cells (HCC1954) | | Low Her2 expressing cells (HCC827) | | No Her2/PD-L1 cells (CHO-K1) | |
|---|---|---|---|---|---|---|
| | CD4+ cell viabilty range (%) | CD8+ cell viabilty range (%) | CD4+ cell viabilty range (%) | CD8+ cell viabilty range (%) | CD4+ cell viabilty range (%) | CD8+ cell viabilty range (%) |
| PRO1543 | 82-93 | 92-96 | 84-92 | 93-97 | 94-95 | 96-97 |
| PRO1546 | 86-94 | 92-98 | 90.9 | 95-98 | 93-94 | 97-98 |
| PRO1557 | 81-94 | 88-97 | 88-92 | 93-96 | 93-94 | 94-97 |

**Table 22. Potencies of PRO1543 for killing of different target cell lines**

| PRO ID | High Her2 expressing cells (HCC1954) | | Her2+/PD-L1- MCF-7 | | Her2+/-/PD-L1+ CHO-PD-L1 | | Her2-/PD-L1+ CHO-PD-L1 (BPS Bioscience) | |
|---|---|---|---|---|---|---|---|---|
| | IC$_{50}$ (pM) | max. lysis (%) | IC$_{50}$ (pM) | max. lysis (%) | IC$_{50}$ (pM) | max. lysis (%) | IC$_{50}$ (pM) | max. lysis (%) |
| | 16h | 16h | 16h | 16h | 16h | 16h | 16h | 16h |
| PRO1543 | 0.23 | 76.81 | 3.11 | 90.38 | 5.30 | 77.94 | no target cell killing | |
| PRO1557 | 2.79 | 77.49 | 18.14 | 90.78 | 449.30 | 81.77 | no target cell killing | |
| PRO957 | 11.78 | 69.97 | 52.58 | 82.70 | 430.20 | 51.64 | no target cell killing | |
| Trastuzumab + Nivolumab | 127.80 | 72.43 | 14.46 | 26.94 | no target cell killing | | no target cell killing | |

**Assessment of the anti-tumor efficacy of PD-L1 blockade and concomitant localized stimulation of CD3 in the human cell line-derived ductal breast carcinoma xenograft model HCC1954**

The anti-tumor activity of the compounds of the present approach was compared to anti-PD-1 therapy and anti-PD-1 anti-Her2 combined therapy in human HCC1954 ductal breast carcinoma xenografts using the immunodeficient NOG mice strain from Taconic and allogeneic human peripheral blood mononuclear cells. Effects of PRO1678 (scMATCH3) and PRO1543 (MATCH4) on tumor volume were compared to treatment with the anti-PD-1 antibody (Nivolumab) and the Nivolumab/anti Her2 antibody (Trastuzumab) combo. An irrelevant IgG palivizumab was used as a control IgG. Animal body weight was followed as well.

Study set-up and treatment schedule:

Female NOG mice received unilateral injections of 5x106 HCC1954 cells. Cells were injected in a mixture of 50 % cell suspension in PBS and 50 % matrigel in a total injection volume of 100 μl. After injection of tumor cells into NOG mice and successful tumor engraftment (median group tumor volume of 80-100 mm3), mice were substituted with 5x106 human PBMCs by intravenous injection. On the day of randomization, four mice of each group were reconstituted with PBMCs of donor A and another four mice with PBMCs of donor B. Treatment will start 1-2 hours after the injection of PBMCs and were applied as follows.

| group ID | compound | total daily dose [mg] | dosing days | route | no. of mice |
|---|---|---|---|---|---|
| 1 | **Palivizumab** | 0.1 | 0,5,10, 15, 20, 25,30 | ip | 8 |
| 2 | **Nivolumab** | 0.1 | 0,5,10, 15, 20, 25,30 | ip | 8 |
| 3 | **PRO1678 low dose** | 0.1 | 0,5,10, 15, 20, 25,30 | ip | 8 |
| 4 | **PRO1678 high dose** | 0.5 | 0,5, 10, 15, 20, 25,30 | ip | 8 |
| 5 | **PRO1543** | 0.05 | 0,5,10, 15, 20, 25,30 | ip | 8 |
| 6 | **Trastuzumab + Nivolumab** | 0.1+0.1 | 0,5,10, 15, 20, 25,30 | ip | 8 |

Body weights (Table 24) and tumor volume by caliper measurement (Table 23 and Figure 13) were performed twice weekly. Animals were terminated at day 33. In some few groups few animals died already at day 27 onwards. No body weight loss was observed.

EP 3 816 185 A1

**Table 231: Relative tumor volume**

| Molecule | Dose | Grp | | Day | | | | | | | | | | |
|---|---|---|---|---|---|---|---|---|---|---|---|---|---|---|
| | | | | 0 | 2 | 6 | 9 | 13 | 16 | 20 | 2b3 | 27 | 30 | 33 |
| Palivizumab | 0.1 | 1 | mean (%) | 100.0 | 125.0 | 195.6 | 267.8 | 319.8 | 402.6 | 521.5 | 635.2 | 740.9 | 840.9 | 1090.8 |
| | | | SD (%) | 0.0 | 10.6 | 37.9 | 56.3 | 65.0 | 86.6 | 117.7 | 152.1 | 157.1 | 178.7 | 275.7 |
| | | | N | 8.0 | 8.0 | 8.0 | 8.0 | 8.0 | 8.0 | 8.0 | 8.0 | 8.0 | 6.0 | 4.0 |
| Nivolumab | 0.1 | 2 | mean (%) | 100.0 | 107.9 | 129.7 | 174.0 | 218.8 | 267.9 | 313.3 | 332.2 | 426.8 | 518.5 | 615.4 |
| | | | SD (%) | 0.0 | 16.5 | 41.4 | 65.3 | 77.3 | 91.9 | 127.9 | 132.7 | 178.8 | 168.4 | 252.3 |
| | | | N | 8.0 | 8.0 | 8.0 | 8.0 | 8.0 | 8.0 | 8.0 | 8.0 | 7.0 | 6.0 | 5.0 |
| PRO1678 | 0.1 | 3 | mean (%) | 100.0 | 115.4 | 156.1 | 182.0 | 204.7 | 242.0 | 287.8 | 368.4 | 428.0 | 512.2 | 489.0 |
| | | | SD (%) | 0.0 | 12.4 | 40.8 | 61.9 | 86.9 | 94.0 | 128.3 | 153.9 | 213.3 | 245.1 | 236.9 |
| | | | N | 8.0 | 8.0 | 8.0 | 8.0 | 8.0 | 8.0 | 8.0 | 8.0 | 8.0 | 8.0 | 7.0 |
| PRO1678 | 0.5 | 4 | mean (%) | 100.0 | 114.8 | 118.9 | 138.5 | 145.6 | 164.5 | 193.7 | 213.4 | 261.8 | 312.3 | 388.7 |
| | | | SD (%) | 0.0 | 8.3 | 21.8 | 32.0 | 32.7 | 45.6 | 45.0 | 61.7 | 57.2 | 102.3 | 162.7 |
| | | | N | 8.0 | 8.0 | 8.0 | 8.0 | 8.0 | 8.0 | 8.0 | 8.0 | 8.0 | 7.0 | 5.0 |
| PRO1543 | 0.05 | 6 | mean (%) | 100.0 | 101.7 | 136.9 | 129.0 | 59.9 | 36.5 | 28.1 | 26.8 | 21.7 | 20.5 | 20.5 |
| | | | SD (%) | 0.0 | 13.2 | 53.5 | 59.5 | 24.4 | 13.2 | 14.5 | 15.9 | 18.0 | 22.3 | 23.3 |
| | | | N | 8.0 | 8.0 | 8.0 | 8.0 | 8.0 | 8.0 | 8.0 | 8.0 | 8.0 | 7.0 | 7.0 |
| Trastuzumab + Nivolumab | 0.1+0.1 | 7 | mean (%) | 100.0 | 100.1 | 94.2 | 93.8 | 106.8 | 110.8 | 118.5 | 132.6 | 141.7 | 170.1 | 237.9 |
| | | | SD (%) | 0.0 | 21.3 | 30.5 | 35.3 | 42.2 | 47.6 | 53.2 | 67.7 | 74.3 | 81.1 | 102.3 |
| | | | N | 8.0 | 8.0 | 8.0 | 8.0 | 8.0 | 8.0 | 8.0 | 8.0 | 8.0 | 8.0 | 6.0 |

**Table 24: Relative body weight**

| Molecule | Dose | Grp | | 0 | 2 | 6 | 9 | 13 | 16 | 20 | 23 | 27 | 30 | 33 |
|---|---|---|---|---|---|---|---|---|---|---|---|---|---|---|
| | | | | | | | | | **Day** | | | | | | |
| Palivizumab | 0.1 | 1 | mean (%) | 100.0 | 100.1 | 102.3 | 106.4 | 106.3 | 109.6 | 112.7 | 110.0 | 107.4 | 112.6 | 117.8 |
| | | | SD (%) | 0.0 | 1.3 | 3.1 | 4.2 | 4.7 | 7.1 | 8.6 | 8.6 | 10.9 | 10.4 | 5.2 |
| | | | N | 8.0 | 8.0 | 8.0 | 8.0 | 8.0 | 8.0 | 8.0 | 8.0 | 8.0 | 6.0 | 4.0 |
| Nivolumab | 0.1 | 4 | mean (%) | 100.0 | 100.4 | 102.0 | 106.3 | 105.2 | 107.3 | 105.9 | 105.4 | 106.7 | 107.1 | 113.6 |
| | | | SD (%) | 0.0 | 1.7 | 2.4 | 4.1 | 4.1 | 5.9 | 8.2 | 10.4 | 7.3 | 6.3 | 10.8 |
| | | | N | 8.0 | 8.0 | 8.0 | 8.0 | 8.0 | 8.0 | 8.0 | 8.0 | 7.0 | 6.0 | 5.0 |
| PRO1678 | 0.1 | 2 | mean (%) | 100.0 | 99.3 | 101.4 | 105.3 | 106.0 | 111.4 | 115.6 | 114.4 | 111.1 | 109.2 | 108.1 |
| | | | SD (%) | 0.0 | 2.9 | 4.7 | 3.1 | 4.2 | 6.6 | 7.0 | 5.9 | 6.2 | 9.5 | 11.0 |
| | | | N | 8.0 | 8.0 | 8.0 | 8.0 | 8.0 | 8.0 | 8.0 | 8.0 | 8.0 | 8.0 | 7.0 |
| PRO1678 | 0.5 | 3 | mean (%) | 100.0 | 99.3 | 102.6 | 106.9 | 105.1 | 111.6 | 111.7 | 110.4 | 106.1 | 106.6 | 106.0 |
| | | | SD (%) | 0.0 | 1.1 | 1.8 | 3.1 | 3.9 | 4.2 | 3.7 | 2.8 | 5.8 | 11.3 | 11.0 |
| | | | N | 8.0 | 8.0 | 8.0 | 8.0 | 8.0 | 8.0 | 8.0 | 8.0 | 8.0 | 7.0 | 5.0 |
| PRO1543 | 0.05 | 9 | mean (%) | 100.0 | 100.0 | 101.8 | 105.1 | 103.8 | 107.0 | 110.3 | 111.8 | 109.4 | 110.4 | 107.8 |
| | | | SD (%) | 0.0 | 2.1 | 2.8 | 6.8 | 5.8 | 5.1 | 5.0 | 6.1 | 7.8 | 7.7 | 11.4 |
| | | | N | 8.0 | 8.0 | 8.0 | 8.0 | 8.0 | 8.0 | 8.0 | 8.0 | 8.0 | 7.0 | 7.0 |
| Trastuzumab + Nivolumab | 0.1 + 0.1 | 14 | mean (%) | 100.0 | 101.5 | 101.1 | 103.9 | 100.3 | 103.0 | 104.4 | 105.2 | 104.8 | 101.3 | 104.7 |
| | | | SD (%) | 0.0 | 2.3 | 3.6 | 5.8 | 5.5 | 7.2 | 6.0 | 7.0 | 7.3 | 7.0 | 2.5 |
| | | | N | 8.0 | 8.0 | 8.0 | 8.0 | 8.0 | 8.0 | 8.0 | 8.0 | 8.0 | 8.0 | 6.0 |

**Example 3: Generation and testing of multispecific constructs targeting HER2 (as an example of a TAA), PD-L1 and CD173 (as example of a costimulatory immune cell antigen):**

Design and production of MATCH4 molecules:

A description of the MATCH-4 molecules designed within this part of the current approach is shown in Figure 14. Molecule composition is shown in Table 25.

**Table 25: Composition of multispecific molecules**

| PRO ID | Format | Domain 1 | Specificity 1 | Domain 2 | Specificity 2 | Domain 3 | Specificity 3 | Domain 4 | Specificity 4 |
|---|---|---|---|---|---|---|---|---|---|
| PRO1778 | MATCH4 | Trastuzumab, VL-G141C/VH-G51C* | Her2 | 38-27-A11 sc07, VL-G141C/VH-G51C* | CD137 | 33-03-G02, VL-Q108A, VL-G109A** | PD-L1 | 23-13-A01-sc02 | HSA |
| PRO1780 | MATCH4 | Trastuzumab, VL-G141C/VH-G51C* | Her2 | 23-13-A01-sc03, VL-G141C/VH-G51C* | HSA | 33-03-G02, VL-Q108A, VL-G109A** | PD-L1 | 38-27-A11 sc07, VL-G141C/VH-G51C* | CD137 |
| PRO1992 | MATCH4 | 23-13-A01-sc02 | HSA | Pertuzumab, VL-G141C/VH-G51C* | Her2 | 33-03-G02, VL-Q108A, VL-G109A** | PD-L1 | 38-27-A11-sc07, VL-G141C/VH-G51C* | CD137 |
| PRO1993 | MATCH4 | Pertuzumab, VL-G141C/VH-G51C* | Her2 | 23-13-A01-sc02 | HSA | 38-27-A11-sc07, VL-G141C/VH-G51C* | CD137 | 33-03-G02, VL-Q108A, VL-G109A** | PD-L1 |

*VL/VH interdomain disulfide bond, numbering according to A. Honegger
**mutations, numbering according to A. Honegger

Method:

Expression of MATCH4 constructs was performed in CHO-S cells using CHOgro transient transfection kit (Mirus). Cultures were harvested after 5-7 days (cell viability <70 %) of expression at 37°C by centrifugation and proteins were purified from clarified culture supernatants by Protein L affinity chromatography followed, if needed, by a polishing step by size-exclusion chromatography. For the quality control of the manufactured material standard analytical methods, such as SE-HPLC, UV280 and SDS-PAGE were used.

**Assessment of the CD137 agonistic effect of anti-Her2xCD137xhSAxPD-L1$_{(low\ affinity)}$ MATCH4 molecules by using a cell-based assay of transgenic NF-kB Jurkat reporter cell line expressing CD137**

In this assay the activation of CD137 signaling in Jurkat cells was assessed. The activity of CD137 signaling is reported by measurement of Luciferase expression which is driven by CD137 induced NF-kB activation in a Jurkat reporter cell line. The expression of Luciferase directly correlates with the activity of CD137. Moreover, clustering of CD137, which is required for activation of the signal pathway, is facilitated via the formation of an immunological synapse between the Jurkat cells and a Her2 expressing cell line. Therefore, Her2 expression is needed for clustering and activation of CD137 on the reporter cell line.

Method:

Cancer cell lines HCC1954 (high levels of expression for Her2 and PD-L1) and HCC827 (low levels of expression for Her2 but high levels for PD-L1) were seeded at 25'000 cells per well on 96-well culture plates. Then, seeded cells were either stimulated with 10 ng/ml IFNy for 24 h or left unstimulated. Next, serial dilutions of MATCH4 molecules of interest as well as the internal reference molecules PRO1186 or PRO1430 (both anti-CD137xHSAxPD-L1 (high affinity) scMATCH3) were prepared and added to the cells. After addition of molecules of interest, Jurkat reporter cells were prepared in assay medium containing HSA at 25 mg/ml and added at a cell density of 40'000 cells per well. Luciferase expression was detected by addition of Luciferase reagent and was read by a luminescence reader 24 h after addition of Jurkat cells. Data were presented by plotting the relative luminescence units (RLU) of the test samples as a function of test sample concentration and fitted using a sigmoidal 4PL fit (GraphPad Prism).

Results:

As shown in Figure 15, PRO1993 was found to induce CD137 signaling in the Jurkat reporter cell line when cultured in the presence of HCC1954 cancer cells, whereas in the presence of HCC827 cancer cells only a slight activation of CD137 was observed. Data of NF-kB reporter gene assay of ND029 molecules are shown in Table 26.

**Table 26: Potencies of ND029 molecules in NF-kB reporter gene assay (CD137 activity assay).**

| | NFkB reporter gene assay | | | | | |
|---|---|---|---|---|---|---|
| | **High her2 expressing cells (HCC1954)** | | | **Low Her2 expressing cells (HCCB27)** | | |
| **PRO ID** | EC$_{50}$ (pM) | rel. EC50 | rel. activation (%) | EC$_{50}$ (pM) | rel. EC50 | rel. activation (%) |
| PROL1715 | 448.4 | 0.24* | 12.5$ | no activation | | |
| PROL1716 | no activation | | | no activation | | |
| PROL1717 | 130247 | 0.001* | 31.8$ | no activation | | |
| PROL1718 | 2662 | 0.040* | 19.0$ | no activation | | |
| PROL1719 | no activation | | | no activation | | |
| PRO1720 | no activation | | | no activation | | |
| PRO1721 | no activation | | | no activation | | |
| PRO1722 | no activation | | | no activation | | |
| PRO1778 | 661.0 | 0.14** | 50.1$$ | no activation | | |
| PRO1779 | 1881.0 | 0.05** | 61.1$$ | no activation | | |
| PRO1780 | 603.1 | 0.15** | 75.4$$ | no activation | | |
| PRO1186 | 125.6 | 1 | 100 | 127.3 | 1 | 100 |
| PRO1430 | 80.4 | 1 | 100 | 85.3 | 1 | 100 |
| PRO1992 | 82.4 | 2.18* | 44.9$ | 252.5 | 0.47* | 9.7$ |

(continued)

| NFkB reporter gene assay | | | | | | |
| --- | --- | --- | --- | --- | --- | --- |
| | High her2 expressing cells (HCC1954) | | | Low Her2 expressing cells (HCCB27) | | |
| PRO1993 | $EC_{50}$ (pM) 167.7 | rel. EC50 1.07* | rel. activation (%) 36.4$ | $EC_{50}$ (pM) 287.3 | rel. EC50 0.41* | rel. activation (%) 10.4$ |
| *: $IC_{50, PRO1430}$ (pM)/$IC_{50, test molecule}$ (pM)<br>**: $IC_{50, PRO1186}$ (pM)/$IC_{50, test molecule}$ (pM)<br>$: rel. to PRO1430<br>$$: rel. to PRO1186 | | | | | | |

**PD-L1/PD-1 neutralization by FC**

Method:

These assays were conducted to assess the ability of the multi-specific low affinity PD-L1x/Her2 constructs to neutralize the interaction between PD-1 and PD-L1 expressed on HCC1954 cells, which also express Her2. Specifically, the molecules should efficiently block PD-1 binding to PD-L1 on HER2/PD-L1 co-expressing target cells (HCC1954), while blockade of the PD-1/PD-L1 interaction on cells not expressing Her2 (HCC827) should be much less potent than with Avelumab/Bavencio® or Nivolumab/Obdivo®. Blockade of PD-1 binding to the cells was analyzed by flow cytometry in presence of human SA as described above. Serial dilutions of the respective molecules to be tested as well as the reference Avelumab were added to the plates. Individual $IC_{50}$ values on each plate were calibrated against the $IC_{50}$ of the reference molecule Avelumab that was taken along on each plate (relative $IC_{50}$: $IC_{50, Avelumab}/IC_{50, test molecule}$). In addition, the ratio between the relative $IC_{50}$ values found on high Her2 expressing HCC1954 and low Her2 expressing HCC827 cells was calculated.

Results:

Potencies of MATCH4 constructs of the present approach are summarized in Table 27. PD-L1 inhibition curve obtained for PRO1993 is shown in Figure 16.

**Table 27. Potencies of multi-specific molecules in PD-L1/PD-1 neutralization by flow cytometry.**
**Neutralization of PD-L1/PD-1 interaction in FC based assay**

| PRO ID | High Her2 expressing cells (HCC1954) | | Low Her2 expressing cells (HCC827) | | Ratio |
| --- | --- | --- | --- | --- | --- |
| | $IC_{50}$ (ng/ml) | rel. $IC_{50}$* | $IC_{50}$ (ng/ml) | rel. $IC_{50}$* | rel. $IC_{50}$ (high Her2) / rel. $IC_{50}$ (low Her2) |
| PRO1778 | 1418 | 0.09 | 6725 | 0.01 | 13.2 |
| PRO1780 | 695.4 | 0.18 | 32646 | 1.41E-03 | 130.2 |
| PRO1992 | 591.6 | 0.22 | no inhibition | | NA |
| PRO1993 | 166.7 | 0.77 | no inhibition | | NA |
| Avelumab | 127.7 | 1 | 46.03 | 1 | 1 |
| Nivolumab | 43.48 | 2.94 | 39.77 | 1.16 | 2.54 |
| NA: not applicable<br>*$IC_{50, Avelumab}$ (ng/ml)/$IC_{50, test molecule}$ (ng/ml) | | | | | |

**Example 4: Generation and testing of multispecific constructs targeting HER2 (as an example of a TAA) and PD-L1:**

**Design and production of scDb-scFv:**

A description of the different scDb-scFv (scMATCH3) molecules designed within this aspect of the present approach is shown in Figure 17. The data regarding the production of all the molecules are detailed in Table 28, which describes the domains composing of the different molecules produced and their positioning within the molecules.

Method:

Expression of scDb-scFv constructs was performed in CHO-S cells using CHOgro transient transfection kit (Mirus). Cultures were harvested after 5-7 days (cell viability <70 %) of expression at 37°C by centrifugation and proteins were purified from clarified culture supernatants by Protein L affinity chromatography followed, if needed, by a polishing step by size-exclusion chromatography. For the quality control of the manufactured material standard analytical methods, such as SE-HPLC, UV280 and SDS-PAGE were used.

**Table 28: Composition of multispecific constructs of the present approach**

| PRO ID | Format | Domain 1 | Specificity 1 | Domain 2 | Specificity 2 | Domain 3 | Specificity 3 | Domain 4 | Specificity 4 |
|---|---|---|---|---|---|---|---|---|---|
| PRO 1678 | scDb-scFv | Trastuzumab, VL-G141 C/VH-G51C* | Her2 | 33-03-G02, VL-Q108A, VL-G109A** | PD-L1 | 23-13-A01-sc02 | HSA | NA | NA |
| PRO 1679 | scDb-scFv | 33-03-G02, VL-Q108A, VL-G109A** | PD-L1 | Trastuzumab, VL-G141C/VH-G51C* | Her2 | 23-13-A01-sc02 | HSA | NA | NA |
| PRO 1680 | scDb-scFv | Trastuzumab, VL-G141C/VH-G51C* | Her2 | 33-03-G02, VL-Q108A, VH-Y112A** | PD-L1 | 23-13-A01-sc02 | HSA | NA | NA |
| PRO 1681 | scDb-scFv | 33-03-G02, VL-Q108A, VH-Y112A** | PD-L1 | Trastuzumab, VL-G141C/VH-G51C* | Her2 | 23-13-A01-sc02 | HSA | NA | NA |
| PRO 1814 | scDb-scFv | Pertuzumab | Her2 | 33-03-G02, VL-Q108A, VL-G109A** | PD-L1 | 19-01-H04-sc03 | HSA | NA | NA |
| PRO 1852 | scDb-scFv | Pertuzumab, VL-G141C/VH-G51C* | Her2 | 33-03-G02, VL-Q108A, VL-G109A** | PD-L1 | 19-01-H04-sc03 | HSA | NA | NA |
| PRO 1853 | scDb-scFv | Pertuzumab | Her2 | 33-03-G02, VL-Q108A, VH-Y112A** | PD-L1 | 19-01-H04-sc03 | HSA | NA | NA |
| PRO 1854 | scDb-scFv | Pertuzumab, VL-G141C/VH-G51C* | Her2 | 33-03-G02, VL-Q108A VH-Y112A** | PD-L1 | 19-01-H04-sc03 | HSA | NA | NA |
| *VL/VH interdomain disulfide bond, numbering according to A. Honegger **mutations, numbering according to A. Honegger | | | | | | | | | |

EP 3 816 185 A1

**Affinities to human Her2, PD-L1 and human and mouse serum albumin (SA) by SPR**

Method:

Affinity to human PD-L1, Her2 and human and mouse serum albumin (SA) was determined by SPR using a Biacore T200 device (GE Healthcare) as described above. Obtained SPR sensorgrams were double-referenced (empty reference channel and zero analyte injection) and fitted using BiaEvaluation software (GE Healthcare) and the 1:1 Langmuir binding model. Quality of the fits was monitored based on Chi2 and U-value (Biacore). Retrieved kinetic parameters were used to calculate the apparent dissociation equilibrium constant ($K_D$). In addition to the kinetic measurement, the value of the $K_D$ for the low affinity PD-L1 measurements was obtained by fitting a plot of response at equilibrium against the concentration (steady-state affinity measurement).

Results:

As shown in Table 29, binding to Her2 and human SA was similar for all molecules tested. Affinity to PD-L1 could be determined only for the molecules PRO1678 and PRO1679 using the steady state analysis and for both proteins, the affinity found was comparable ($K_D$ values, PRO1678: 156nM, PRO1679: 122 nM).

**Table 29: Summary of affinities of scMATCH3 constructs of the present approach directed at human Her2, PD-L1 and human and mouse serum albumin (SA) at pH5.5.**

| PRO ID | Affinity to human Her2 | | | Affinity to human PD-L1 | | | | Affinity to human SA (at pH5.5) | | | Affinity to mouse SA (at pH5.5) | | |
| --- | --- | --- | --- | --- | --- | --- | --- | --- | --- | --- | --- | --- | --- |
| | | | | kinetic analysis | | | steady state analysis | | | | | | |
| | $k_a$ (M$^{-1}$ s$^{-1}$) | $k_d$ (s$^{-1}$) | $K_D$ (M) | $k_a$ (M$^{-1}$ s$^{-1}$) | $k_d$ (s$^{-1}$) | $K_D$ (M) | $K_D$ (M) | $k_a$ (M$^{-1}$ s$^{-1}$) | $k_d$ (s$^{-1}$) | $K_D$ (M) | $k_a$ (M$^{-1}$ s$^{-1}$) | $k_d$ (s$^{-1}$) | $K_D$ (M) |
| PRO1678 | 2.13E+05 | 7.11E-05 | 3.34E-10 | not determined | | | 1.56E-07 | 2.21E+05 | 7.62E-04 | 3.45E-09 | 1.37E+05 | 5.18E-03 | 3.78E-08 |
| PRO1679 | 2.00E+05 | 6.49E-05 | 3.24E-10 | not determined | | | 1.22E-07 | 1.88E+ 05 | 7.33E-04 | 3.89E-09 | not determined | | |
| PRO1680 | 2.15E+05 | 6.87E-05 | 3.19E-10 | not determined | | | | 1.88E+05 | 7.32E-04 | 3.90E-09 | not determined | | |
| PRO1681 | 1.96E+05 | 6.26E-05 | 3.19E-10 | not determined | | | | 1.91E+05 | 7.37E-04 | 3.85E-09 | not determined | | |
| PRO1814 | not measured | | | not measured | | | | not measured | | | not measured | | |
| PRO1852 | not measured | | | not measured | | | | not measured | | | not measured | | |
| PRO1853 | not measured | | | not measured | | | | not measured | | | not measured | | |
| PRO1854 | not measured | | | not measured | | | | not measured | | | not measured | | |
| Avelumab | NA | | | 4.91E+05 | 1.21E-05 | 2.47E-11 | | NA | | | NA | | |
| Nivolumab | NA | | | not measured | | | | NA | | | NA | | |
| NA: not applicable | | | | | | | | | | | | | |

**PD-L1/PD-1 neutralization by FC**

Method:

The assay was conducted to assess the ability of the multi-specific low affinity PD-L1x/Her2 constructs to neutralize the interaction between PD-1 and PD-L1 expressed on HCC1954 cells, which also express Her2. Specifically, the molecules should efficiently block PD-1 binding to PD-L1 on Her2/PD-L1 co-expressing target cells (HCC1954), while blockade of the PD-1/PD-L1 interaction on cells not expressing Her2 (HCC827) should be much less potent than with Avelumab/Bavencio® or Nivolumab/ Obdivo®. Blockade of PD-1 binding to the cells was analyzed by flow cytometry in presence of human SA as described above. Individual $IC_{50}$ values on each plate were calibrated against the $IC_{50}$ of the reference molecule Avelumab that was taken along on each plate (relative $IC_{50}$: $IC_{50}$, Avelumab/$IC_{50}$, test molecule). In addition, the ratio between the relative $IC_{50}$ values found on high Her2 expressing HCC1954 and low Her2 expressing HCC827 cells was calculated.

Results:

Potencies of scMATCH3 constructs of the present approach are summarized in Table 30.

**Table 30. Potencies of constructs of the present approach to neutralize PD-L1/PD-1 interaction in flow cytometry assay.**

| PRO ID | Neutralization of PD-L1/PD-1 interaction in FC based assay | | | | |
|---|---|---|---|---|---|
| | High Her2 expressing cells (HCC1954) | | Low Her2 expressing cells (HCC827) | | Ratio |
| | $IC_{50}$ (ng/ml) | rel. $IC_{50}$* | $IC_{50}$ (ng/ml) | rel. $IC_{50}$* | rel. $IC_{50}$ (high Her2) / rel. $IC_{50}$ (low Her2) |
| PRO1678 | 428.3 | 0.29 | 43466 | 2.89E-03 | 100.4 |
| PRO1679 | 652.4 | 0.19 | 118941 | 1.06E-03 | 179.7 |
| PRO1680 | 1146 | 0.11 | 2.10E+06 | 5.98E-05 | 1840.9 |
| PRO1681 | 2011 | 0.06 | 1.52E+06 | 8.27E-05 | 725.9 |
| PRO1814 | 52.53 | 0.86 | 53797 | 1.02E-03 | 841.7 |
| PRO1852 | 55.55 | 0.81 | 16835 | 3.26E-03 | 249.1 |
| PRO1853 | 69.48 | 0.65 | 88234 | 6.22E-04 | 1043.7 |
| PRO1854 | 56.06 | 0.80 | 53489 | 1.03E-03 | 784.2 |
| Avelumab | 127.7 | 1 | 46.03 | 1 | 1 |
| Nivolumab | 43.48 | 2.94 | 39.77 | 1.16 | 2.54 |

NA: not applicable

*$IC_{50, Avelumab}$ (ng/ml)/$IC_{50, test molecule}$ (ng/ml)

**Claims**

1. A multispecific antibody comprising

   (a) a first domain specifically binding to PD-L1 comprising a VH sequence of SEQ ID NO: 11 and a VL sequence of SEQ ID NO: 16, and
   (b) a second domain specifically binding to a tumor-associated antigen (TAA).

2. The multispecific antibody of claim 1, wherein said TAA is selected from the group consisting of EGFRvIII, 5T4, CD19, CD20, CD22, CD38, BCMA, IL4RA, mesothelin, GD2, Tn antigen, sTn antigen, Tn-O-Glycopeptides, sTn-O-Glycopeptides, PSMA, CD97, TAG72, CD44v6, CEA, EPCAM, KIT, IL-13Ra2, leguman, GD3, CD171, IL-11 Ra, IL-13RA2, ROR1, PSCA, MAD-CT-1, MAD-CT-2, VEGFR2, CLEC12A, LewisY, CD24, PDGFR-beta, SSEA-4, folate receptor alpha, ERBBs (e.g., ERBB2), Her2/neu (HER2), MUC1, MUC16, EGFR, NCAM, Ephrin B2, CAIX, LMP2, sLe, HMWMAA, o-acetyl-GD2, folate receptor beta, TEM1/CD248, CD33, CD123, CD133, CD135, TEM7R, FAP, Legumain, HPV E6 or E7, ML-IAP, CLDN6, TSHR, GPRC5D, ALK, Polysialic acid, Fos-related antigen, neutrophil elastase, TRP-2, CYP1B1, sperm protein 17, beta human chorionic gonadotropin, AFP, thyroglobulin, PLAC1, globoH, RAGE1, MN-CA IX, human telomerase reverse transcriptase, intestinal carboxyl esterase, mut hsp 70-2, NA-17, NY-BR-1, UPK2, HAVCR1, ADRB3, PANX3, NY-ESO-1, GPR20, Ly6k, OR51 E2, TARP, GFRalpha4; GPC3, CDH3, B7H3; FGFR1, SSTR2, CECAM6, GA733, and gp120; particularly, wherein said TAA is selected from HER2 and mesothelin, more particularly HER2.

3. The multispecific antibody of claim 2, wherein said second domain comprises the VH sequence of SEQ ID NO: 34 and the VL sequence of SEQ ID NO: 36.

4. The multispecific antibody of any one of claims 1 to 3 further comprising a third domain specifically binding to an immune cell antigen, in particular wherein said immune cell antigen is present on T cell or NK cell.

5. The multispecific antibody of claim 4, wherein said third domain is an agonist and said second immune checkpoint antigen is a stimulatory immune cell antigen, particularly wherein said stimulatory immune cell antigen is selected from the group consisting of CD3, and CD16, more particularly CD3.

6. The multispecific antibody of claim 5, wherein said third domain comprises the VH sequence of SEQ ID NO: 43 and the VL sequence of SEQ ID NO: 44.

7. The multispecific antibody of claim 4, wherein said third domain is an agonist and said second immune checkpoint antigen is a co-stimulatory immune cell antigen, particularly wherein said co-stimulatory immune cell antigen is selected from the group consisting of CD137, CD28, ICOS, HVEM, CD27, OX40, DR3, GITR, CD30, SLAM, CD2, 2B4, TIM1, TIM2, CD226., more particularly CD137.

8. The multispecific antibody of claim 7, wherein said third domain specifically binds to CD137 at an epitope comprised in the distal part of the extracellular domain of CD137, particularly within the cysteine-rich domains CRD1 and/or CRD2, more particularly within amino acid residues 24-86 of SEQ ID NO: 130, provided that amino acid residue Asn42 of CD137 is not a critical residue for binding.

9. The multispecific antibody of claim 8, wherein said third domain comprises the VH sequence of SEQ ID NO: 67 and the VL sequence of SEQ ID NO: 68.

10. The multispecific antibody of claim 4, wherein said third domain is an inhibitor and said second immune checkpoint antigen is an inhibitory immune checkpoint antigen, particularly wherein said inhibitory immune checkpoint antigen is selected from the group consisting of cytotoxic T-lymphocyte-associated protein 4 (CTLA4), PD-1, lymphocyte-activation gene 3, and T-cell immunoglobulin mucin-3, BTLA, TIM3, TIGIT, CD160, LAIR1, B7-1, and B7-H1.

11. The multispecific antibody of any one of the preceding claims further comprising a domain specifically binding to human serum albumin (HSA).

12. The multispecific antibody of claim 11, wherein said domain specifically binding to HSA comprises:

(a) the VH sequence of SEQ ID NO: 51 and the VL sequence of SEQ ID NO: 52, or
(b) the VH sequence of SEQ ID NO: 59 and the VL sequence of SEQ ID NO: 60.

13. The multispecific antibody of any one of the preceding claims, wherein said multispecific antibody is in a format selected from the group consisting of a single-chain diabody (scDb), a tandem scDb (Tandab), a linear dimeric scDb (LD-scDb), a circular dimeric scDb (CD-scDb), a bispecific T-cell engager (BiTE; tandem di-scFv), a tandem tri-scFv, a tribody (Fab-(scFv)2) or bibody (Fab-(scFv)1), Fab, Fab-Fv2, Morrison (IgG CH3-scFv fusion (Morrison L) or IgG CL-scFv fusion (Morrison H)), triabody, scDb-scFv, bispecific Fab2, diminiantibody, tetrabody, scFv-Fc-scFv fusion, scFv-HSA-scFv fusion, didiabody, DVD-Ig, COVD, IgG-scFab, scFab-dsscFv, Fv2-Fc, IgG-scFv fusions, such as bsAb (scFv linked to C-terminus of light chain), Bs1Ab (scFv linked to N-terminus of light chain), Bs2Ab (scFv linked to N-terminus of heavy chain), Bs3Ab (scFv linked to C-terminus of heavy chain), Ts1Ab (scFv linked to N-terminus of both heavy chain and light chain), Ts2Ab (dsscFv linked to C-terminus of heavy chain), Bispecific antibodies based on heterodimeric Fc domains, such as Knob-into-Hole antibodies (KiHs); an scDb, tandem-di-scFv, tandem tri-scFv, Fab-(scFv)2, Fab-(scFv)1, Fab, Fab-Fv2, COVD fused to the N- and/or the C-terminus of either chain of a heterodimeric Fc domain or any other heterodimerization domain, a MATCH and DuoBodies, particularly wherein said antibody is a scDb-scFv, tribody, DVD-tribody, MATCH, in particular wherein said multi-specific antibody is in a MATCH or tribody format, more particularly wherein said multispecific antibody is in a MATCH format, more particularly wherein said multispecific antibody is a MATCH3 or a MATCH4.

14. The multispecific antibody of any one of the preceding claims, wherein said multispecific antibody is in the MATCH4 format and comprises the two chains according to SEQ ID NOs: 113 and 114.

15. An antibody-based domain specifically binding to PD-L1 comprising a VH comprising an amino acid sequence that is at least 90 percent, in particular at least 95 percent identical to the amino acid sequence SEQ ID NO: 11; and a VL comprising an amino acid sequence that is at least 90 percent, in particular at least 95 percent identical to the amino acid sequence SEQ ID NO: 16.

**Figure 1**:

**Figure 2**:

**Figure 3**:

Tribody                    DVD-Tribody                    MATCH4

| | |
|---|---|
| ⌐¬ | Inderdomain disulfide bond (ID DiS) |
| ⌐ ↗ | Peptide linker |
| N | N-terminus |
| C | C-terminus |
| CL/CH₁ | Constant light (CL) and heavy 1 (CH1) domain |
| VLx/VHx | Variable light (VL) and heavy (VH) domain # |

## Figure 4:

A  Her2/PD-L1 expressing cells (HCC1954)

B  PD-L1 expressing cells (HCC827)

## Figure 5:

A  Her2/PD-L1 expressing cells (HCC1954)

B  PD-L1 expressing cells (HCC827)

## Figure 6:

A — Her2/PD-L1 expressing cells (HCC1954)
— PRO1454, PRO1456, PRO1455

B — Her2/PD-L1 expressing cells (HCC1954)
— PRO1497, PRO1456, PRO1498

## Figure 7:

A — Activation of NFAT/luc-reporter T cells Co-cultured with Her2+++ HCC1954
— PRO1543, PRO1543 + Nivolumab, PRO1557, PRO1557 + Nivolumab, PRO1557 + PRO1434, PRO1546, PRO1546 + Nivolumab

B — Activation of NFAT/luc-reporter T cells Co-cultured with Her2⁻/PD-L1⁺ CHO PD-L1 cells
— PRO1543, PRO1543 + Nivolumab, PRO1557, PRO1557 + Nivolumab, PRO1557 + PRO1434, PRO1546, PRO1546 + Nivolumab

# Figure 8:

A  Her2$^{+++}$/PD-L1$^+$ HCC1954 target cell killing
Co-cultured with hPBMCs

- PRO1543
- PRO1543 + Nivolumab
- PRO1557
- PRO1557 + Nivolumab
- PRO1557 + PRO1434
- PRO1546
- PRO1546 + Nivolumab

B  Her2$^-$/PD-L1$^+$ CHO target cells killing
Co-cultured with hPBMCs

- PRO1543
- PRO1543 + Nivolumab
- PRO1557
- PRO1557 + Nivolumab
- PRO1557 + PRO1434
- PRO1546
- PRO1546 + Nivolumab

# Figure 9:

A  CD8+ cells activation
in presence of Her2$^{+++}$/PD-L1$^+$ target cells (HCC1954)

- PRO1543
- PRO1543 + Nivolumab
- PRO1557
- PRO1557 + Nivolumab
- PRO1557 + PRO1434
- PRO1546
- PRO1546 + Nivolumab

B  CD8+ cell activation
in presence of Her2$^-$/PD-L1$^+$ target cells (CHO PD-L1)

- PRO1543
- PRO1543 + Nivolumab
- PRO1557
- PRO1557 + Nivolumab
- PRO1557 + PRO1434
- PRO1546
- PRO1546 + Nivolumab

**Figure 10**:

## Figure 11:

A  Her2$^{+++}$/PD-L1$^{+}$ HCC1954 target cell killing
Co-cultured with hPBMCs

B  Her2$^{+}$/PD-L1$^{-}$ MCF-7 target cell killing
Co-cultured with hPBMCs

C  CD8$^{+}$ cells activation
in presence of Her2$^{+++}$/PD-L1$^{+}$ HCC1954 cells

D  CD8$^{+}$ cells activation
in presence of Her2$^{+}$/PD-L1$^{-}$ MCF-7 cells

**Figure 12:**

A  Her2+/−/PD-L1+ HCC827 target cell killing
   Co-cultured with hPBMCs

B  Her2−/PD-L1+ CHO-PD-L1 target cell killing
   Co-cultured with hPBMCs

C  CD8+ cells activation
   in presence of Her2+/−/PD-L1+ HCC827 cells

D  CD8+ cell activation
   in presence of Her2−/PD-L1+ CHO-PD-L1 cells

# Figure 13:

**HCC1954 xeno in humanized NOG**
Tumor localized CD3 activation/PD-L1 blockade

—▽— Palivizumab (5 mg/kg)

—△— Nivolumab (5 mg/kg)

—⊖— PRO1678 (5 mg/kg)

—●— PRO1678 (25 mg/kg)

—■— PRO1543 (2.5 mg/kg)

—◆— Trastuzumab (5 mg/kg) + Nivolumab (5 mg/kg)

**Figure 14**:

MATCH4

| | |
|---|---|
| ⌐ | Inderdomain disulfide bond (ID DiS) |
| ⌒⌒ | Peptide linker |
| N | N-terminus |
| C | C-terminus |
| CL/CH₁ | Constant light (CL) and heavy 1 (CH1) domain |
| VLx/VHx | Variable light (VL) and heavy (VH) domain # |

Figure 15:

**CD137 activity in presence of diffent target cell lines**

Her2$^{+++}$/PD-L1$^{+}$ (HCC1954)

Her2$^{+/-}$/PD-L1$^{+}$ (HCC827)

**Figure 16:**

Inhibition of PD-1 binding to Her2$^{+++}$ HCC1954 cells
in presence of human SA

Inhibition of PD-1 binding to Her2$^{+/-}$ HCC827 cells
in presence of human SA

# Figure 17:

scDb-scFv

⌒  Peptide linker

N  N-terminus

C  C-terminus

VLx/VHx  Variable light (VL) and heavy (VH) domain #

**Figure 18:**

A    Her2/PD-L1 expressing cells
(HCC1954)

B    PD-L1 expressing cells
(HCC827)

## EUROPEAN SEARCH REPORT

**Application Number**

EP 19 20 6959

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | EP 3 470 426 A1 (NUMAB THERAPEUTICS AG [CH]) 17 April 2019 (2019-04-17)<br>* paragraphs [0001], [0015] - [0026], [0185] - [0190], [0221], [0280] - [0284], [0305]; tables 6,7; sequences 88,98,112,122,134,144 * | 15<br><br>1-14 | INV.<br>C07K16/28<br>C07K16/32 |
| A | WO 2017/220988 A1 (KYMAB LTD [GB]) 28 December 2017 (2017-12-28)<br>* page 5, line 12 - page 6, line 32 *<br>* page 99, line 35 - page 101, line 8 * | 1-15 | |
| A | WO 2019/072868 A1 (NUMAB THERAPEUTICS AG [CH]) 18 April 2019 (2019-04-18)<br>* page 6 - page 16; sequences 73,85 * | 1-15 | |
| A | WO 2018/224441 A1 (NUMAB INNOVATION AG [CH]) 13 December 2018 (2018-12-13)<br>* paragraphs [0014] - [0023]; sequences 4,8 * | 6 | |

**TECHNICAL FIELDS SEARCHED (IPC)**

C07K

The present search report has been drawn up for all claims

| Place of search | Date of completion of the search | Examiner |
|---|---|---|
| Munich | 6 May 2020 | Page, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 20 6959

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2020

| Patent document cited in search report | | Publication date | Patent family member(s) | | Publication date |
|---|---|---|---|---|---|
| EP 3470426 | A1 | 17-04-2019 | NONE | | |
| WO 2017220988 | A1 | 28-12-2017 | AU | 2017281830 A1 | 13-12-2018 |
| | | | BR | 112018076260 A2 | 26-03-2019 |
| | | | BR | 112018076281 A2 | 26-03-2019 |
| | | | CA | 3026474 A1 | 28-12-2017 |
| | | | CA | 3026477 A1 | 28-12-2017 |
| | | | CN | 109475602 A | 15-03-2019 |
| | | | CN | 109475603 A | 15-03-2019 |
| | | | EP | 3471753 A1 | 24-04-2019 |
| | | | EP | 3471754 A1 | 24-04-2019 |
| | | | JP | 2019528083 A | 10-10-2019 |
| | | | JP | 2020511932 A | 23-04-2020 |
| | | | KR | 20190030208 A | 21-03-2019 |
| | | | KR | 20190032355 A | 27-03-2019 |
| | | | SG | 11201810509P A | 28-12-2018 |
| | | | TW | 201803904 A | 01-02-2018 |
| | | | TW | 201803905 A | 01-02-2018 |
| | | | TW | 201811820 A | 01-04-2018 |
| | | | TW | 201900680 A | 01-01-2019 |
| | | | WO | 2017220988 A1 | 28-12-2017 |
| | | | WO | 2017220989 A1 | 28-12-2017 |
| | | | WO | 2017220990 A1 | 28-12-2017 |
| WO 2019072868 | A1 | 18-04-2019 | AU | 2018348429 A1 | 12-03-2020 |
| | | | CA | 3075969 A1 | 18-04-2019 |
| | | | WO | 2019072868 A1 | 18-04-2019 |
| WO 2018224441 | A1 | 13-12-2018 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017123650 A
- WO 2016149201 A
- WO 2017182672 A1
- US 6075181 A
- US 6150584 A
- US 5766886 A
- WO 200492219 A, Hinton
- WO 200442072 A
- EP 0486525 A
- US 6267964 B
- WO 2004001064 A
- WO 2002076489 A
- WO 200145746 A
- WO 2004003019 A
- WO 2008096158 A
- WO 2005118642 A
- WO 20060591056 A
- WO 2011006915 A

- WO 2009040562 A
- WO 2010035012 A
- WO 2011086091 A
- WO 2019057787 A
- WO 20160202457 A
- EP 404097 A
- WO 9301161 A
- WO 9957150 A
- US 5260203 A
- US 5455030 A
- US 4881175 A
- US 5132405 A
- US 5091513 A
- US 5476786 A
- US 5013653 A
- US 5258498 A
- US 5482858 A
- US 4458066 A

### Non-patent literature cited in the description

- **KATSUYA Y et al.** *Lung Cancer,* 2015, vol. 88 (2), 154-159
- **NAKANISHI et al.** *Cancer Immunol Immunother.,* 2007, vol. 56 (8), 1173-1182
- **NOMI T et al.** *Clin Cancer Res.,* 2007, vol. 13 (7), 2151-2157
- **FAY AP et al.** *Immunother Cancer,* 2015, vol. 3 (3
- **STROME SE et al.** *Cancer Res.,* 2003, vol. 63 (19), 6501-6505
- **JACOBS JF et al.** *Neuro Oncol.,* 2009, vol. 11 (4), 394-402
- **WILMOTTE R et al.** *Neuroreport,* 2005, vol. 16 (10), 1081-1085
- **DONG H et al.** *Nat Med.,* 2002, vol. 8 (8), 793-800
- **WANG et al.** *Onco Targets Ther.,* 2016, vol. 9, 5023-5039
- **POSTOW MA et al.** *J Clin Oncol.,* 2015, vol. 33 (17), 1974-82
- **BUTTE MJ et al.** *Immunity,* 2007, vol. 27, 111-122
- **CHESTER C. et al.** *Cancer Immunol Immunother,* 2016, vol. 65 (10), 1243-8
- **DURAISWAMY J et al.** *Cancer Res,* 2013, vol. 73, 6900-6912
- **WANG et al.** *Cancer J.,* 2018, vol. 24, 36-40

- **WANG et al.** *Immunol Rev.,* 2009, vol. 229 (1), 192-215
- **ZHANG et al.** *J Immunol.,* 2010, vol. 184 (2), 787-795
- **BROIL K et al.** *Am J Clin Pathol.,* 2001, vol. 115 (4), 543-549
- **SEAMAN et al.** *Cancer Cell,* 2007, vol. 11 (6), 539-554
- **OLOFSSON et al.** *Circulation,* 2008, vol. 117 (10), 1292-1301
- **CHESTER C. et al.** *Cancer Immunol Immunother Oct,* 2016, vol. 65 (10), 1243-8
- **NIU L et al.** *J Immunol,* 2007, vol. 178 (7), 4194-4213
- **DUBROT et al.** *Int J Cancer,* 2011, vol. 128 (1), 105-118
- **SZNOL M. et al.** *J Clin Oncol,* 2008, vol. 26 (115S), 3007
- **ASCIERTO PA et al.** *Semin Oncol,* 2010, vol. 37 (5), 508-516
- **WARD et al.** *Nature,* 1989, vol. 341, 544-546
- **KABAT et al.** Sequences of Proteins of Immunological Interest. National Institutes of Health, 1991
- **AL-LAZIKANI et al.** *JMB,* 1997, vol. 273, 927-948

- **LEFRANC, M.-P.** *The Immunologist,* 1999, vol. 7, 132-136
- **LEFRANC, M.-P. et al.** *Dev. Comp. Immunol.,* 2003, vol. 27, 55-77
- **HONEGGER ; PLÜCKTHUN.** *J. Mol. Biol.,* 2001, vol. 309, 657-670
- **HOOGENBOOM ; WINTER.** *J. Mol. Biol,* 1991, vol. 227, 381
- **MARKS et al.** *J. Mol. Biol,* 1991, vol. 222, 581
- **COLE et al.** Monoclonal Antibodies and Cancer Therapy. *Alan R. Liss,* 1985, 77
- **BOEMER et al.** *J. Immunol,* 1991, vol. 147 (I), 86-95
- **VAN DIJK ; VAN DE WINKEL.** *Curr. Opin. Pharmaco,* 2001, vol. 5, 368-74
- **LI et al.** *Proc. Natl. Acad. Sci. USA,* 2006, vol. 103, 3557-3562
- **MORRISON et al.** *Proc. Natl. Acad. Sci. USA,* 1984, vol. 81, 6851-6855
- **MORRISON ; OI.** *Adv. Immunol.,* 1988, vol. 44, 65-92
- **VERHOEYEN et al.** *Science,* 1988, vol. 239, 1534-1536
- **PADLAN.** *Molec. Immun.,* 1991, vol. 28, 489-498
- **PADLAN.** *Molec. Immun.,* 1994, vol. 31, 169-217
- **M. DAERON.** *Annu. Rev. Immunol.,* 1997, vol. 5, 203-234
- **RAVETCH ; KINET.** *Annu. Rev. Immunol.,* 1991, vol. 9, 457-92
- **CAPET et al.** *Immunomethods,* 1994, vol. 4, 25-34
- **DE HAAS et al.** *Lab. Clin. Med.,* 1995, vol. 126, 330-41
- **GUYER et al.** *J. Immunol.,* 1976, vol. 117, 587
- **KIM et al.** *J. Immunol.,* 1994, vol. 24, 249
- **GHETIE ; WARD.** *Immunol.,* 1997, vol. 18 (12), 592-8
- **GHETIE et al.** *Nature Biotechnology,* 1997, vol. 15 (7), 637-40
- **HINTON et al.** *J. Biol. Chem.,* 2004
- **SHIELDS et al.** *J. Biol. Chem.,* 2001, vol. 9 (2), 6591-6604
- **MEDASAN et al.** *J. Immunol.,* 1997, vol. 158, 2211-2217
- **SUGIO.** *Protein Eng,* 1999, vol. 12, 439-446
- **FANALI.** *Molecular Aspects of Medicine,* 2012, vol. 33, 209-290
- **SMITH et al.** *Bioconjugate Chem.,* 2001, vol. 12, 750-756
- **HOLT et al.** *Protein Engineering, Design & Selection,* vol. 21 (5), 283-288
- **KNAPPIK et al.** *J. Mol. Biol.,* 2000, vol. 296, 57-86
- **EGAN T. et al.** *mAbs,* 2017, vol. 9, 68-84
- *MAbs,* February 2017, vol. 9 (2), 182-212
- **HUDSON et al.** *Nat. Med.,* 2003, vol. 9, 129-134
- **HOLLINGER et al.** *Proc. Natl. Acad. Sci. USA,* 1993, vol. 90, 6444-6448
- **FISCHER, N. ; LEGER, O.** *Pathobiology,* 2007, vol. 74, 3-14
- **HORNIG, N. ; FÄRBER-SCHWARZ, A.** *Methods Mol. Biol.,* 2012, vol. 907, 713-727
- **LABRIJN et al.** *Proc. Natl. Acad. Sci. USA,* 2013, vol. 110, 5145-5150
- **DE KRUIF et al.** *Biotechnol. Bioeng.,* 2010, vol. 106, 741-750
- **ZHU et al.** *Cancer Lett.,* 1994, vol. 86, 127-134
- **SURESH et al.** *Methods Enzymol.,* 1986, vol. 121, 210-228
- **YOKOYAMA et al.** Curr. Protoc. Immunol. 2006
- **CHAMES ; BATY.** *FEMS Microbiol. Letters,* 2000, vol. 189, 1-8
- **KARPOVSKY et al.** *J. Exp. Med.,* 1984, vol. 160, 1686
- **LIU, M A et al.** *Proc. Natl. Acad. Sci. USA,* vol. 82, 8648
- **PAULUS.** *Behring Ins. Mitt.,* 1985, vol. 78, 118-132
- **BRENNAN et al.** *Science,* 1985, vol. 229, 81-83
- **GLENNIE et al.** *J. Immunol.,* 1987, vol. 139, 2367-2375
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **NARANG et al.** *Meth. Enzymol.,* 1979, vol. 68, 90
- **BROWN et al.** *Meth. Enzymol.,* 1979, vol. 68, 109
- **BEAUCAGE et al.** *Tetra. Lett.,* 1981, vol. 22, 1859
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.,* 1991, vol. 19, 967
- **ECKERT et al.** *PCR Methods and Applications,* 1991, vol. 1, 17
- **HARRINGTON et al.** *Nat Genet.,* 1997, vol. 15, 345
- *Smith, Annu. Rev. Microbiol.,* 1995, vol. 49, 807
- **ROSENFELD et al.** *Cell,* 1992, vol. 68, 143
- **SCHARF et al.** *Results Probl. Cell Differ.,* 1994, vol. 20, 125
- **BITTNER et al.** *Meth. Enzymol.,* 1987, vol. 153, 516

- **WINNACKER.** FROM GENES TO CLONES. VCH Publishers, 1987
- **QUEEN et al.** *Immunol. Rev.,* 1986, vol. 89, 49-68
- **ELLIOT ; O'HARE.** *Cell,* 1997, vol. 88, 223
- **REMINGTON.** The Science and Practice of Pharmacy. Mack Publishing Co, 2000
- Sustained and Controlled Release Drug Delivery Systems. Marcel Dekker, Inc, 1978